(19) Europäisches Patentamt
European Patent Office
Office européen des brevets

(11) Publication number: **0 295 056 B1**

(12) # EUROPEAN PATENT SPECIFICATION

(45) Date of publication of patent specification: **05.08.92**

(51) Int. Cl.[5]: **C07D 307/60**, C07D 307/32, C07D 409/06, A61K 31/365

(21) Application number: **88305189.8**

(22) Date of filing: **07.06.88**

The file contains technical information submitted after the application was filed and not included in this specification

(54) **Anti-inflammatory furanones.**

(30) Priority: **08.06.87 US 59282**

(43) Date of publication of application:
**14.12.88 Bulletin 88/50**

(45) Publication of the grant of the patent:
**05.08.92 Bulletin 92/32**

(84) Designated Contracting States:
**AT BE CH DE ES FR GB GR IT LI LU NL SE**

(56) References cited:
**EP-A- 0 133 376**
**EP-A- 0 209 274**
**WO-A-87/05019**

**CHEMICAL ABSTRACTS, vol. 107, nr. 5. 3rd August 1987, abstract no. 36893k; M.R. KERNAN et al.: "The luffariellins, novel antiinflammatory sester-terpenes of chemotaxonomic importance from the marine sponge Luffariella variabilis"; & J. ORG. CHEM 1987, 53(14), 3081-3**

(73) Proprietor: **ALLERGAN, INC**
**2525 Dupont Drive**
**Irvine, California 92715-1599(US)**

(72) Inventor: **Bonfiglio, John N.**
**22572 Via Santiago**
**Mission Viejo, CA 92691(US)**
Inventor: **Garst, Michael E.**
**2433 Vista Hogar**
**Newport Beach California 92660(US)**
Inventor: **Lee, Gary C. M.**
**3700 Parkview Lane, Apt. 34D**
**Irvine California 92715(US)**
Inventor: **Tallman, Elizabeth A.**
**4036 Brighton Circle**
**Cypress California 90630(US)**

(74) Representative: **Hutchins, Michael Richard et al**
**Fry, Heath & Spence St. Georges House 6**
**Yattendon Road**
**Horley, Surrey RH6 7BS(GB)**

**EP 0 295 056 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**TETRAHEDRON LETTERS, vol. 26, no 47, 1985, pages 5827-5830; S. KATSUMURA et al.: "Total synthesis of manoalide and seco-manoalide"**

**CHEMICAL ABSTRACTS, vol. 95, no. 19. 9th November 1981, page 772, abstract no. 169484h; F. BOHLMANN et al.: "Naturally occurring terpene derivatives. 315. Seco-eremophilanolides from Senecio macrotis"; & PHYTOCHEMISTRY 1981, 20(5), 1155-7**

## Description

This invention relates to new furanone compounds having anti-inflammatory activity, pharmaceutical compositions comprising these compounds and to methods of using them.

Manoalide is a furanone compound isolated from marine sponge as reported by E.D. de Silva et al., Tetrahedron Letters 21:1611-1614 (1980). Anti-inflammatory, immunosuppressive and analgesic properties of manoalide are disclosed in U.S. Patent 4,447,445. Manoalide has the following structural formula:

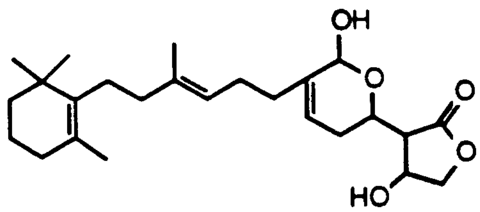

The anti-inflammatory activity of seco-manoalide and dehydro-seco-manoalide is also disclosed in U.S. Patent 4,447,445.

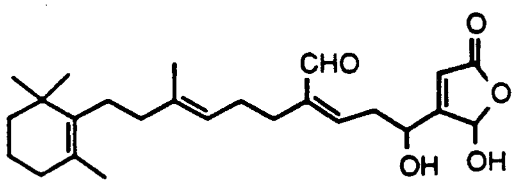

seco-manoalide

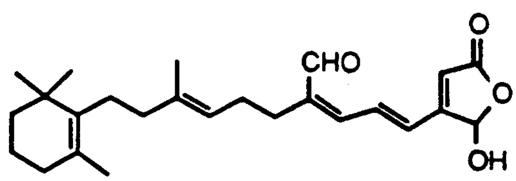

dehydro-seco-manoalide

EP-A-0 209 274 discloses a series of $C_2$-$C_{12}$ hydrocarbyl substituted furanones as phospholipase $A_2$ inhibitors useful as anti inflammatory and antiallergy agents.

The compounds of the present invention are represented by the following formula:

FORMULA I

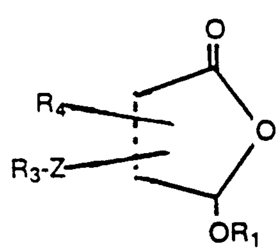

in which:

the dotted line represents a single or double bond;

$R_1$ is hydrogen, $C_1$-$C_4$ alkyl, benzyl or $C_1$-$C_{20}$ alkanoyl, cyclohexanoyl, benzoyl, phenyl ($C_{1-4}$ alkanoyl) or naphthoyl;

Z is -CO- or -C($OR_2$)H-;

$R_2$ is $C_1$-$C_{20}$ alkanoyl, trihaloacetyl, cyclohexanoyl, benzoyl, phenyl($C_{1-4}$ alkanoyl), phenyl($C_2$-$C_{14}$ alkenoyl), naphthoyl, or carbamoyl optionally N-substituted by one or two $C_{1-4}$ alkyl groups or by one α-($C_1$-$C_4$ alkyl)benzyl group;

$R_3$ is hydrogen, $C_1$-$C_{20}$ straight chain alkyl, phenyl($C_1$-$C_{20}$ straight chain alkyl or $C_1$-$C_{20}$ straight chain alkenyl of 1-6 unconjugated double bonds), cyclohexyl($C_1$-$C_{20}$ straight chain alkyl or $C_1$-$C_{20}$ straight chain alkenyl having 1-6 unconjugated double bonds), phenyl, cyclohexyl or benzothienyl($C_1$-$C_{20}$ alkyl or $C_1$-$C_{20}$ alkenyl having 1-6 unconjugated double bonds), 4-methyl-3-pentenyl, 4-methyl-6-(2,6,6-trimethyl-cyclohex-1-enyl)hex-3-enyl, 1-(2-ethenyl)-1,5,9-trimethyl-deca-4,8-dienyl, Y-$(CH_2)_n$ or B-(straight chain $C_3$-$C_{14}$ alkynyl);

$R_4$ is hydrogen, bromo or chloro but is not bromo or chloro when $R_3$ contains a double bond;

n is 6-12;

Y is $OR_5$, $CO_2R_6$, t-butyl, t-butyldimethylsilyl, diphenylmethyl, triphenylmethyl, pyridyl, thienyl, quinolyl, N-$C_1$-$C_4$ alkylpyrrolyl, N-$C_1$-$C_4$ alkylpiperidyl, N-$C_1$-$C_4$ alkylpyridinium halide or naphthyl;

$R_5$ is hydrogen, $C_1$-$C_4$ alkyl or $C_1$-$C_4$ alkanoyl;

$R_6$ is hydrogen or $C_1$-$C_4$ alkyl;

B is hydrogen, phenyl, pyridyl or naphthyl; said phenyl in the definition of $R_3$ being optionally substituted by $C_1$-$C_{14}$ alkyl, $C_1$-$C_{14}$ alkenyl, $C_1$-$C_{14}$ alkynyl or aryl, $CO_2R_6$, $C_1$-$C_4$ alkoxy or halo.

Particular compounds of this invention are represented by Formula I in which:

$R_1$ is hydrogen or $C_1$-$C_{20}$ alkanoyl;

$R_2$ is $C_1$-$C_{20}$ alkanoyl, phenyl ($C_1$-$C_4$ alkanoyl) or carbamoyl optionally N-substituted by one or two $C_1$-$C_4$ alkyl groups or by one α-($C_1$-$C_4$ alkyl)benzyl group;

$R_3$ is $C_5$-$C_{20}$ straight chain alkyl or benzothienyl($C_1$-$C_{20}$ alkyl); and

$R_4$ is hydrogen.

Preferred compounds of this invention are:

4-(1-acetoxy-7-benzo[b]thien-2-yl-heptyl)-5-hydroxy-2(5H)-furanone;

4-(1-acetoxy-6-phenylhexyl)-5-hydroxy-2(5H)-furanone;

4-[1-acetoxy-5-methyl-7-(2,6,6-trimethylcyclohex-1-enyl)-hept-4-enyl]-5-hydroxy-2(5H)-furanone;

4-(1-acetoxytridecyl)-5-hydroxy-2(5H)-furanone and 4-[1-(α-methylbenzylcarbamoyloxy)tridecyl]-5-hydroxy-2(5H)-furanone.

The compounds of this invention contain chiral centers and accordingly, may be prepared as enantiomeric or diasteriomeric mixtures or in optically pure form. Unless otherwise specified herein, the preparations are racemates at each chiral center. However, the scope of the invention is not to be considered as limited to these forms but also to encompass the individual optical isomers of the compounds.

Compounds of the invention are prepared by the following procedures:

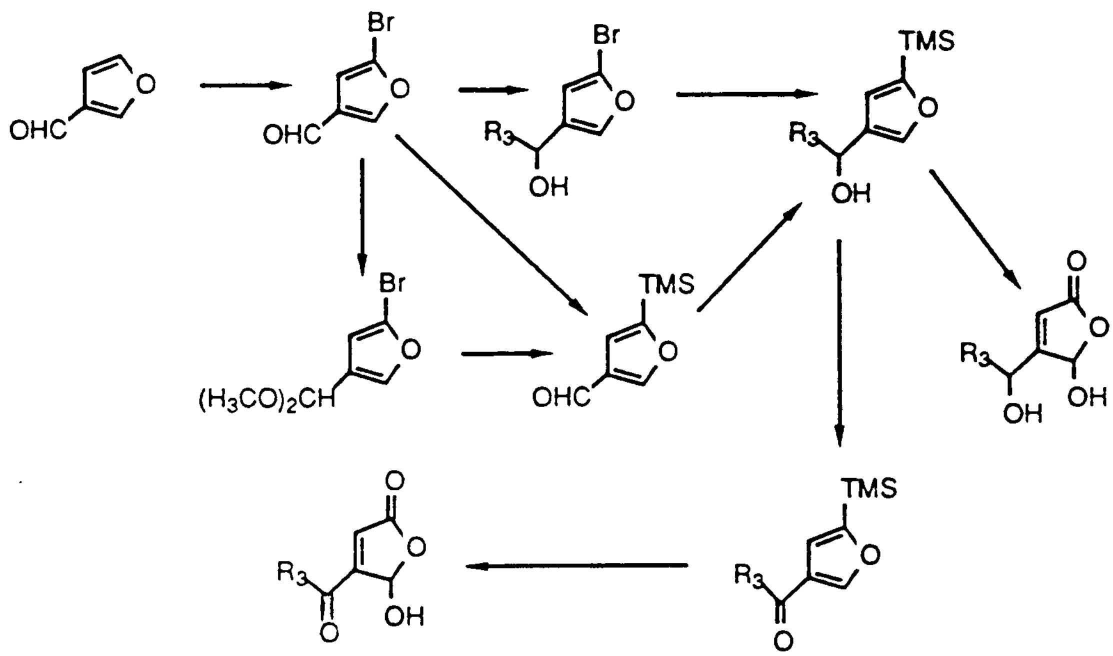

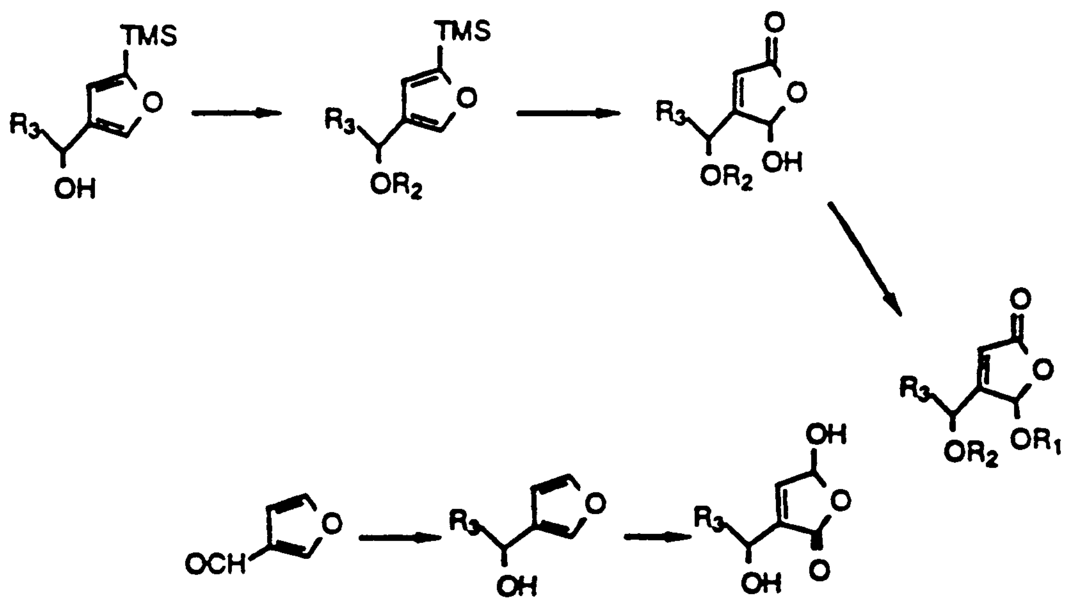

$R_1$, $R_2$ and $R_3$ are as defined hereabove and TMS is trimethylsilyl.

According to the above procedure, 3-furaldehyde is brominated to give 5-bromo-3-furaldehyde which is then reacted with a Grignard reagent ($R_3MgX$) and the resulting mixture treated with t-butyl lithium and trimethylsilyl chloride to give 4-[$R_3$-CH(OH)]-2-trimethylsilylfuran. Alternatively, 5-bromo-3-furaldehyde is converted to the dimethylacetal, then treated with t-butyl lithium and trimethylsilyl chloride to give 5-trimethylsilyl-3-furaldehyde which is reacted with $R_3MgX$ or $R_3Li$ to give 4-[$R_3$-CH(OH)]-2-trimethylsilylfuran.

A preferred method for preparing 5-trimethylsilyl-3-furaldehyde is by reacting lithium morpholide and butyl lithium with 5-bromo-3-furaldehyde to protect the aldehyde group, then reacting with butyl lithium and trimethylsilyl chloride to give 5-trimethylsilyl-3-furaldehyde.

The 4-[$R_3$-CH(OH)]-2-trimethylsilylfuran is reacted with an acyl anhydride or halide to give 4-[$R_3$-CH-($OR_2$)]-2-trimethylsilylfuran. Treatment with oxygen and irradiation using an initiator such as Rose Bengal gives 4-[$R_3$-CH($OR_2$)]-5-hydroxy- 2(5H)-furanone. The corresponding 5-alkanoyl compounds are prepared by reacting the 5-hydroxyfuranone with an acyl anhydride or halide.

The 3-($R_3$-Z)-furanones of Formula I are prepared by reacting 3-furaldehyde with a Grignard reagent

($R_3MgX$) to give 3-[$R_3$-CH(OH)]- furan. This intermediate is treated with oxygen and irradiated using an initiator such as Rose Bengal to give 3-[$R_3$-CH(OH)]-5-hydroxy-2(5H)-furanone and the corresponding 4-[$R_3$-CH(OH)] isomer. These isomers are separated, for example, by chromatography.

The compounds in which $R_1$ is alkyl are prepared by alkylating the corresponding hydroxy compounds by standard procedures, for example, by using an alkyl halide. When $R_1$ and $R_2$ are both hydroxy groups, one may be protected, for example, with an ester group while the other is alkylated (in the case of $R_1$) or acylated.

The compounds in which $R_1$ is benzyl are prepared by standard procedures, for example, by treating the corresponding hydroxy compound with benzyl alcohol with a trace of acid in benzene.

The compounds of Formula I in which $R_4$ is bromo or chloro are prepared by brominating or chlorinating a 5-methoxy-2(5H)-furanone and then dehydrobrominating or dehydrochlorinating the resulting 3,4-dihalo furanone intermediate by standard procedures.

The compounds of Formula I wherein Z is -CO- are prepared by oxidizing the hydroxy group of the 3-$R_3$-CH(OH) furan intermediates using an oxidizing agent such as pyridinium chlorochromate.

In addition, this invention relates to pharmaceutical compositions containing the compounds of Formula I as active ingredients. These compounds are useful in treating inflammation, in suppressing unwanted immune responses and in retarding proliferation of cells. Uses include treatment of rheumatoid arthritis, osteoarthritis, rheumatic carditis and autoimmune diseases such as allergic diseases, bronchial asthma and myasthenia gravis and ocular and dermal inflammatory diseases. The compounds are useful in treating psoriasis, acne, atopic diseases and allergic conjunctivitis. They are also useful as adjuvant therapy associated with organ and tissue transplants.

The activity of the compounds of this invention is demonstrated by inhibition of the enzyme phospholipase $A_2$ in vitro and by reduction of inflammation in the mouse ear anti-inflammatory assay in vivo.

The compounds also inhibit ornithine decarboxylase, a rate limiting enzyme in cellular growth, which indicates use in treating psoriasis and neoplasis.

The compounds also modify calcium homeostasis. This activity is shown by effect on intracellular calcium levels in experiments using gastric glands, spleen cells, epithelial cells, $GH_3$ cells, etc. Calcium is inhibited from entering through the plasma membrane calcium channels and calcium release from intracellular stores is also blocked. Modification of calcium homeostasis is expected to have application in diseases of the nervous system involving modification of membrane lipids or transmitter release (Parkinson's, Alzheimer's), diseases of the cardiovascular system involving application of cardiac or vascular smooth muscle contractility and platelet aggregation (hypertension, cardiac infarction and atherosclerosis), diseases of the gastrointestinal tract such as ulcer disease, diarrhea, motility due to secretion of acid or $Cl^-$, diseases of the kidney involving renal handling of fluid and electrolytes (metabolic acidosis, alkalosis), and disease of abnormal growth (neoplasia, psoriasis).

The compounds of this invention have activity which is similar to that of manoalide, that is the compounds appear to be devoid of the endocrine properties of the glucocorticoids while having anti-inflammatory and immunosuppressive properties. They have activity in substantially the same dose range as manoalide.

In the methods of this invention, the compounds of the invention are administered to mammals, including humans, in an effective amount to produce the desired activity, preferably in an amount of about 0.05 to 100 mg per day per kilogram of body weight. The amount of the compounds depends upon the disease or condition being treated, the severity thereof, the route of administration and the nature of the host. The compounds may be administered topically, orally, parenterally or by other standard routes of administration.

Pharmaceutical compositions of this invention comprise compounds of Formula I and pharmaceutical carriers suitable for the route of administration. Standard methods for formulating pharmaceutical compositions of this type may be found in Remington's Pharmaceutical Sciences, Mack Publishing Company, Easton, PA.

For topical administration, the pharmaceutical composition may be in the form of a salve, cream, ointment, spray, powder or the like. Standard pharmaceutical carriers for such compositions may be used. Preferably, compositions for topical administration will contain 0.05-5% of the active ingredient.

A typical cream formulation may contain the following:

| Ingredient | Parts by Weight |
|---|---|
| Water/glycol mixture (15% or more glycol) | 50-99 |
| Fatty alcohol | 1-20 |
| Non-ionic surfactant | 0-10 |
| Mineral oil | 0-10 |
| Typical pharmaceutical adjuvants | 0-5 |
| Active ingredient | 0.05-5 |

A typical ointment formulation may contain the following:

| Ingredients | Parts by Weight |
|---|---|
| White petrolatum | 40-94 |
| Mineral Oil | 5-20 |
| Glycol solvent | 1-15 |
| Surfactant | 0-10 |
| Stabilizer | 0-10 |
| Active ingredient | 0.05-5 |

For oral administration, suitable pharmaceutical carriers include mannitol, lactose, starch, magnesium stearate, talcum, glucose and magnesium carbonate. Oral compositions may be in the form of tablets, capsules, powders, solutions, suspensions, sustained release formulations, and the like.

A typical tablet or capsule may contain the following:

| Ingredients | Percent w/w |
|---|---|
| Lactose, spray-dried | 40-99 |
| Magnesium stearate | 1-2 |
| Cornstarch | 10-20 |
| Active ingredient | 0.001-20 |

Parenteral compositions are prepared in conventional suspension or solution forms, as emulsions or as solid forms for reconstruction. Suitable carriers are water, saline, dextrose, Hand's solution, Ringer's solution, glycerol, and the like. Parenteral administration is usually by injection which may be subcutaneous, intramuscular or intravenous.

The compounds of this invention may be combined with other known anti-inflammatory/immunosuppressive agents such as steroids or non-steroidal anti-inflammatory agents (NSAID) in the pharmaceutical compositions and methods described herein.

The following examples are intended to illustrate the invention but are not limiting. All temperatures are in degrees Centigrade. NMR data are recorded in delta ppm.

## Example 1

### 5-Bromo-3-furaldehyde, dimethylacetal

To a 250 ml 3 neck round-bottom flask equipped with a reflux condenser and addition funnel, containing a rapidly stirring solution of 3-furaldehyde (2.8 g., 29.44 mmol) in 100 ml of methylene chloride at 0° was added aluminum bromide (11.9 g., 44.22 mmol) under a positive pressure of argon. This mixture was then heated to reflux and bromine (5.30 g., 33.17 mmol) in 20 ml methylene chloride was added dropwise. After one hour the reaction mixture was cooled to room temperature and quenched by pouring over crushed ice. The resulting mixture was filtered through celite and partitioned between methylene chloride and a 5% sodium bicarbonate solution. The organic portion was washed with water, saturated sodium chloride solution, dried over magnesium sulfate, filtered and concentrated to yield the bromoaldehyde as an amber oil. This was immediately diluted with 100 ml of a 0.4 M methanolic solution of cerium chloride heptahydrate. Trimethylorthoformate (32.2 g., 0.303 mol) was then added and the mixture was stirred at room temperature for 3.5 hours. The methanol was evaporated and the residue partitioned between ether

and a 5% sodium bicarbonate solution. The organic portion was washed with saturated sodium chloride solution, dried over magnesium sulfate, filtered and concentrated to give an amber oil. This material was further purified by flash chromatography (5% ethyl acetate/hexane) to give the desired bromoacetal.

$^1$H NMR ($CDCl_3$): 7.43 (s, 1H); 6.32 (s, 1H); 5.36 (s, 1H); 3.30 (s, 3H); 3.29 (s, 3H).

$^{13}$C NMR ($CDCl_3$): 142.4, 126.4, 122.5, 110.4, 97.7, 52.0

5-Trimethylsilyl-3-furaldehyde

To a stirred solution of 5-bromo-3-furaldehyde, dimethyl acetal (1.26 g., 5.7 mmol) in 10 ml dry tetrahydrofuran at 78° under argon was added t-butyllithium (9.69 mmol in pentane), followed by the subsequent addition of trimethylsilyl chloride (1.36 g., 12.54 mmol). After two hours the reaction was quenched with a 10% ammonium chloride solution and the organic portion was washed with 5% sodium bicarbonate, water, saturated sodium chloride solution, dried over magnesium sulfate, filtered and concentrated to give a yellow oil. Purification by flash chromatography (10% ether/pet. ether) yielded the desired aldehyde.

$^1$H NMR ($CDCl_3$): 9.95 (s, 1H); 8.25 (s, 1H); 6.98 (s, 1H); 0.29 (s, 9H).

$^{13}$C NMR ($CDCl_3$): 184.5, 164.1, 155.3, 128.9, 116.2, -2.0.

MS exact mass calculated for $C_8H_{12}O_2Si(M^+)$ 168.0607, found 168.0588.

4-(1-Hydroxy-4-phenylbutyl)-2-trimethylsilylfuran

To a stirred solution of 3-phenylpropylmagnesium bromide (3.3 ml of an 0.313 M solution in ethyl ether, 1.03 mmol, generated from 1-bromo-3-phenylpropane and magnesium) at 0° under argon, was added dropwise 5-trimethylsilyl-3-furaldehyde (0.145 g., 0.862 mmol) in 5 ml ethyl ether. This solution was allowed to warm to room temperature, stirred for one hour, and then poured over crushed ice containing several drops of concentrated sulfuric acid. The resulting mixture was partitioned between ethyl ether and 5% sodium bicarbonate. The organic portion was washed with water, saturated sodium chloride solution, dried over magnesium sulfate, filtered and concentrated to give the desired alcohol.

$^1$H NMR ($CDCl_3$): 7.54 (s, 1H); 7.1-7.3 (m, 5H); 6.59 (s, 1H); 4.67 (t, J = 6.8 Hz, 1H); 2.65 (t, J = 7.5 Hz, 2H); 1.5 to 1.9 (m, 5H); 0.25 (s, 9H).

$^{13}$C NMR ($CDCl_3$): 161.5, 143.1, 142.2, 129.0, 128.4, 128.3, 125.8, 118.2, 66.8, 37.4, 35.6, 27.4, -1.7.

4-(1-Acetoxy-4-phenylbutyl)-2-trimethylsilylfuran

A solution of 4-(1-hydroxy-4-phenylbutyl)-2-trimethylsilylfuran (0.249 g., 0.862 mmol), acetic anhydride 1 ml, excess) and pyridine (3 to 4 drops, excess) was stirred at room temperature until no starting material remained (as monitored by TLC). The reaction mixture was then partitioned between ethyl ether and a 5% ammonium chloride solution. The organic portion was washed repeatedly with saturated sodium bicarbonate solution, twice with aqueous cupric sulfate solution, water, saturated sodium chloride solution, dried over magnesium sulfate, filtered and concentrated to give a pale yellow oil (0.396 g). This material was further purified by flash chromatography (silica, 5% ethyl acetate/hexane) to give the desired acetate.

$^1$H NMR ($CDCl_3$): 7.56 (s, 1H); 7.33 to 7.10 (m, 5H); 6.55 (s, 1H); 5.80 (t, J = 7.1 Hz, 1H); 2.63 (t, J = 7.4 Hz, 2H); 2.03 to 1.55 (m, 4H); 2.03 (s, 3H); 0.24 (s, 9H).

$^{13}$C NMR ($CDCl_3$): 170.4, 161.2, 144.4, 141.9, 128.3, 126.0, 125.8, 124.7, 118.5, 68.3, 35.4, 34.2, 27.2, 21.3, -1.7.

4-(1-Acetoxy-4-phenylbutyl)-5-hydroxy-2(5H)-furanone

A stirred solution of 4-(1-acetoxy-4-phenylbutyl)-2-trimethylsilylfuran (0.065 g., 0.20 mmol) and Rose Bengal (trace) in methanol was flushed with oxygen and cooled to -78°. The solution was subsequently irradiated with a 150 Watt flood lamp while under a constant positive pressure of oxygen until starting material was no longer visible by TLC. The solution was warmed to room temperature, concentrated to a red oil (0.117 g.), and purified by flash chromatography (silica, 30% ethyl acetate/hexane) to give the title hydroxybutenolide.

$^1$H NMR (mixture of diasteriomers) ($CDCl_3$): 7.35 to 7.10 (m, 5H); 6.17 (s, 0.3H); 5.95 (m, 1.7H); 5.51 (m, 1H); 5.33 (broad s, 0.7H); 5.20 (broad s, 0.3H); 2.75 to 2.55 (m, 2H); 2.12 (s, 2H); 2.09 (s, 1H); 2.0 to 1.6 (m, 4H)

$^{13}$C NMR (mixture of diasteriomers) ($CDCl_3$): 171.0 and 170.6, 170.3 and 169.9, 166.8 and 166.4, 141.4

and 141.1, 128.5 and 128.4, 128.3, 126.1 and 126.0, 119.2 and 118.5, 98.0 and 97.8, 69.5 and 69.0, 35.2 and 35.1, 32.4, 26.7 and 26.6, 20.8.

m/z Calculated for $C_{14}H_{14}O_3$ ($M^+$ - HOAc): 230.0943, obtained ($EI^+$): 230.0939.

EXAMPLE 2

4-(1-Hydroxy-5-methyl-4-hexenyl)-2-trimethylsilylfuran

To a stirred solution of 4-methyl-3-pentenylmagnesium bromide (0.74 mmol), prepared from 0.74 mmol 5-bromo-2-methyl-2-pentene and 1.46 mmol magnesium) in two ml anhydrous ethyl ether at 0° under argon was added dropwise 5-trimethylsilyl-3-furaldehyde (0.124 g., 0.74 mmol) in 1.5 ml ethyl ether. This solution was allowed to warm to room temperature, stirred for 30 minutes, and then poured over crushed ice containing several drops of concentrated sulfuric acid. The resulting mixture was partitioned between ethyl ether and 5% sodium bicarbonate. The organic portion was washed with water, saturated sodium chloride, dried over magnesium sulfate, filtered and concentrated to give a colorless oil. This material was purified by flash chromatography (silica, 10% ethyl acetate/hexane) to give the desired alcohol.

$^1H$ NMR ($CDCl_3$): 7.54 (s, 1H); 6.62 (s, 1H); 5.14 (broad t, J=6.5 Hz, 1H); 4.64 (t, J=6.5 Hz, 1H); 2.0 to 2.15 (m, 2H); 1.94 (s, 1H); 1.70 to 1.85 (m, 2H); 1.69 (s, 3H); 1.59 (s, 3H); 0.25 (s, 9H).

$^{13}C$ NMR ($CDCl_3$): 161.3, 143.1, 132.2, 129.0, 123.7, 118.3; 66.5, 37.8, 25.7, 24.3, 17.6, -1.7.

m/z Calculated for $C_{14}H_{24}O_2Si$: 252.1546; obtained ($EI^+$): 252.1544.

4-(1-Acetoxy-5-methyl-4-hexenyl)-2-trimethylsilylfuran

A solution of 4-(1-hydroxy-5-methyl-4-hexenyl)-2-trimethylsilylfuran (0.074 g., 0.293 mmol), acetic anhydride (1 ml, excess), and pyridine (3 to 4 drops, excess) was stirred at room temperature until no starting material remained (as monitored by TLC). The reaction mixture was then partitioned between ethyl ether and a 5% ammonium chloride solution. The organic portion was washed repeatedly with saturated sodium bicarbonate solution, twice with aqueous cupric sulfate solution, water, saturated sodium chloride solution, dried over magnesium sulfate, filtered and concentrated to give a pale yellow oil. This material was further purified by flash chromatography (silica, 8% ether/petroleum ether) to give the desired acetate.

$^1H$ NMR ($CDCl_3$): 7.52 (s, 1H); 6.52 (s, 1H); 5.68 (t, J=6.8 Hz, 1H); 5.02 (broad t, J=6.8 Hz, 1H); 2.0 to 1.65 (m, 4H); 1.96 (s, 3H); 1.60 (s, 3H); 1.48 (s, 3H); 0.17 (s, 9H).

$^{13}C$ NMR ($CDCl_3$): 170.5, 161.2, 144.6, 132.4, 124.8, 123.1, 118.7, 68.1, 34.8, 25.7, 24.1, 21.3, 17.6, -1.7.

m/z Calculated for $C_{16}H_{26}O_3Si$: 294.1651; obtained ($EI^+$): 294.1653.

4-(1-Acetoxy-5-methyl-4-hexenyl)-5-hydroxy-2(5H)-furanone

A stirred solution of 4-(1-acetoxy-5-methyl-4-hexenyl)-2-trimethylsilylfuran (0.072 g., 0.245 mmol) and Rose Bengal (trace) in methanol was flushed with oxygen and cooled to -78°. The solution was subsequently irradiated with a 150 Watt flood lamp while under a constant positive pressure of oxygen until starting material was no longer visible by TLC. The solution was warmed to room temperature, concentrated to a red oil, and purified by flash chromatography (silica, 40% ethyl acetate/hexane) to give the desired hydroxybutenolide.

$^1H$ NMR (mixture of diasteriomers), ($CDCl_3$): 6.15 (d, J=9.4 Hz, 0.3H); 6.0 to 5.85 (m, 1.7H); 5.35 to 5.45 (m, 1H); 5.20 (d, J=10.1 Hz, 0.7H); 4.95 to 5.05 (m, 1.3H); 2.15 to 1.95 (m, 2H); 2.10 (s, 2H); 2.07 (s, 1H); 1.90 to 1.70 (m, 2H); 1.65 (5, 3H); 1.54 (s, 3H).

$^{13}C$ NMR (mixture of diasteriomers), ($CDCl_3$): 171.2 and 170.7, 170.3 and 170.0, 167.1 and 166.6, 133.7 and 133.4, 122.2 and 121.9, 119.1 and 118.3, 98.0 and 97.9, 69.3 and 68.8, 33.1 and 32.9, 25.6, 23.6 and 23.4, 20.8, 18.0 and 17.7.

m/z Calculated for $C_{13}H_{19}O_5$ ($MH^+$): 255.1232; obtained ($CI^+$): 255.1232.

EXAMPLE 3

4-[1-Hydroxy-5-methyl-7-(2,6,6-trimethylcyclohex-1-enyl)-4-heptenyl]-2-trimethylsilylfuran

To a stirred solution of 4-methyl-6-(2,6,6-trimethylcyclohex-1-enyl)-3-hexenylmagnesium bromide (0.70 mmol, prepared from 0.21 g., 0.70 mmol 1-bromo-4-methyl-6-(2,6,6-trimethylcyclohex-1-enyl)-3-hexene and 2.1 mmol magnesium, with a catalytic amount of 1,2-dibromoethane as initiator), in one ml ethyl ether at 0°

under argon was added dropwise 5-trimethylsilyl-3-furaldehyde (0.13 g., 0.77 mmol) in one ml ether. This solution was allowed to warm to room temperature, stirred for 30 minutes, and then poured over crushed ice containing several drops of hydrochloric acid. The resulting mixture was partitioned between ethyl ether and 5% sodium bicarbonate. The organic portion was washed with water, saturated sodium chloride, dried over magnesium sulfate, filtered and concentrated to give a colorless oil. This material was purified by flash chromatography (silica, 20% ethyl ether/hexane) to give the desired alcohol.

$^1$H NMR ($CDCl_3$): 7.57 (s, 1H); 6.63 (s, 1H); 5.18 (t, J = 7.2 Hz, 1H); 4.66 (t, J = 7.2 Hz, 1H); 2.0 to 2.2 (m, 5H); 1.7 to 2.0 (m, 6H); 1.5 to 1.7 (m, 2H); 1.64 (s, 3H); 1.61 (s, 3H); 1.4 to 1.5 (m, 2H); 1.00 (s, 6H); 0.25 (s, 9H).

$^{13}$C NMR ($CDCl_3$): 161.3, 143.1, 137.01, 137.00, 129.0, 126.9, 122.9, 118.3, 66.6, 40.3, 39.8, 37.8 34.9, 32.7, 28.6, 27.9, 24.3, 19.8, 19.5, 16.0, -1.7.

m/z Calculated for $C_{24}H_{40}O_2Si$: 388.2798; obtained ($EI^+$): 388.2783.

4-[1-Acetoxy-5-methyl-7-(2,6,6-trimethylcyclohex-1-enyl)-hept-4-ene]-2-trimethylsilylfuran.

A stirred solution of 4-[1-hydroxy-5-methyl-7-(2,6,6-trimethylcyclohex-1-enyl)-hept-4-ene]-2-trimethylsilylfuran (0.134 g., 0.345 mmol), acetic anhydride (1 ml, excess), and pyridine (3 to 4 drops, excess) was stirred at room temperature until no starting material remained (as monitored by TLC). The reaction mixture was then partitioned between ethyl ether and a 5% ammonium chloride solution. The organic portion was washed repeatedly with saturated sodium bicarbonate solution, twice with aqueous cupric sulfate solution, water, saturated sodium chloride solution, dried over magnesium sulfate, filtered and concentrated to give a pale yellow oil. This material was further purified by flash chromatography (silica, 20% ethyl acetate/hexane) to give the desired acetate.

$^1$H NMR ($CDCl_3$): 7.60 (s, 1H); 6.60 (s, 1H); 5.77 (t, J = 7.2 Hz, 1H); 5.14 (broad t, J = 7.2 Hz, 1H); 2.1 to 1.7 (m, 13H); 1.65 to 1.50 (m, 8H); 1.45 to 1.37 (m, 2H); 1.00 (s, 6H); 0.25 (s, 9H).

$^{13}$C NMR ($CDCl_3$): 170.4, 161.1, 144.5, 137.1, 137.0, 126.9, 124.8, 122.3, 118.6, 68.1, 40.2, 39.8, 34.9, 34.8, 32.7, 32.6, 28.6, 27.8, 24.0, 21.3, 19.8, 19.5, 15.9, -1.7.

m/z Calculated for $C_{24}H_{38}OSi$ ($M^+$ - HOAc): 370.2692; obtained ($EI^+$): 370.2700.

4-[1-Acetoxy-5-methyl-7-(2,6,6-trimethylcyclohex-1-enyl)-hept-4-ene]-5-hydroxy-2(5H)-furanone.

A stirred solution of 4-[1-acetoxy-5-methyl-7-(2,6,6-trimethylcyclohex-1-enyl)-hept-4-ene]-2-trimethylsilylfuran (0.119 g., 0.276 mmol) and Rose Bengal (trace) in methanol was flushed with oxygen and cooled to -78 °C. The solution was subsequently irradiated with a 150 Watt flood lamp while under a constant pressure of oxygen until starting material was no longer visible by TLC. The solution was warmed to room temperature, concentrated to a red oil, and purified by flash chromatography (silica, 30% ethyl acetate/hexane) to give the captioned compound.

$^1$H NMR (mixture of diasteriomers) ($CDCl_3$): 6.2 (s, 0.4H); 6.15 to 5.95 (m, 1.6H); 5.6 to 5.4 (m, 1H); 5.3 (broad s, 1H); 5.2 to 5.0 (m, 1H); 2.3 to 1.75 (m, 13H); 1.75 to 1.3 (m, 10H); 0.99 (s, 6H).

$^{13}$C NMR (mixture of diasteriomers) ($CDCl_3$): 171.1 and 170.7, 170.3 and 170.1, 167.1 and 166.7, 138.2 and 137.9, 136.9 and 136.8, 127.0, 121.5 and 121.2, 119.0 and 118.3, 98.1 and 98.0, 69.4 and 68.9, 40.1, 39.7, 34.9, 33.0, 32.7, 28.5, 27.7 and 27.2, 23.5, 23.4 and 23.3, 20.8, 19.8 and 19.5, 16.0.

m/z Calculated for $C_{21}H_{30}O_3$ ($M^+$ - HOAc): 330.2195; obtained ($EI^+$): 330.2203.

EXAMPLE 4

5-Cyclohexylpentanol

To a solution of 5-phenylpentanol (2.21 g., 13.5 mmol) in ten ml glacial acetic acid was added amorphous platinum oxide (0.40 g., 1.76 mmol, oxygen activated). This mixture was subjected to three atm. of hydrogen with agitation (Parr hydrogenator) at room temperature for four hours. The reaction mixture was diluted with ethyl ether, filtered through celite, washed repeatedly and saturated sodium bicarbonate, followed by water and saturated sodium chloride solution. The organic portion was filtered, dried over magnesium sulfate, filtered and concentrated to give the desired alcohol.

$^1$H NMR ($CDCl_3$): 3.60 (t, J = 7.1 Hz, 2H); 2.30 (s, 1H); 1.75 to 1.8 (m, 19 H).

$^{13}$C NMR ($CDCl_3$): 62.8, 37.6, 37.4, 33.4, 32.7, 26.7, 26.6, 26.4, 26.0.

1-Bromo-5-cyclohexylpentane

To a stirred solution of 5-cyclohexylpentanol (1.74 g., 10.25 mmol) and pyridine (0.94 g., 11.1 mmol) at 0° under argon was added phosphorus tribromide (1.21 g., 4.5 mmol) dropwise. The reaction mixture became viscous, therefore 25 ml tetrahydrofuran was added. This mixture was stirred for 1.5 hours at room temperature, then partitioned between ethyl ether and 5% aqueous hydrochloric acid. The organic portion was washed with a 5% sodium bicarbonate solution, water, saturated sodium chloride solution, dried over magnesium sulfate, filtered and concentrated to give a dark yellow oil. Purification by flash chromatography (silica, hexane) yielded the desired alkyl halide.

$^1$H NMR ($CDCl_3$): 3.39 (t, J=7.1 Hz, 2H); 1.85 (m, 2H); 1.56 to 1.77 (m, 5H); 1.11 to 1.45 (m, 10H); 0.78 to 0.96 (m, 2H).

$^{13}$C NMR ($CDCl_3$): 37.5, 37.2, 33.8, 33.3, 32.8, 28.4, 26.7, 26.4, 25.9.

4-(6-Cyclohexyl-1-hydroxyhexyl)-2-trimethylsilylfuran

To a stirred solution of 5-cyclohexylpentyl- magnesium bromide (1.32 mmol, prepared from 0.31 g., 1.32 mmol 1-bromo-5-cyclohexylpentane and 2.88 mmol magnesium, with a catalytic amount of 1,2-dibromoethane as initiator), in 2.5 ml anhydrous tetrahydrofuran at 0° under argon was added dropwise 5-trimethylsilyl-3-furaldehyde (0.23 g., 1.40 mmol) in 2.5 ml tetrahydrofuran. This solution was allowed to warm to room temperature, stirred for one hour, and then poured over crushed ice containing several drops of concentrated sulfuric acid. The resulting mixture was partitioned between ethyl ether and 5% sodium bicarbonate. The organic portion was washed with water, saturated sodium chloride, dried over magnesium sulfate, filtered and concentrated to give a colorless oil (0.173 g.). This material was purified by flash chromatography (silica, 90% petroleum ether/ethyl ether) to give the desired alcohol.

$^1$H NMR ($CDCl_3$): 7.55 (s, 1H); 6.62 (s, 1H); 4.63 (t, J=7.1 Hz, 1H); 1.85 (s, 1H); 1.5-1.8 (m, 7H); 1.0-1.5 (m, 12H); 0.75 to 0.92 (m, 2H); 0.25 (s, 9H).

m/z Calculated for $C_{19}H_{34}O_2Si$: 322.2328; obtained ($EI^+$): 322.2317.

4-(1-Acetoxy-6-cyclohexylhexyl)-2-trimethylsilylfuran

A stirred solution of 4-(6-cyclohexyl-1-hydroxyhexyl)-2-trimethylsilylfuran (0.065 g., 0.205 mmol), acetic anhydride (1 ml, excess), and pyridine (3 to 4 drops, excess) was stirred at room temperature until no starting material remained (as monitored by TLC). The reaction mixture was then partitioned between ethyl ether and a 5% ammonium chloride solution. The organic portion was washed repeatedly with saturated sodium bicarbonate solution, twice with aqueous cupric sulfate solution, water, saturated sodium chloride solution, dried over magnesium sulfate, filtered and concentrated to give a pale yellow oil. This material was further purified by flash chromatography (silica, 5% ethyl ether/hexane) to give the desired acetate.

$^1$H NMR ($CDCl_3$): 7.59 (s, 1H), 6.59 (s, 1H); 5.76 (t, J=7.2 Hz, 1H); 2.04 (s, 3H); 1.9 to 1.5 (m, 7H); 1.45 to 1.0 (m, 12H); 0.95 to 0.75 (m, 2H); 0.25 (s, 9H).

m/z Calculated for $C_{21}H_{36}O_3Si$: 364.2434; obtained ($EI^+$): 364.2434.

4-(1-Acetoxy-6-cyclohexylhexyl)-5-hydroxy-2(5H)-furanone

A stirred solution of 4-(1-acetoxy-6-cyclohexylhexyl)-2-trimethylsilylfuran (0.072 g., 0.198 mmol) and Rose Bengal (trace) in methanol was flushed with oxygen and cooled to -78°. The solution was subsequently irradiated with a 150 Watt flood lamp while under a constant pressure of oxygen until starting material was no longer visible by TLC. The solution was warmed to room temperature, concentrated to a red oil, and purified by flash chromatography (silica, 40% ethyl ether/hexane) to give the desired hydroxybutenolide.

$^1$H NMR (mixture of diasteriomers) ($CDCl_3$): 6.20 (s, 0.4H); 6.1 to 5.95 (m, 1.6H); 5.55 to 5.45 (m, 1H); 5.35 (broad s, 0.6H); 5.17 (broad s, 0.4H); 2.15 (s, 1.8H), 2.12 (s, 1.2H); 1.95 to 1.55 (m, 7H); 1.55 to 1.05 (m, 12H); 0.95 to 0.75 (m, 2H).

$^{13}$C NMR (mixture of diasteriomers) ($CDCl_3$): 171.2 and 170.8, 170.4 and 170.0, 167.2 and 166.6, 119.1 and 118.3, 98.1 and 98.0, 69.8 and 69.2, 37.5, 37.3, 33.4 33.0, 29.4, 26.7, 26.5, 26.4, 24.9, 20.8.

m/z Calculated for $C_{18}H_{29}O_5$ ($MH^+$): 325.2015; obtained ($EI^+$): 325.2030.

EXAMPLE 5

6-(Benzo[b]thien-2-yl)-1-bromohexane

To a stirred solution of benzo[b]thiophene (1.92 g., 14.3 mmol) in 90 ml anhydrous tetrahydrofuran at -10° to 0° under argon was added n-butyllithium (7.8 ml of a 1.86 M solution in hexane). After two hours, this solution was transferred dropwise into a stirred, 0° solution of 1,6-dibromohexane (6.5 ml, 0.043 mol) in 250 ml tetrahydrofuran. The solution was warmed to room temperature, stirred for 15 hours, quenched with water and concentrated. The residue was acidified and partitioned between ethyl ether and water. The aqueous portion was extracted twice with ether, and the combined organic portions were washed with water, saturated sodium chloride solution, dried over magnesium sulfate, filtered and concentrated to give a brown oil. Excess benzo[b]thiophene and 1,6-dibromohexane were removed by bulb to bulb distillation (85 to 90°/0.1mm) to leave a dark brown syrup. This material was purified by flash chromatography (silica, hexane to 5% ethyl ether/ hexane) to give the desired alkyl halide.

$^{1}$H NMR ($CDCl_3$): 7.77 (d, J=7.6 Hz, 1H); 7.67 (d, J=7.2 Hz, 1H); 7.29 (m, 2H); 7.00 (s, 1H); 3.41 (t, J=6.8 Hz, 2H); 2.92 (t, J=6.5 Hz, 2H); 1.85 (m, 4H); 1.5 (m, 4H).

$^{13}$C NMR ($CDCl_3$): 146.4, 140.2, 139.3, 124.0, 123.4, 122.7, 122.1, 120.5, 33.8, 32.7, 30.9, 30.6, 28.2, 27.9.

m/z Calculated for $C_{14}H_{17}BrS$: 296.0234; obtained ($EI^+$): 296.0220.

4-(7-Benzo[b]thien-2-yl-1-hydroxyheptyl)-2-trimethylsilylfuran.

To a stirred solution of 6-(benzo[b]thien-2-yl)hexylmagnesium bromide (1.22 mmol, prepared from 1.22 mmol 6-(benzo[b]thien-2-yl)-1-bromohexane and 2.44 mmol magnesium, initiated with a catalytic amount of 1,2- dibromoethane) in 1.5 ml tetrahydrofuran at 0° under argon was added dropwise 5-trimethylsilyl-3-furaldehyde (0.167 g., 0.99 mmol) in 1 ml tetrahydrofuran. This solution was allowed to warm to room temperature, stirred for 30 minutes, and then quenched with a 5% ammonium chloride solution. The resulting mixture was partitioned between ethyl ether and 5% sodium bicarbonate. The organic portion was washed with water, saturated sodium chloride, dried over magnesium sulfate, filtered and concentrated to give a pale yellow oil. This material was purified by flash chromatography (silica, 100% hexane to 20% ethyl acetate/hexane) to give the desired alcohol.

$^{1}$H NMR ($CDCl_3$): 7.73 (d, J= 7.7 Hz, 1H); 7.63 (d, J=7.9 Hz, 1H); 7.52 (s, 1H); 7.21 to 7.33 (m, 2H); 6.95 (s, 1H); 6.60 (s, 1H); 4.59 (t, J=6.5 Hz, 1H); 2.86 (t, J=7.4 Hz, 2H); 1.83 (s, 1H); 1.63 to 1.83 (m, 4H); 1.27 to 1.52 (m, 6H); 0.24 (s, 9H).

$^{13}$C NMR ($CDCl_3$): 161.3, 146.6, 143.0, 140.2, 139.2, 129.2, 123.9, 123.3 122.6, 122.0, 120.4, 118.2, 66.8, 37.8, 31.0, 30.7, 29.1, 28.9, 25.6, -1.7.

m/z Calculated for $C_{22}H_{30}O_2SSi$: 386.1736; obtained ($EI^+$): 386.1728.

4-(1-Acetoxy-7-benzo[b]thien-2-ylheptyl)-2-trimethylsilylfuran.

A stirred solution of 4-(7-benzo[b]thien-2-yl-1-hydroxyheptyl)-2-trimethylsilylfuran (0.102 g., 0.246 mmol), acetic anhydride (1 ml, excess), and pyridine (3 to 4 drops, excess) was stirred at room temperature until no starting material remained (as monitored by TLC). The reaction mixture was then partitioned between ethyl ether and a 5% ammonium chloride solution. The organic portion was washed repeatedly with saturated sodium bicarbonate solution, twice with aqueous cupric sulfate solution, water, saturated sodium chloride solution, dried over magnesium sulfate, filtered and concentrated to give a pale yellow oil. This material was further purified by flash chromatography (silica, 5% to 10% ethyl ether/petroleum ether) to give the desired acetate.

$^{1}$H NMR ($CDCl_3$): 7.72 (d, J=8.1 Hz, 1H); 7.63 (d, J=7.5 Hz, 1H); 7.58 (s, 1H); 7.4 to 7.15 (m, 2H); 6.95 (s, 1H); 6.59 (s, 1H); 5.77 (t, J=7.1 Hz, 1H); 2.85 (t, J=7.5 Hz, 2H); 2.02 (s, 3H); 2.0 to 1.65 (m, 4H); 1.5 to 1.2 (m, 6H); 0.24 (s, 9H).

$^{13}$C NMR ($CDCl_3$): 170.3, 161.1, 146.5, 144.4, 140.2, 139.2, 124.9, 123.9, 123.3, 122.6, 122.0, 120.4, 118.6, 68.4, 34.7, 30.9, 30.6, 28.9, 28.8, 25.3, 21.2, -1.7.

m/z Calculated for $C_{24}H_{32}O_3SSi$: 428.1841; obtained ($EI^+$): 428.1843.

4-(1-Acetoxy-7-benzo[b]thien-2-ylheptyl)-5-hydroxy-2(5H)-furanone.

A stirred solution of 4-(1-acetoxy-7-benzo[b]-thien-2-ylheptyl)-2-trimethylsilylfuran (0.097 g., 0.226 mmol) and Rose Bengal (trace) in acetone was flushed with oxygen and cooled to -78°. The solution was subsequently irradiated with a 150 Watt flood lamp while under a constant pressure of oxygen until starting material was no longer visible by TLC. The solution was warmed to room temperature, concentrated to a red oil, and purified by flash chromatography (silica, 50% ethyl acetate/hexane) to give the desired

hydroxybutenolide.

$^1$H NMR ($CDCl_3$): 7.74 (d, J=7.3 Hz, 1H); 7.64 (d, J=7.2 Hz, 1H); 7.4 to 7.15 (m, 2H); 6.2 to 5.9 (m, 2H); 5.7 to 5.3 (m, 2H); 2.87 (t, J=7.2 Hz, 2H); 2.09 (s, 3H); 1.95 to 1.60 (m, 4H); 1.55 to 1.15 (m, 6H).

$^{13}$C NMR ($CDCl_3$): 170.8, 170.2, 166.8, 146.4, 140.1, 139.2, 124.0, 123.3, 122.6, 122.0, 120.5, 118.5, 98.0, 69.4, 32.8, 30.8, 30.6, 28.7, 28.6, 24.7, 20.7.

m/z Calculated for $C_{21}H_{24}O_5S$: 388.1344; obtained ($EI^+$): 388.1354.

EXAMPLE 6

4-(1-Cyclohexyl-1-hydroxymethyl)-2-trimethylsilylfuran.

To a stirred solution of cyclohexylmagnesium chloride (0.24 ml, 0.484 mmol, 2.0 M solution in ethyl ether) under argon at 0°, was added dropwise 5-trimethylsilyl-3-furaldehyde (0.072 g., 0.44 mmol) in 5 ml of dry tetrahydrofuran. This solution was allowed to warm to room temperature, stirred for 30 minutes and poured over crushed ice containing several drops of concentrated sulfuric acid. The resulting mixture was partitioned between ethyl ether and 5% sodium bicarbonate. The organic portion was washed with water, saturated sodium chloride solution, dried over magnesium sulfate, filtered and concentrated to give a colorless oil. This material was purified by flash chromatography (silica, 10% ethyl ether/pentane) to yield the desired alcohol.

$^1$H NMR ($CDCl_3$): 7.51 (s, 1H); 6.58 (s, 1H); 4.34 (d, J=6.7 Hz, 1H); 1.9 to 2.1 (m, 2H); 1.4 to 1.9 (m, 4H); 0.8 to 1.4 (m, 6H); 0.25 (s, 9H).

$^{13}$C NMR ($CDCl_3$): 161.0, 143.6, 127.7, 118.5, 71.6, 44.0, 29.1, 28.7, 26.4, 26.0, 25.9, -1.7.

m/z Calculated for $C_{14}H_{24}O_2Si$: 252.1546; obtained ($EI^+$): 252.1549.

4-(1-Acetoxy-1-cyclohexylmethyl)-2-trimethylsilylfuran.

A solution of 4-(1-cyclohexyl-1-hydroxymethyl)-2-trimethylsilylfuran (0.093 g., 0.37 mmol), acetic anhydride (excess), and pyridine (3 to 4 drops) was stirred at room temperature until no starting material remained (as monitored by TLC). The reaction mixture was then partitioned between ethyl ether and a 5% ammonium chloride solution. The organic portion was washed repeatedly with saturated sodium bicarbonate solution, twice with aqueous cupric sulfate solution, water, saturated sodium chloride solution, dried over magnesium sulfate, filtered and concentrated to give a pale yellow oil. This material was further purified by flash chromatography (silica, 5% ethyl ether/n-pentane) to give the desired acetate.

$^1$H NMR ($CDCl_3$): 7.55 (s, 1H); 6.54 (s, 1H); 5.55 (d, J=7.5 Hz, 1H); 2.04 (s, 3H); 1.9 to 1.5 (m, 6H); 1.30 to 0.85 (m, 5H); 0.25 (s, 9H).

$^{13}$C NMR ($CDCl_3$): 170.4 160.9, 144.7, 123.6, 118.8, 72.7, 41.9, 28.9, 26.3, 25.8, 21.2, -1.7.

m/z Calculated for $C_{16}H_{26}O_3Si$: 294.1651; Obtained ($EI^+$): 294.1656.

4-(1-Acetoxy-1-cyclohexylmethyl)-5-hydroxy-2(5H)-furanone

A stirred solution of 4-(1-acetoxy-1-cyclohexylmethyl)-2-trimethylsilylfuran (0.073 g., 0.248 mmol) and Rose Bengal (trace) in methanol was flushed with oxygen and cooled to -78°. The solution was subsequently irradiated with a 150 Watt flood lamp while under a constant positive pressure of oxygen until starting material was no longer visible by TLC. The solution was warmed to room temperature, concentrated to a red oil, and purified by flash chromatography (silica, 30% ethyl acetate/hexane) to give the desired hydroxybutenolide.

$^1$H NMR (mixture of diasteriomers) ($CDCl_3$): 6.20 (s, 0.03H); 6.1 to 5.9 (m, 1.7H); 5.7 (broad s, 0.7H); 5.6 (broad s, 0.3H); 5.34 (d, J=5.4 Hz, 0.3H); 5.26 (d, J=5.4 Hz, 0.7H); 2.15 (s, 2H); 2.11 (s, 1H); 1.95 to 1.6 (m, 6H); 1.45 to 1.0 (m, 5H).

$^{13}$C NMR (mixture of diasteriomers) ($CDCl_3$): 171.4 and 170.9, 170.3 and 170.0, 166.5 and 165.4, 119.9 and 119.1, 98.54 and 98.46, 73.6 and 73.5, 40.7 and 40.2, 29.4 and 28.9, 27.6 and 27.0, 25.8 and 25.7, 20.7.

m/z Calculated for $C_{13}H_{18}O_5$: 254.1154; obtained ($EI^+$): 254.1177.

EXAMPLE 7

4-(1-Hydroxy-1-phenylmethyl)-2-trimethylsilylfuran.

To a stirred solution of phenylmagnesium bromide (0.41 ml, 1.27 mmol, 3.1 M solution in ethyl ether)

under argon at 0°, was added dropwise 5-trimethylsilyl-3-furaldehyde (0.194 g., 1.15 mmol) in 2 ml tetrahydrofuran. This solution was allowed to warm to room temperature, stirred for 30 minutes, and poured over crushed ice containing several drops of concentrated sulfuric acid. The resulting mixture was partitioned between ethyl ether and 5% sodium bicarbonate. The organic portion was washed with water, saturated sodium chloride solution, dried over magnesium sulfate, filtered and concentrated to give a colorless oil. This material was purified by flash chromatography (silica, 10% ethyl acetate/hexane) to give the desired alcohol.

$^{1}H$ NMR ($CDCl_3$): 7.1 to 7.4 (m, 6H); 6.54 (s, 1H); 5.70 (s, 1H); 2.44 (s, 1H); 0.22 (s, 9H).

$^{13}C$ NMR ($CDCl_3$): 161.5, 144.0, 143.2, 128.8, 128.4, 127.6, 126.3, 118.9, 69.3, -1.7.

m/z Calculated for $C_{14}H_{18}O_2Si$: 246.1076; obtained ($EI^+$): 246.1074.

4-(1-Acetoxy-1-phenylmethyl)-2-trimethylsilylfuran

A stirred solution of 4-(1-hydroxy-1-phenylmethyl)-2-trimethylsilylfuran (0.21 g., 0.852 mmol), acetic anhydride (1 ml, excess), and pyridine (3 to 4 drops, excess) was stirred at room temperature until no starting material remained (as monitored by TLC). The reaction mixture was then partitioned between ethyl ether and a 5% ammonium chloride solution. The organic portion was washed repeatedly with saturated sodium bicarbonate solution, twice with aqueous cupric sulfate solution, water, saturated sodium chloride solution, dried over magnesium sulfate, filtered and concentrated to give a pale yellow oil. This material was further purified by flash chromatography (silica, 8% ethyl ether/n-pentane) to give the desired acetate.

$^{1}H$ NMR ($CDCl_3$): 7.5 to 7.25 (m, 6H); 6.81 (s, 1H); 6.53 (s, 1H); 2.10 (s, 3H); 0.22 (s, 9H).

$^{13}C$ NMR ($CDCl_3$): 170.0, 161.5, 145.0, 139.5, 128.4, 128.0, 126.8, 125.4, 119.2, 70.3, 21.2, -1.8.

m/z Calculated for $C_{16}H_{20}O_3Si$: 288.1182; obtained ($EI^+$): 288.1182.

4-(1-Acetoxy-1-phenylmethyl)-5-hydroxy-2(5H)-furanone.

A stirred solution of 4-(1-acetoxy-1-phenyl-methyl)-2-trimethylsilylfuran (0.15 g., 0.555 mmol) and Rose Bengal (trace) in methanol was flushed with oxygen and cooled to -78°. The solution was subsequently irradiated with a 150 Watt flood lamp while under a constant pressure of oxygen until starting material was no longer visible by TLC. The solution was warmed to room temperature, concentrated to a red oil, and purified by flash chromatography (silica, 30% to 40% ethyl acetate/hexane) to give the desired hydroxybutenolide.

$^{1}H$ NMR (mixture of diasteriomers) ($CDCl_3$): 7.31 (s, 5H); 6.51 to 6.48 (m, 1H); 6.2 to 6.08 (m, 1H); 5.75 to 5.5 (m, 2H); 2.07 (s, 1.5H); 2.05 (s, 1.5H).

$^{13}C$ NMR (mixture of diasteriomers) ($CDCl_3$): 170.6 and 170.4, 170.0 and 169.9, 166.2 and 166.0, 135.6 and 135.0, 129.4 and 129.1, 128.8, 127.6 and 127.3, 120.8 and 117.1, 98.0 and 97.5, 71.8 and 70.6, 20.8.

m/z Calculated for $C_{11}H_8O_3$ ($M^+$ - HOAc): 188.0473; obtained ($EI^+$): 188.0473.

EXAMPLE 8

4-(1-Hydroxypentyl)-2-trimethylsilylfuran.

To a stirred solution of 5-trimethylsilyl-3-furaldehyde (0.134 g., 0.796 mmol) in 15 ml tetrahydrofuran at -78° under argon was added n-butyllithium (0.836 mmol in hexane). The reaction mixture was allowed to warm to room temperature, stirred for 30 minutes and quenched with a 5% ammonium chloride solution. The resulting mixture was partitioned between ethyl ether and 5% sodium bicarbonate solution. The organic portion was washed with water, saturated sodium chloride solution, dried over magnesium sulfate, filtered and concentrated to give the desired alcohol. This material was carried on without further purification.

$^{1}H$ NMR ($CDCl_3$): 7.55 (s, 1H); 6.61 (s, 1H); 4.62 (t, J=7.2 Hz, 1H); 1.65 to 1.95 (m, 3H); 1.15 to 1.45 (m, 4H); 0.70 to 0.98 (m, 3H); 0.24 (s, 9H).

$^{13}C$ NMR ($CDCl_3$): 160, 143, 128,118, 66.3, 36.4, 26.5, 22.0, 18.5, -1.7.

4-(1-Acetoxypentyl)-2-trimethylsilylfuran

A stirred solution of 4-(1-hydroxypentyl)-2-trimethylsilylfuran (approx. 0.80 mmol), acetic anhydride (1 ml, excess), and pyridine (3 to 4 drops, excess) was stirred at room temperature until no starting material remained (as monitored by TLC). The reaction mixture was then partitioned between ethyl ether and a 5% ammonium chloride solution. The organic portion was washed repeatedly with saturated sodium bicarbonate

solution, twice with aqueous cupric sulfate solution, water, saturated sodium chloride solution, dried over magnesium sulfate, filtered and concentrated to give a pale yellow oil. This material was further purified by flash chromatography (silica, 10% ethyl ether/petroleum ether) to give the desired acetate.

$^1$H NMR ($CDCl_3$): 7.59 (s, 1H); 6.58 (s, 1H); 5.77 (t, J = 7.2 Hz, 1H); 2.05 (s, 3H); 1.95 to 1.7 (m, 2H); 1.4 to 1.2 (m, 4H); 0.90 (t, J = 6.8 Hz, 3H); 0.25 (s, 9H).

$^{13}$C NMR ($CDCl_3$): 170.5, 160.8, 144.4, 125.0, 118.6, 68.6, 34.5, 27.7, 22.4, 21.3, 14.0, -1.7.

m/z Calculated for $C_{14}H_{243}O_3Si$: 268.1495; obtained ($EI^+$): 268.1509.

4-(1-Acetoxypentyl)-5-hydroxy-2(5H)-furanone

A stirred solution of 4-(1-acetoxypentyl)-2-trimethylsilylfuran (0.110 g., 0.410 mmol) and Rose Bengal (trace) in acetone was flushed with oxygen and cooled to -78° C. The solution was subsequently irradiated with a 150 Watt flood lamp while under a constant pressure of oxygen until starting material was no longer visible by TLC. The solution was warmed to room temperature, concentrated to a red oil, and purified by flash chromatography (silica, 50% ethyl ether/petroleum ether) to give the desired hydroxybutenolide.

$^1$H NMR (mixture of diasteriomers) ($CDCl_3$): 6.1 (broad s, 1H); 5.98 (s, 1H); 5.72 (broad s, 1H); 5.54 (m, 1H); 2.14 (s, 3H); 1.95 to 1.70 (m, 2H); 1.45 to 1.20 (m, 4H); 1.0 to 0.85 (m, 3H).

$^{13}$C NMR (mixture of diasteriomers) ($CDCl_3$): 170.9, 170.4, 167.0, 118.6 and 118.4, 98.1, 69.6 and 69.5, 32.5, 26.9, 22.2, 20.7, 13.7.

m/z Calculated for $C_{11}H_{17}O_5$ ($MH^+$):229.1076; obtained ($EI^+$): 229.1069.

EXAMPLE 9

4-[(Acetoxy)benzo[b]thien-2-ylmethyl]-5-hydroxy-2(5H)-furanone

To a stirred solution of benzo[b]thiophene (0.193 g., 1.44 mmol) in 7 ml tetrahydrofuran at -20° under argon was added n-butyllithium (1.51 mmol in hexane). After one hour the solution was cooled to -78° C, and 5-trimethylsilyl-3-furaldehyde (0.22 g., 1.31 mmol) was added dropwise in 5 ml tetrahydrofuran. The reaction mixture was allowed to warm to room temperature and then quenched with a 10% ammonium chloride solution. The organic layer was washed with a 5% sodium bicarbonate solution, water, saturated sodium chloride solution, dried over magnesium sulfate, filtered and concentrated to yield the desired alcohol as an unstable, pale yellow oil. The alcohol was treated immediately with acetic anhydride (excess) and pyridine (0.20 g., 2.6 mmol) at room temperature under argon and stirred until TLC showed no remaining starting material. The mixture was partitioned between ethyl ether and a 5% ammonium chloride solution, and the organic portion was washed repeatedly with saturated sodium bicarbonate, twice with 5% aqueous cupric sulfate solution, water, saturated sodium chloride solution, dried over magnesium sulfate, filtered and concentrated to give the acetate (also unstable) as a yellow oil. This material was taken up in oxygenated methanol and cooled to -78°. A catalytic amount of Rose Bengal was added, and while oxygen was continuously being bubbled through the solution it was irradiated with a 150 Watt flood lamp until starting material was no longer visible by TLC. The mixture was warmed to room temperature and concentrated to give a red oil. Purification by flash chromatography (0.173 g. material, silica, 70% ethyl ether/hexane) yielded the hydroxybutenolide.

$^1$H NMR (mixture of diasteriomers) ($CDCl_3$): 7.72 to 7.82 (m, 2H); 7.33 to 7.45 (m, 3H); 6.92 (s, 1H); 6.27 (s, 0.3H); 6.24 (s, 0.7H); 6.07 (s, 0.3H); 5.88 (s, 0.7H); 4.9 to 5.3 (s, 1H); 2.17 (s, 2H); 2.15 (s, 1H).

$^{13}$C NMR (mixture of diasteriomers) ($CDCl_3$): 169.9 and 169.7, 169.8 and 169.6, 164.7 and 164.4, 140.1 and 139.8, 138.7 and 138.1, 137.4 125.5, 125.4 and 125.3, 124.9 and 124.8, 124.4 and 124.2, 122.5 and 122.45, 121.5 and 118.2, 97.5 and 97.3, 67.4 and 66.8, 20.8 and 20.75.

m/z Calculated for $C_{15}H_{12}O_5S$: 304.0405; obtained ($EI^+$): 304.0419.

EXAMPLE 10

5-Bromo-3-furaldehyde

3-Furaldehyde (11.6 g, 121 mmol) was added dropwise to a solution of anhydrous aluminum bromide (48.1 g, 180 mmol) in dry dibromomethane (200 ml). An exothermic reaction ensued and a dark brown color developed. After 10 min, bromine (6.82 ml, 133 mmol) was added dropwise and the mixture was warmed at ca. 50° for 1h. On cooling, the mixture was poured into crushed ice and the organic layer was separated. The aqueous phase was extracted with methylene chloride. All the organic layers were combined, washed

(successively with water, 5% aqueous sodium bicarbonate and brine) and filtered through celite (to assist separation) if necessary. Distillation of the dried (magnesium sulfate) extract gave 5-bromo-3-furaldehyde as a colorless oil: bp 55°/0.25 torr.

$^1H$ NMR ($CDCl_3$): 6.80 (s, 1H), 8.10 (s, 1H) and 9.91 (s, 1H).

4-(1-Hydroxynonyl)-2-trimethylsilylfuran

A mixture of 1-bromooctane (1.33 g, 6.9 mmol) and magnesium turnings (174 mg, 7.3 mmol) in tetrahydrofuran (8 ml) was refluxed under argon for 60 min. After cooling to 0°, a solution of 5-bromo-3-furaldehyde (1.21 g, 6.9 mmol) in THF (1 ml) was added and conditions maintained for 60 min. The mixture was further cooled to -78° and tert-butyl lithium (a 1.7M solution in pentane, 4.26 ml, 7.3 mmol) was added dropwise, followed by chlorotrimethylsilane (2.63 ml, 20.7 mmol) after 1h. Stirring was continued overnight while the cooling bath attained room temperature. The mixture was quenched with saturated aqueous ammonium chloride, diluted with water (15 ml) and extracted with ether. Evaporation of the dried (magnesium sulfate) extract gave a brown oil, which was subjected to flash chromatography on silica using 15% ethyl ether/petroleum ether. Fractions with $R_f$ 0.2 on evaporation gave the captioned compound as a yellow oil.

$^1H$ NMR ($CDCl_3$): 0.32 (s,9H), 0.94 (t, 3H, J=7.3 Hz), 1.33 (broad s, 12H), 1.70 (br, 1H), 1.85 (m, 2H), 4.71 (t, 1H, J=6.8 Hz), 7.32 (s, 1H) and 7.63 (s, 1H).

$^{13}C$ NMR ($CDCl_3$): - 1.70, 14.0, 22.6, 25.4, 25.6, 25.7, 29.2, 29.5, 31.8, 36.8, 37.6, 27.9, 66.7, 66.9, 110.2, 118.3, 129.2, 140.4, 143.4 and 161.2.

MS m/e (% abundance) 282 ($M^+$, 2) 265 (3), 249 (4), 247 (4), 182 (3), 177 (11), 175 (12), 169 (9), 167 (3) and 147 (5); exact mass calculated for $C_{16}H_{30}SiO_2$ 282.2015, found 282.2009.

4-(1-Acetoxynonyl)-2-trimethylsilylfuran

A mixture of 4-(1-hydroxynonyl)-2-trimethylsilylfuran (1.65 g, 5.9 mmol), acetic anhydride (2 ml) and pyridine (3 ml) was stirred under argon at ca. 20° for 17h. After most of the solvent was removed under high vacuum (<40°), the residue was dissolved in ether (40 ml) and washed thoroughly with aqueous copper sulfate and water. Drying (magnesium sulfate) and evaporation gave a brown oil, which was flash chromatography on silica using 5% ethyl ether/petroleum ether. Fractions with $R_f$ 0.32 on evaporation afforded 4-(1-acetoxynonyl)-2-trimethyl-silylfuran as a pale yellow oil.

$^1H$ NMR ($CDCl_3$): 0.26 (s, 9H), 0.89 (t, 3H, J=7.6 Hz), 1.27 (broad s, 12H), 1.90 (m, 2H), 2.06 (s, 3H), 5.78 (t, 1H, J=6.7 Hz), 6.60 (s, 1H) and 7.61 (s, 1H).

$^{13}C$ NMR ($CDCl_3$): - 1.73, 14.0, 21.1, 21.2, 22.6, 25.3, 25.5, 29.2, 29.2, 29.4, 31.8, 34.4, 34.8, 68.0, 68.3, 68.6, 110.5, 118.6, 125.0, 141.8, 144.4, 161.1 and 170.4.

MS m/e (% abundance) 325 ($M^+$+1, 5), 324 (21), 283 (13), 282 (56), 265 (16), 183 (27), 170 (36), 169 (41), 154 (14), 153 (13), 117 (32) and 73 (100); exact mass calculated for $C_{18}H_{32}SiO_3$ 324.2121, found 324.2115.

4-(1-Acetoxynonyl)-5-hydroxy-2(5H)-furanone

A mixture of 4-(1-acetoxynonyl)-2-trimethylsilylfuran (323 mg, 0.9 mmol) and Rose Bengal (5 mg) in THF (10 ml) was exposed to singlet oxygen for 5.5h at -78°. The residue, after solvent removal was flash chromato- graphed on silica using 60% ethyl ether/petroleum ether. Fractions with $R_f$ 0.13 on evaporation afforded the captioned compound as colorless prisms.

mp 54-5°.

$^1H$ NMR ($CDCl_3$): 0.96 (t, 3H, J=6.7 Hz), 1.34 (broad s, 12H), 1.89 (m, 2H), 2.20 (s, 3H), 2.21 (s, 3H), 4.54 (d, 1H, J=7.6 Hz, exchanged with $D_2O$), 5.12 (d, 1H, J=10.5 Hz, exchanged with $D_2O$), 5.47 (t, 1H, J=6.3 Hz), 5.55 (t, 1H, J=6.3 Hz), 6.06 (2d + s) and 6.26 (d, 1H, J=7.3 Hz).

$^{13}C$ NMR ($CDCl_3$): 14.0, 20.8, 22.6, 24.9, 25.0, 29.1, 29.3, 31.7, 32.9, 33.0, 69.3, 69.8, 98.0, 98.2, 118.3, 119.0, 166.7, 167.1, 170.0 and 171.1.

MS m/e: exact mass cald for $C_{15}H_{24}O_5$ 284.1624, found 284.1691.

EXAMPLE 11

5-Acetoxy-4-(1-acetoxynonyl)-2(5H)-furanone

A mixture of 4-(1-acetoxynonyl)-5-hydroxy-2(5H)-furanone (77.3 mg, 0.27 mmol), acetic anhydride (1/2 ml) and pyridine (1 ml) was stirred under argon at ca. 20° for 20h. After most of the solvent was removed under high vacuum (<40°), the residue was dissolved in ether (20 ml) and washed thoroughly with aqueous copper sulfate and water. Drying (magnesium sulfate) and evaporation gave a brown oil, which was flash chromatographed on silica using 30% ethyl ether/petroleum ether. Fractions with $R_f$ 0.22 on evaporation afforded the title compound as a light yellow oil.

$^1H$ NMR ($CDCl_3$): 0.92 (t, 3H, J=7.4 Hz), 1.31 (broad s, 12H), 1.83 (m, 2H), 2.13 - 2.24 (4s, 6H), 5.58 (t, 1H, J=7.5 Hz), 5.69 (t, 1H, J=7.5 Hz), 6.11 (s, 1H), 6.14 (s, 1H), 6.95 (s, 1H) and 7.04 (s, 1H).

$^{13}C$ NMR ($CDCl_3$): 14.0, 20.6, 22.5, 24.7, 29.1, 29.2, 31.7, 32.6, 33.1, 68.0, 69.1, 92.3, 92.6, 119.2, 120.0, 164.5, 164.6, 168.7, 168.8, 169.7 and 170.0.

MS m/e: exact mass cald for $C_{17}H_{27}O_6$ $(M+H)^+$ 326.1808, found 326.1804.

EXAMPLE 12

4-(1-Acetoxynonyl)-5-octanoyloxy-2(5H)-furanone.

Octanoyl chloride (83.6 µl, 0.49 mmol), followed by triethylamine (68.2 µl, 0.49 mmol), was added to 4-(1-acetoxynonyl)-5-hydroxy-2(5H)-furanone (132.6 mg, 0.47 mmol) in tetrahydrofuran (6 ml) at 0°. Stirring was continued for 14h while the ice bath attained room temperature. The mixture was diluted with water (10 ml) and extracted with dichloromethane. Evaporation of the dried (magnesium sulfate) extract gave a yellow oil, which was flash chromatographed on silica using 30% ethyl ether/ petroleum ether. Fractions with $R_f$ 0.38 on evaporation gave the title compound as a colorless oil.

$^1H$ NMR ($CDCl_3$): 0.95 (t, 3H, J=5.7 Hz), 1.30 (broad s, 20H), 1.7 (m, 4H), 2.16 (s, 3H), 2.45 (t, 2H, J=7.2 Hz), 5.68 (t, 1H, J=5.6 Hz), 6.11 (s, 1H), 6.96 (s, 1 H).

$^{13}C$ NMR ($CDCl_3$): 13.9, 20.5, 22.4, 24.3, 24.4, 24.6, 24.7, 28.7, 28.8, 29.0, 29.2, 31.5, 31.7, 32.6, 33.1, 33.8, 68.0, 69.1, 92.2, 92.5, 119.0, 119.9, 164.6, 168.7, 169.6 and 171.5.

MS m/e: exact mass cald for $C_{23}H_{38}O_6$ 410.2668, found 410.2674.

EXAMPLE 13

4-(1-Hydroxyundecyl)-2-trimethylsilylfuran

A mixture of 1-bromodecane (2.83 g, 13 mmol) and magnesium turnings (322 mg, 13.5 mmol) in THF (10 ml) was refluxed under argon for 60 min. After cooling to 0°, a solution of 5-bromo-3-furaldehyde (2.24 g, 13 mmol) in tetrahydrofuran (3 ml) was added and conditions maintained for 20 min. The mixture was further cooled to -78° and tert-butyl lithium (a 1.7M solution in pentane; 9.04 ml, 15.4 mmol) was added dropwise, followed by chlorotrimethylsilane (4.88 ml, 38.4 mmol) after 20 min. Stirring was continued overnight (12h) while the cooling bath attained room temperature. The mixture was quenched with saturated aqueous ammonium chloride, diluted with water (15 ml) and extracted with ether. Evaporation of the dried (magnesium sulfate) extract gave a brown oil, which was flash chromatographed on silica using 15% ethyl ether/petroleum ether. Fractions with about $R_f$ 0.22 on evaporation gave the title compound as a yellow oil.

$^1H$ NMR ($CDCl_3$): 0.26 (s, 9H),0.89 (t, 3H, J=7.3 Hz), 1.27 (broad s, 16H), 1.62 (br, 1H), 1.75 (m, 2H), 4.65 (t, 1H, J = 6.8 Hz), 6.63 (s, 1H) and 7.57 (s, 1H).

MS m/e (% abundance) 293 ($M^+$-OH, 2), 221 (5), 177 (31), 175 (33), 97 (14), 87 (10), 85 (64), 83 (100), 73 (14) and 57 (12).

4-(1-Acetoxyundecyl)-2-trimethylsilylfuran

A mixture of 4-(1-hydroxyundecyl)-2-trimethylsilylfuran (1.21 g, 3.89 mmol), acetic anhydride (4 ml) and pyridine (6 ml) was stirred under argon at ca. 20° for 14h. After most of the solvent was removed under high vacuum (<40°), the residue was dissolved in ether (40 ml) and washed thoroughly with aqueous copper sulfate and water. Drying (magnesium sulfate) and evaporation gave a brown oil, which was flash chromatographed on silica using 5% ethyl ether/petroleum ether. Fractions with $R_f$ of about 0.3 on evaporation afforded the captioned compound as a pale yellow oil.

$^1H$ NMR ($CDCl_3$): 0.26 (s, 9H), 0.89 (t, 3H, J=7.6 Hz), 1.26 (broad s, 16H), 1.85 (m, 2H), 2.06 (s, 3H), 5.77 (t, 1H, J=6.8 Hz), 6.59 (s, 1H) and 7.60 (s, 1H).

MS m/e (% abundance) 353 ($M^+$ + 1, 9), 352 (33), 311 (23), 310 (90), 293 (12), 292 (12), 183 (28), 170 (38), 169 (39), 154 (20), 153 (11), 117 (30) and 73 (100).

4-(1-Acetoxyundecyl)-5-hydroxy-2(5H)-furanone

A mixture 4-(1-acetoxyundecyl)-2-trimethylsilylfuran (290.8 mg, 0.83 mmol) and Rose Bengal (5 mg) in tetrahydrofuran (7 ml) was exposed to singlet oxygen for 3.5h at -78°. The residue, after solvent removal, was flash chromatographed on silica using 40% ethyl ether/ petroleum ether. Fractions with $R_f$ of about 0.26 (60% ethyl ether/petroleum ether) on evaporation gave the title furanone as colorless prisms.

mp 59-60°.

$^1$H NMR ($CDCl_3$): 0.91 (t, 3H, J=7.1 Hz), 1.29 (broad s, 16H), 1.83 (m, 2H), 2.15 (s, 3H), 2.17 (s, 3H), 4.80 (br, 1H), 5.36 (t, 1H, J = 6.8 Hz), 5.50 (t, 1H, J=6.8 Hz), 6.02 (s, 2H), 6.03 (s, 1H) and 6.22 (s, 1H).

$^{13}$C NMR ($CDCl_3$): 14.1, 20.8, 22.6, 24.9, 25.1, 29.1, 29.3, 29.3, 29.5, 32.9, 33.1, 69.2, 69.8, 98.0, 98.1, 118.4, 119.1, 166.8, 167.2, 170.0, 170.8 and 171.2.

MS m/e: exact mass calculated for $C_{17}H_{28}O_5$ 312.2015, found 312.2025

EXAMPLE 14

4-(1-Hydroxytridecyl)-2-trimethylsilylfuran

A mixture of 1-bromododecane (3.45 g, 14 mmol) and magnesium turnings (349 mg, 14.5 mmol) in tetrahydrofuran (10 ml) was refluxed under argon for 1h. After cooling to 0°, a solution of 5-bromo-3-furaldehyde (2.42 g, 14 mmol) in tetrahydrofuran (3 ml) was added and conditions maintained for 20 min. The mixture was further cooled to -78° and tert-butyl lithium (a 1.7M solution in pentane: 9.77 ml, 1.67 mmol) was added dropwise, followed by chlorotrimethylsilane (5.27 ml, 41.5 mmol) after 20 min. Stirring was continued overnight (12h) while the cooling bath attained room temperature. The mixture was quenched with saturated aqueous ammonium chloride, diluted with water (15 ml) and extracted with ethyl ether. Evaporation of the dried (magnesium sulfate) extract gave a brown oil, which was flash chromatographed on silica using 15% ethyl ether/petroleum ether. Fractions with $R_f$ of about 0.25 on evaporation afforded the title trimethylsilylfuran as a pale yellow oil.

$^1$H NMR ($CDCl_3$): 0.26 (s, 9H), 0.91 (t, 3H, J=6.7 Hz), 1.29 (broad s, 20H), 1.64 (br, 1H), 1.77 (m, 2H), 4.67 (t, 1H, J=6.8 Hz), 6.65 (s, 1H) and 7.59 (s, 1H).

MS m/e (% abundance) 339 ($m^+$ + 1, 9), 338 (31), 170 (35), 169 (100), 75 (15) and 73 (50).

4-(1-Acetoxytridecyl)-2-trimethylsilylfuran

A mixture of 4-(1-hydroxytridecyl)-2-trimethylsilylfuran (1.32 g, 3.89 mmol), acetic anhydride (4 ml) and pyridine (6 ml) was stirred under argon at ca. 20° for 16 h. After most of the solvent was removed under high vacuum (<40°), the residue was dissolved in ethyl ether (40 ml) and washed thoroughly with aqueous copper sulfate and water. Drying (magnesium sulfate) and evaporation gave a brown oil, which was flash chromatographed on silica using 5% ethyl ether/petroleum ether. Fractions with $R_f$ of about 0.55 (10% ethyl ether/petroleum ether) on evaporation gave the desired trimethylsilylfuran as a pale yellow oil.

$^1$H NMR ($CDCl_3$): 0.29 (s, 9H), 0.93 (t, 3H, J=6.8 Hz), 1.30 (broad s, 20H), 1.90 (m, 2H), 2.09 (s, 3H), 5.81 (t, 1H, J=6.8 Hz), 6.63 (s, 1H) and 7.64 (s, 1H).

MS m/e (% abundance) 381 ($M^+$ + 1, 13), 380 (42), 346 (11), 339 (28), 338 (100), 321 (29), 320 (17), 183 (23), 170 (36), 169 (29), 154 (26), 153 (11), 117 (27), 75 (23) and 73 (90).

4-(1-Acetoxytridecyl)-5-hydroxy-2(5H)-furanone

A mixture of 4-(1-acetoxytridecyl)-2-trimethylsilylfuran (314.2 mg, 0.83 mmol) and Rose Bengal (5 mg) in tetrahydrofuran (7 ml) was exposed to singlet oxygen for 2.5 hrs. at -78°. The residue, after solvent removal, was flash chromatographed on silica using 45% ethyl ether/ petroleum ether. Fractions with $R_f$ of about 0.21 (60% ethyl ether/petroleum ether) on evaporation afforded the 4-(1-acetoxytridecyl)-5-hydroxy-2-(5H)-furanone as colorless prisms: mp 67-8°.

$^1$H NMR ($CDCl_3$): 0.88 (t, 3H, J=7.5 Hz), 1.26 (broad s, 20H), 1.82 (m, 2H), 2.11 (s, 3H), 2.14 (s, 3H), 4.06 (broad d, 1H, exchanged with $D_2O$), 4.86 (broad d, 1H, exchanged with $D_2O$), 5.36 (t, 1H, J=5.6 Hz), 5.50 (t, 1H, J=5.6 Hz), 5.95 (s, 1H), 5.99 (s, 1H), 6.00 (d, 1H, J=10 Hz) and 6.19 (d, 1H, H=7.5 Hz).

$^{13}$C NMR ($CDCl_3$): 14.1, 20.8, 22.7, 25.0, 25.1, 29.2, 29.3, 29.3, 29.5, 31.9, 33.0, 33.2, 69.2, 69.8, 98.0, 118.5, 119.2, 167.1, 169.8, 170.7 and 171.2.

MS m/e: exact mass calculated for $C_{19}H_{36}O_5N$ $(M+NH_4)^+$ 358.2593, found 358.2597.

EXAMPLE 15

4-(1-Acetoxy-4-phenylbutyl)-5-methoxy-2(5H)-furanone

A solution of 4-(1-acetoxy-4-phenylbutyl)-5-hydroxy-2(5H)-furanone (0.020 g.,0.069 mmol) and p-toluene sulfonic acid (approx. 3 mg.) in 2.5 ml methanol was stirred for 72 hours at room temperature. The reaction mixture was concentrated and partitioned between ethyl ether and water. The organic portion was washed with saturated sodium chloride solution, dried over magnesium sulfate, filtered and concentrated to give an oil (0.027 g.) This material was purified by flash chromatography (30% ethyl acetate/hexane) to give a diasteriomeric mixture of the two methoxybutenolides. The mixture was subsequently separated by semi-preparative HPLC on a Whatman Partisil M-9 silica column, with an elution rate of 4 ml/min with 20% ethyl acetate/hexane. One diasteriomer (A) was eluted at 11 minutes, followed by the other (B) at 12 minutes. Concentration gave the purified compounds.

Diasteriomer (A). - $^1$H NMR ($CDCl_3$): 7.4 to 7.1 (m, 5H); 5.97 (s, 1H); 5.69 (m, 1H); 5.57 (s, 1H); 3.50 (s, 3H); 2.55 to 2.75 (m, 2H); 2.13 (s, 3H); 1.5 to 1.9 (m, 4H).

$^{13}$C NMR ($CDCl_3$): 169.8, 169.3, 164.4, 141.2, 128.5, 128.4, 126.1, 119.2, 102.6, 69.2, 56.7, 35.1, 31.8, 26.3, 20.8

Diasteriomer (B). - $^1$H NMR ($CDCl_3$): 7.35 to 7.1 (m, 5H); 5.98 (s, 1H); 5.78 (s, 1H); 5.44 (broad t, J = 6.7 Hz, 1H); 3.56 (s, 3H); 2.7 to 2.55 (m, 2H); 2.09 (s, 3H); 1.95 to 1.5 (m, 4H).

$^{13}$C NMR ($CDCl_3$): 170.1, 169.4, 164.7, 141.4, 128.41, 128.36, 126.0, 119.7, 103.7, 68.7, 57.8, 35.2, 32.3, 26.7, 20.7.

m/z calculated for $C_{17}H_{21}O_5$ ($MH^+$) = 305.1389, obtained ($CI^+$) = 305.1395.

EXAMPLE 16

4-(1-Dodecoyloxy-4-phenylbutyl)-2-trimethylsilylfuran

To a stirred solution of 3-phenylpropylmagnesium bromide (2.28 ml of a 0.6 M solution in tetrahydrofuran, 1.37 mmol, generated from 1-bromo-3-phenylpropane and magnesium) at 0° under argon, was added dropwise 5-trimethylsilyl-3-furaldehyde (0.20 g., 1.19 mmol) in two ml tetrahydrofuran. This solution was warmed to room temperature and stirred for thirty minutes, then cooled again to 0°. Lauroyl chloride (0.296 g., 1.35 mmol) was added dropwise and the reaction mixture was warmed to room temperature and partitioned between ethyl ether and a 5% sodium bicarbonate solution. The organic portion was washed with water, saturated sodium chloride solution, dried over magnesium sulfate, filtered and concentrated to give an oil (0.844 g.). Purification by flash chromatography (silica, 5% ethyl ether/petroleum ether) yielded the desired furan ester.

$^1$H NMR ($CDCl_3$): 7.55 (s, 1H); 7.3 to 7.1 (m, 5H); 6.54 (s, 1H); 5.82 (t, J = 6.8 Hz, 1H); 2.63 (t, J = 7.5 Hz, 2H); 2.27 (t, J = 7.5 Hz, 2H); 2.0 to 1.5 (m, 6H); 1.45 to 1.15 (m, 16H); 0.88 (t, J = 6.2 Hz, 3H); 0.24 (s, 9H).

$^{13}$C NMR ($CDCl_3$): 173.1, 161.1, 144.3, 141.8, 128.29, 128.25, 125.8, 124.9, 118.5, 68.0, 35.3, 34.5, 34.3, 31.9, 29.6, 29.4, 29.3, 29.2, 29.0, 27.2, 25.0, 22.6, 14.1, -1.7.

m/z Calculated for $C_{29}H_{46}O_3Si$: 470.3216. obtained ($EI^+$): 470.3202

4-(1-Dodecoyloxy-4-phenylbutyl)-5-hydroxy-2(5H)-furanone

A stirred solution of 4-(1-dodecoyloxy-4-phenylbutyl)-2-trimethylsilylfuran (0.563 g., 1.19 mmol) and Rose Bengal (trace) in acetone was flushed with oxygen and cooled to -78°. The solution was subsequently irradiated with a 150 Watt flood lamp while under a constant, positive pressure of oxygen until starting material was no longer visible by TLC. The solution was warmed to room temperature, concentrated to a red oil, and purified by flash chromatography (silica, 40% ethyl ether/petroleum ether) to give the desired hydroxybutenolide.

$^1$H NMR (mixture of diasteriomers) ($CDCl_3$): 7.05 to 7.3 (m, 5H); 6.17 (s, 0.4H); 5.95 (broad s, 0.4H); 5.91 (m, 1.6H); 5.75 (broad s, 0.6H); 5.60 (m, 0.6H); 5.52 (m, 0.4H); 2.7 to 2.5 (m, 2H); 2.25 to 2.4 (m, 2H); 2.0 to 1.5 (m, 6H); 1.4 to 1.15 (m, 16H), 0.86 (t, J = 6.6 Hz, 3H).

$^{13}$C NMR (mixture of diasteriomers) ($CDCl_3$): 173.4 and 173.3, 170.7 and 170.4, 167.0 and 166.8, 141.4 and 141.1, 128.3, 128.2, 125.9 and 125.8, 118.7 and 118.1, 98.2 and 97.8, 69.1 and 68.7, 35.1 and 35.0, 34.0, 32.2 and 32.1, 31.7, 29.4, 29.3, 29.2, 29.1, 29.0, 26.6 and 26.4, 24.7, 22.5 and 14.0.

m/z Calculated for $C_{26}H_{38}O_5$: 430.2719, obtained ($EI^+$): 430.2707.

EXAMPLE 17

4-(1-Hydroxynonadecyl)-2-trimethylsilylfuran.

To a stirred solution of octadecylmagnesium bromide (10.2 ml of a 0.34 M solution in ethyl ether, 3.46 mmol, generated from 1-bromooctadecane and magnesium using iodine as an initiator) at 0° under argon, was added dropwise 5-trimethylsilyl-3-furaldehyde (0.265 g., 1.57 mmol) in 2 ml tetrahydrofuran. This solution was allowed to warm to room temperature, stirred for 30 minutes, and quenched with a 5% ammonium chloride solution. The resulting mixture was partitioned between ethyl ether and 5% sodium bicarbonate. The organic portion was washed with 10% sodium bisulfite, water, saturated sodium chloride solution, dried over magnesium sulfate, filtered and concentrated to give a residue. This was taken up in ethyl ether and filtered. The ether portion was concentrated to give a waxy residue which was purified by flash chromatography (silica, 10% ethyl ether/petroleum ether) to give the desired alcohol.

$^1$H NMR ($CDCl_3$): 7.56 (s, 1H); 6.62 (s, 1H); 4.64 (t, J=6.7 Hz, 1H); 1.7 to 1.8 (m, 2H); 1.64 (broad s, 1H); 1.2 to 1.4 (m, 32 H); 0.88 (t, J=6.7 Hz, 3H); 0.25 (s, 9H).

$^{13}$C NMR ($CDCl_3$): 161.4, 143.1, 129.2, 118.3, 67.0, 37.9, 31.9, multiple peaks from 29.7 to 29.3, 25.8, 22.7, 14.1, -1.7.

m/z Calculated for $C_{26}N_{50}O_2Si$: 422.3580, obtained ($EI^+$): 422.3583.

4-(1-Acetoxynonadecyl)-2-trimethylsilylfuran

A solution of 4-(1-hydroxynonadecyl)-2-trimethylsilylfuran (0.294 g., 0.697 mmol), acetic anhydride (3 ml, excess) and pyridine (0.064 g., 0.761 mmol) was stirred at room temperature until no starting material remained (as monitored by TLC). The reaction mixture was then concentrated under high vacuum for 6 hours to give a yellow oil. This material was purified by flash chromatography (silica, 2.5% ethyl ether/petroleum ether) to give the desired acetate.

$^1$H NMR ($CDCl_3$): 7.58 (s, 1H); 6.59 (s, 1H); 5.77 (t, J=7.2 Hz, 1H); 2.03 (s, 3H); 1.7 to 1.95 (m, 2H); 1.2 to 1.4 (m, 32 H); 0.88 (t, J=6.5 Hz, 3H); 0.25 (s, 9H).

$^{13}$C NMR ($CDCl_3$): 170.4, 161.0, 144.4, 124.9, 118.6, 68.5, 34.7, 31.9, multiple peaks from 29.7 to 29.3, 25.5, 22.7, 21.2, 14.1, -1.7.

m/z Calculated for $C_{28}N_{52}O_3Si$: 464.3686, obtained ($EI^+$): 464.3682.

4-(1-Acetoxynonadecyl)-5-hydroxy-2(5H)-furanone.

A stirred solution of 4-(1-acetoxynonadecyl)-2-trimethylsilylfuran (0.240 g., 0.517 mmol) and Rose Bengal (trace) in 250 ml acetone was flushed with oxygen and cooled to -78°. The solution was subsequently irradiated with a 150 Watt flood lamp while under a constant positive pressure of oxygen until starting material was no longer visible by TLC. The solution was warmed to room temperature, concentrated to a red oil and the residue was purified by flash chromatography (silica, ethyl ether) to give the desired hydroxybutenolide.

$^1$N NMR (mixture of diasteriomers) ($CDCl_3$): 6.2 (broad s, 0.4H); 5.95 to 6.05 (m, 1.6H); 5.4 to 5.5 (m, 1H); 5.2 to 5.3 (m, 0.6H); 5.0 (broad s, 0.4H); 2.14 (s, 1.8H); 2.11 (s, 1.2H); 1.75 to 1.9 (m, 2H); 1.2 to 1.45 (m, 32H); 0.88 (m, 3H).

$^{13}$C NMR (mixture of diasteriomers) ($CDCl_3$): 171.2 and 170.7, 170.1 and 169.9, 167.2 and 166.5, 119.1 and 118.4, 98.0, 69.7 and 69.2, 33.1, 33.0, 31.9, multiple peaks between 29.7 and 29.2, 25.1, 25.0, 22.7, 20.8, 14.1.

m/z Calculated for $C_{25}H_{48}NO_5$ $(M + NH_4)^+$: 442.3532, obtained ($CI^+$): 442.3546.

EXAMPLE 18

4-(1-Hydroxymethyl)-2-trimethylsilylfuran.

To a stirred suspension of lithium aluminum hydride (0.01 g., 0.263 mmol) in 1 ml dry tetrahydrofuran at 0° under argon was added dropwise 5-trimethylsilyl-3-furaldehyde (0.09 g., 0.535 mmol). This mixture was allowed to warm to room temperature, stirred for 15 minutes, quenched with a 5% ammonium chloride solution, and extracted twice with ethyl ether. The organic portion was washed with 5% sodium bicarbonate, water, saturated sodium chloride solution, dried over magnesium sulfate, filtered and concentrated to give the desired alcohol. This material was used without further purification.

$^1$H NMR ($CDCl_3$): 7.57 (s, 1H); 6.64 (s, 1H); 4.50 (s, 2H); 2.75 (broad s, 1H); 0.25 (s, 9H).
$^{13}$C NMR ($CDCl_3$): 161.5, 144.0, 125.0, 119.7, 56.2, -1.8.
m/z Calculated for $C_6H_{14}O_2Si$: 170.0763, obtained ($EI^+$): 170.0766.

4-Dodecoyloxymethyl-2-trimethylsilylfuran.

To a stirred solution of 4-hydroxymethyl-2-trimethylsilylfuran (0.288 g., 1.70 mmol) and pyridine (0.214 g., 2.55 mmol) in 30 ml dry tetrahydrofuran at 0° was added lauroyl chloride (0.408 g., 1.86 mmol). This solution was allowed to warm to room temperature, stirred 30 minutes, and partitioned between ethyl ether and 5% sodium bicarbonate solution. The organic portion was washed with aqueous cupric sulfate solution, water, saturated sodium chloride solution, dried over magnesium sulfate, filtered and concentrated to a colorless oil. This material was purified by flash chromatography (silica, 5% ethyl ether/petroleum ether) to give the desired ester.

$^1$H NMR ($CDCl_3$): 7.64 (s, 1H); 6.63 (s, 1H); 4.97 (s, 2H); 2.31 (t, J=7.3 Hz, 2H); 1.55 to 1.7 (m, 2H); 1.2 to 1.4 (m, 16H); 0.89 (t, J=6.3 Hz, 3H); 0.25 (s, 9H).

$^{13}$C NMR ($CDCl_3$): 173.5, 161.4, 145.6, 120.3, 57.4, 34.2, 31.8, 29.5, 29.4, 29.3, 29.2, 29.0, 24.9, 22.6, 14.0, -1.8.

m/z Calculated for $C_{20}N_{36}O_3Si$: 352.2434, obtained ($CI^+$): 352.2448.

4-Dodecoyloxymethyl-5-hydroxy-2(5H)-furanone.

A stirred solution of 4-dodecoyloxymethyl-2-trimethylsilylfuran (0.375 g., 1.07 mmol) and Rose Bengal (trace) in 275 ml of acetone was flushed with oxygen and cooled to -78°. The solution was subsequently irradiated with a 300 Watt flood lamp while under constant positive pressure of oxygen until the starting material was no longer visible by TLC. The solution was warmed to room temperature and concentrated to a pale, orange solid residue. This material was purified by passing through a small plug of silica (ethyl ether as eluent) to give the desired hydroxybutenolide.

$^1$H NMR ($CDCl_3$): 6.16 (broad s, 1H); 6.05 (broad s, 1H); 5.55 (broad s, 1H); 4.97 (m, 2H); 2.41 (m, 2H); 1.6 to 1.75 (m, 2H); 1.2 to 1.4 (m, 16H); 0.88 (m, 3H).

$^{13}$C NMR ($CDCl_3$): 173.4, 170.6, 163.2, 118.7, 97.6, 58.8, 33.9, 31.9, 29.5, 29.4, 29.3, 29.2, 29.1, 24.8, 22.6, 14.1.

m/z Calculated for $C_{17}H_{32}NO_5$ $(M + NH_4)^+$: 330.2280, obtained ($CI^+$): 330.2282.

EXAMPLE 19

4-[1-hydroxy-2-(2-ethenyl)-2,6,10-trimethylundeca-5.9-diene]-2-trimethylsilylfuran

To a stirred solution of farnesylmagnesium chloride (1.83 mmol, prepared from 1.83 mmol farnesyl chloride and 3.65 mmol magnesium, initiated with a catalytic amount of 1,2-dibromoethane) in 1.5 ml tetrahydrofuran at 0° under argon was added dropwise 5-trimethylsilyl-3-furaldehyde (0.277 g., 1.64 mmol) in 3 ml tetrahydrofuran. This solution was allowed to warm to room temperature, stirred for 1 hour, and then quenched with a 5% ammonium chloride solution. The resulting mixture was partitioned between ethyl ether and 5% sodium bicarbonate. The organic portion was washed with water, saturated sodium chloride, dried over magnesium sulfate, filtered and concentrated to give a pale yellow oil. This material was purified by flash chromatography (silica, 5% to 10% ethyl ether/petroleum ether) to give the alcohol.

$^1$H NMR (mixture of diasteriomers) ($CDCl_3$): 7.53 (s, 0.6H); 7.50 (s, 0.4H); 6.59 (s, 0.6H); 6.54 (s, 0.4H); 5.75 to 5.95 (m, 1H); 5.15 to 5.35 (m, 2H); 5.0 to 5.15 (m, 2H); 4.41 (s, 1H); 2.0 to 2.15 (s, 1H); 1.8 to 2.0 (m, 6H); 1.67 (s, 3H); 1.59 (s, 3H); 1.56 (s, 3H); 1.3 to 1.5 (m, 2H)); 1.08 (s, 1.2H); 0.98 (s, 1.8H); 0.25 (s, 9H).

$^{13}$C NMR (mixture of diasteriomers) ($CDCl_3$): 160.2, 144.5 and 144.2, 144.0 and 142.9, 134.9 and 134.8, 131.3, 125.8 and 125.0, 124.7 and 124.6, 124.4, 120.0 and 119.8, 115.8 and 115.1, 74.0 and 73.2, 45.5 and 44.9, 39.7, 37.5 and 36.5, 26.7, 25.7, 22.6, 19.0, 17.7, 16.5 and 16.0, -1.6.

m/z Calculated for $C_{23}H_{38}O_2Si$: 374.2641, obtained ($EL^+$): 374.2634.

4-[1-Acetoxy-2-(2-ethenyl)-2,6,10-trimethylundeca-5,9-dienel-2-trimethylsilylfuran.

A stirred solution of 4-[1-hydroxy-2-(2-ethenyl)-2,6,10-trimethylundeca-5,9-diene]-2-trimethylsilylfuran (0.060 g., 0.160 mmol), acetic anhydride (0.5 ml, excess), and pyridine (5 drops, excess) was stirred at

room temperature until no starting material remained (as monitored by TLC). The reaction mixture was then partitioned between ethyl ether and a 5% ammonium chloride solution. The organic portion was washed repeatedly with saturated sodium bicarbonate solution, twice with aqueous cupric sulfate solution, water, saturated sodium chloride solution, dried over magnesium sulfate, filtered and concentrated to give a pale yellow oil. This material was further purified by flash chromatography (silica, 5% ethyl ether/ petroleum ether) to give the desired acetate as a diasteriomeric mixture.

$^1H$ NMR (mixture of diasteriomers) ($CDCl_3$): 7.53 (s, 0.6H); 7.50 (s, 0.4H); 6.54 (s, 0.6H); 6.51 (s, 0.4H); 5.75 to 5.95 (m, 1H); 5.67 (s, 1H); 4.95 to 5.25 (m, 4H); 2.06 (s, 1.8H); 2.03 (s, 1.2H); 1.85 to 2.03 (m, 6H); 1.67 (s, 3H); 1.59 (s, 3H); 1.56 (s, 3H); 1.3 to 1.5 (m, 2H); 1.04 (s, 1.8H); 1.00 (s, 1.2H); 0.24 (s, 9H).

$^{13}C$ NMR (mixture of diasteriomers) ($CDCl_3$): 170.0, 160.0, 145.0 and 144.7, 142.8 and 142.2, 134.9, 131.2, 124.5 and 124.4, 124.3, 122.3 and 122.2, 120.2, 114.6, 74.9 and 74.4, 44.1 and 43.7, 39.6, 37.2 and 36.6, 26.7, 25.6, 22.5, 21.1, 18.8, 18.0 and 17.6, 15.9, -1.7.

m/z Calculated for $C_{25}H_{40}O_3Si$: 416.2747, obtained ($EI^+$): 416.2756.

4-[1-Acetoxy-2-(2-ethenyl)-2.6.10-trimethylundeca-5.9-diene]-5-hydroxy-2(5H)-furanone

A stirred solution of 4-[1-acetoxy-2-(2-ethenyl)-2,6,10-trimethylundeca-5,9-diene]-2-trimethylsilylfuran (0.042 g., 0.10 mmol) and Rose Bengal (trace) in acetone was flushed with oxygen and cooled to -78°. The solution was subsequently irradiated with a 150 Watt flood lamp while under a constant pressure of oxygen until starting material was no longer visible by TLC. The solution was warmed to room temperature, concentrated to a red oil and purified by flash chromatography (silica, 40° ethyl acetate/hexane) to give the desired hydroxybutenolide as a mixture of several diasteriomers.

$^1H$ NMR (mixture of diasteriomers) ($CDCl_3$): 5.7 to 6.0 (m, 3H); 5.2 to 5.4 (m, 2H); 4.95 to 5.0 (m, 3H); 4.94 (s, 1H); 1.2 to 2.2 (m, 20H); 1.14 (s, 1.8H); 1.09 (s, 1.3H).

$^{13}C$ NMR (mixture of diasteriomers) ($CDCl_3$): 172.3, 169.3, 165.6 and 165.0, 100.2 and 100.0, 75.3 and 75.0, 20.9.

m/z Calculated for $C_{22}H_{36}NO_5$ $(M + NH_4)^+$: 394.2593, obtained ($CI^+$): 394.2571.

EXAMPLE 20

3-(1-Hydroxy-4-phenylbutyl)furan

To a stirred solution of 3-phenylpropyl magnesium bromide (4.5 ml of a 2.73 M solution in ethyl ether, 12.3 mmol, generated from 1-bromo-3-phenylpropane and magnesium and initiated with iodine) at 0° under argon, was added dropwise 3-furaldehyde (1.08 g., 11.2 mmol) in 5 ml ethyl ether. This solution was allowed to warm to room temperature, stirred for 20 minutes, and then poured over crushed ice containing several drops of concentrated sulfuric acid. The resulting mixture was partitioned between ethyl ether and 5% sodium bisulfite. The organic portion was washed with 5% sodium bicarbonate solution, water, saturated sodium chloride solution, dried over magnesium sulfate, filtered and concentrated to give an oil. This material was further purified by flash chromatography (silica, 20% ethyl acetate/hexane) to give the desired alcohol.

$^1H$ NMR ($CDCl_3$): 7.1 to 7.35 (m, 7H); 6.3 (s, 1H); 4.57 (t, 1H); 2.60 (t, 2H); 2.25 (s, 1H); 1.55 to 1.70 (m, 4H).

$^{13}C$ NMR ($CDCl_3$) : 143.2, 142.1, 138.9, 128.9, 128.3, 128.2, 125.7, 108.4, 66.6, 37.1, 35.5, 27.3.

3-(1-Acetoxy-4-phenylbutyl)furan

A solution of 3-(1-hydroxy-4-phenylbutyl)-furan (0.167 g., 0.772 mmol), acetic anhydride (1 ml, excess) and pyridine (3 to 4 drops, excess), was stirred at room temperature until no starting material remained (as monitored by TLC). The reaction mixture was then partitioned between ethyl ether and a 5% ammonium chloride solution. The organic portion was washed repeatedly with saturated sodium bicarbonate solution, aqueous cupric sulfate solution, water, saturated sodium chloride solution, dried over magnesium sulfate, filtered and concentrated to give 3-(1-acetoxy-4-phenylbutyl)furan.

$^1H$ NMR ($CDCl_3$): 7.1 to 7.3 (m, 7H); 6.35 (s, 1H); 5.79 (t, 1H); 2.63 (t, 2H); 2.04 (s, 3H); 1.6 to 1.95 (m, 4H).

3-(1-Acetoxy-4-phenylbutyl)-5-hydroxy-2(5H)-furanone

4-(1-Acetoxy-4-phenylbutyl)-5-hydroxy-2(5H)-furanone

A stirred solution of 3-(1-acetoxy-4-phenylbutyl)furan (0.244 g., 0.95 mmol) and Rose Bengal (trace) in methanol was flushed with oxygen and cooled to -78°. The solution was subsequently irradiated with a 150 Watt flood lamp while under a constant positive pressure of oxygen until starting material was no longer visible by TLC. The solution was warmed to room temperature, concentrated to a red oil (0.284 g.), and purified by flash chromato- graphy (silica, 20% ethyl acetate/methylene chloride) to give a mixture of two regioisomers [0.236 g.] The mixture was separated by semi-preparative HPLC on a Whatman Partisil M-9 silica column, using an elution rate of 4 ml/min. with 20% ethyl acetate/methylene chloride. The 4-substituted furanone product was eluted at 9.5 minutes, followed by the 3-substituted furanone product at 10.5 minutes. Concentration gave the purified the titled compounds, separately, as clear glasses which solidified on standing.

4-(1-Acetoxy-4-phenylbutyl)-5-hydroxy-2(5H)-furanone

Spectral data for this compound was the same here as described for this compound in Example 1.

3-(1-Acetoxy-4-phenylbutyl)-5-hydroxy-2(5H)-furanone

$^1$H NMR (mixture of diasteriomers) ($CDCl_3$): 7.1 to 7.3 (m, 5H); 6.7 (s, 1H); 6.09 (2 s, 1H); 5.75 (broad s, 1H); 5.57 (broad s, 1H); 2.62 (m, 2H); 2.08 (s, 1.8H); 2.07 (s, 1.2H); 1.85 (m, 2H); 1.65 (m, 2H).

$^{13}$C NMR (mixture of diasteriomers) ($CDCl_3$): 170.4 and 170.3, 169.41 and 169.40, 145.3 and 145.2, 141.5, 136.53 and 136.46, 128.5 and 128.2, 125.8, 97.1 and 96.8, 68.3 and 68.1, 60.5, 35.1, 32.3 and 32.1, 26.6, 20.9 and 20.7.

m/z Calculated for $C_{14}H_{14}O_3$ ($M^+$ - HOAc): 230.0943, obtained ($EI^+$): 230.0952.

EXAMPLE 21

3-Dodecoyloxymethylfuran.

To a stirred solution of 3-hydroxymethylfuran (1.01 g., 10.3 mmol) and pyridine (0.87 g., 10.3 mmol) in 30 ml dry tetrahydrofuran at 0° under argon was added lauroyl chloride (2.36 g., 10.8 mmol). This solution was allowed to warm to room temperature, during which time a white precipitate formed. After one hour, the mixture was filtered and the solution partitioned between ethyl ether and a 5% ammonium chloride solution. The organic portion was washed twice with 5% sodium bicarbonate solution, three times with aqueous cupric sulfate solution, water, saturated sodium chloride solution, dried over magnesium sulfate, filtered and concentrated to give a yellow oil. This material was further purified by flash chromatography (silica, 5% ethyl ether/petroleum ether) to give the desired ester.

$^1$H NMR ($CDCl_3$): 7.45 (broad s, 1H); 7.37 (broad s, 1H); 6.41 (broad s, 1H); 4.97 (s, 2H); 2.30 (t, 2H); 1.55 to 1.7 (m, 2H); 1.2 to 1.4 (m, 16H); 0.88 (t, 3H).

$^{13}$C NMR ($CDCl_3$): 173.3, 143.1, 141.3, 120.5, 110.4, 57.3, 34.1, 31.8, 29.5, 29.3, 29.2, 29.1, 29.0, 24.8, 22.6, 13.9.

m/z Calculated for $C_{17}H_{28}O_3$: 280.2194, obtained ($EI^+$): 280.2042.

3-Dodecoyloxymethyl-5-hydroxy-2(5H)-furanone.

A stirred solution of 3-dodecoyloxymethylfuran (0.602 g., 2.15 mmol) and Rose Bengal (trace) in 225 ml acetone was flushed with oxygen and cooled to -78°. The solution was subsequently irradiated with a 150 Watt flood lamp while under a constant pressure of oxygen until starting material was no longer visible by TLC. The solution was warmed to room temperature, concentrated to a red oil and the residue was purified by flash chromatography (silica, 60% ethyl ether/petroleum ether) to give the 3-substituted hydroxybutenolide as the only isolated product.

$^1$H NMR ($CDCl_3$): 7.11 (broad s, 1H); 6.20 (broad s, 1H); 5.42 (broad s, 1H); 4.85 (s, 2H); 2.38 (t, 2H); 1.55 to 1.7 (m, 2H); 1.2 to 1.4 (m, 16H); 0.88 (t, 3H).

$^{13}$C NMR ($CDCl_3$): 173.5, 170.0, 146.4, 133.0, 97.5, 57.2, 33.9, 31.8, 29.5, 29.4, 29.3, 29.2, 29.1, 24.8, 22.6, 14.1.

m/z Calculated for $C_{17}H_{32}NO_5$ $(M + NH_4)^+$: 330.2281, obtained ($CI^+$): 330.2283.

Example 22

5-Dodecoyloxy-3-dodecoyloxymethyl-2(5H)-furanone.

To a stirred solution of 3-dodecoyloxymethyl-5-hydroxy-2(5H)-furanone (0.098 g., 0.313 mmol) and pyridine (0.026 g., 0.313 mmol) in 3 ml anhydrous tetrahydrofuran at 0° was added lauroyl chloride (0.082 g., 0.376 mmol). The solution was allowed to warm to room temperature, stirred until starting material was no longer visible by TLC, and partitioned between ethyl ether and a 5% ammonium chloride solution. The organic portion was washed twice with 5% sodium bicarbonate solution, twice with aqueous cupric sulfate solution, water, saturated sodium chloride solution, dried over magnesium sulfate, filtered and concentrated to give a solid residue. This material was further purified by recrystallization (acetone/methanol) to give the desired ester.

$^1H$ NMR ($CDCl_3$): 7.13 (s, 1H); 6.97 (s, 1H); 4.88 (s, 2H); 2.35 to 2.50 (m, 4H); 1.6 to 1.75 (m, 4H); 1.2 to 1.5 (m, 32H); 0.85 to 0.95 (m, 6H).

$^{13}C$ NMR ($CDCl_3$): 172.9, 171.6, 168.4, 144.0, 133.9, 92.4, 57.1, 33.9, 33.8, 31.8, multiple peaks between 30.3 and 28.9, 24.8, 24.4, 22.6, 14.1.

m/z Calculated for $C_{29}H_{54}NO_6$ $(M + NH_4)^+$: 512.3951, obtained ($CI^+$): 512.3939.

EXAMPLE 23

5-Acetoxy-3-dodocoyloxymethyl-2(5H)-furanone.

A stirred solution of 3-dodecoyloxymethyl-5-hydroxy-2(5H)furanone (0.165 g., 0.527 mmol), acetic anhydride (0.5 ml, excess), and pyridine (7 drops, excess) was stirred at room temperature until no starting material remained (as monitored by TLC). The reaction mixture was then partitioned between ethyl ether and 5% ammonium chloride solution. The organic portion was washed repeatedly with saturated sodium bicarbonate solution, twice with aqueous cupric sulfate solution, water, saturated sodium chloride solution, dried over magnesium sulfate, filtered and concentrated to give a dark, yellow oil. This material was further purified by flash chromatography (silica, 30% to 40% ethyl ether/petroleum ether) to give the titled diester.

$^1H$ NMR ($CDCl_3$): 7.13 (s, 1H); 6.95 (s, 1H); 4.89 (s, 2H); 2.39 (t, 2H); 2.17 (s, 3H); 1.6 to 1.75 (m, 2H); 1.2 to 1.5 (m, 16H); 0.88 (t, 3H).

$^{13}C$ NMR ($CDCl_3$): 172.9, 168.8, 168.4, 143.9, 134.0, 92.5, 57.1, 33.9, 31.9, 29.6, 29.4, 29.3, 29.2, 29.1, 24.8, 22.7, 20.6, 14.1.

m/z Calculated for $C_{19}H_{34}NO_6$ $(M + NH_4)^+$: 372.2386, obtained ($CI^+$): 372.2369.

EXAMPLE 24

3-(1-Hydroxy)undecylfuran.

A solution of decyl bromide in (3.00 g, 13.5 mmol) in 25 ml of tetrahydrofuran was added to 0.33 g. of magnesium turnings (0.33 9., 13.7 mmol). The mixture was stirred at room temperature for 2hr, then heated at reflux for 1hr, then cooled to room temperature. A solution of 3-furaldehyde (1.31 g., 13.6 mmol) in 25 ml of THF was added dropwise over 1hr. The mixture was stirred for 1hr, then cooled to 0° and quenched with a saturated aqueous ammonium chloride solution. The mixture was diluted with 100 ml of ether, washed with brine (3 x 50 ml), dried over magnesium sulfate and filtered. The solvent was removed in vacuo to give an orange oil. The product was purified by flash chromatography (10% ethyl acetate/hexane on silica gel) to give 3-(1-hydroxy)undecylfuran.

$^1H$ NMR ($CDCl_3$): 7.36 (s, 2H), 6.38 (s, 1H), 4.61 (t, J=6.5 Hz, 1H), 2.10 (s, 1H), 1.72 (m, 2H), 1.25 (m, 16H, $CH_2$ envelope), 0.88 (t, J=5.6 Hz, 3H) $^{13}C$ NMR ($CDCl_3$): 143.21, 138.90, 129.23, 108.41, 66.94, 37.81, 31.93, 29.36, 25.62, 22.66, 14.01.

3-(1-Keto)undecylfuran.

A solution of 0.35 g. of 3-(1-hydroxy)undecylfuran in 5 ml of dichloromethane was added to a flask containing 0.32 g. of pyridinium chlorochromate and 25 ml of dry dichloromethane. The reaction was stirred at room temperature for 12hr before diluting with ether (100 ml) and filtering through celite. The filtrate was washed with 5% aqueous sodium bicarbonate solution (50 ml) and brine. It was then dried over magnesium sulfate, filtered and the solvent removed to give a brown oil. Flash chromatography (10% ethyl

acetate/hexane on silica gel) yielded the 3-(1-keto)undecylfuran, m.p. 51-52°.

$^{1}H$ NMR ($CDCl_3$): 8.02 (s, 1H), 7.43 (s, 1H), 6.77 (s, 1H) 2.73 (t, J=7.2 Hz, 2 H) 1.31 (m, 14H, $CH_2$ envelope), 0.88 (t, J=5.8 Hz, 3H).

$^{13}C$ NMR ($CDCl_3$): 195.31, 146.92, 144.11, 127.70, 108.64, 40.52, 31.83, 29.57, 29.45, 29.37, 24.45, 22.61, 14.01.

5-Hydroxy-4-(1-keto)undecyl-2(5H)-furanone.

A mixture of 20 ml of methanol and 150 ml of tetrahydrofuran was saturated with oxygen by bubbling gas through the solution. The solution was cooled to -78° (dry ice/acetone) and 0.23 g. of 3-(1-keto)-undecylfuran and 0.005 g of Rose Bengal were added to the solution. The solution was irradiated with a 300 Watt quartz-halogen spotlight for 4hr at -78° while a stream of oxygen was passed continuously through the solution. The irradiation was stopped, the reaction warmed to room temperature and the solvent removed by evaporation. The residue was chromatographed on silica gel (20% ethyl acetate/hexane) to yield a fraction with $R_f$ of about 0.1. This fraction was triturated with 40% ethyl acetate/hexane to yield the captioned furanone, m.p. 65-67°.

$^{1}H$ NMR ($CDCl_3$): 6.59 (s, 1H), 6.45 (s, 1H), 2.80 (m, 2H), 1.67 (m, 2H), 1.26 (m, 14H, $CH_2$ envelope) and 0.88 (t, J = 5.2 Hz, 3H).

$^{13}C$ NMR ($CDCl_3$): 196.11, 157.81, 126.10, 97.15, 41.22, 31.91, 29.64, 29.51, 29.41, 29.12, 23.39, 22.74, 14.21.

Exact mass calculated for $C_{15}H_{24}O_4$ m/z 268.1674, found 268.1669.

EXAMPLE 25

4-(1-Acetoxytridecyl)-5-hydroxy-2-tetrahydrofuranone.

A solution of 4-(1-acetoxytridecyl)-5-hydroxy-2(5H)-furanone (0.11g, 0.32 mmol) and 5% rhodium on alumina (24 mg) in 6 ml of methanol was stirred rapidly under a hydrogen atmosphere for two hours. The mixture was concentrated, taken up in ethyl acetate, filtered through celite, and concentrated to give the tetrahydrofuranone as a colorless glass.

$^{1}H$ NMR (mixture of diasteriomers) ($CDCl_3$): 5.75 (brs, 1H, sharpens to 2S, 5.81 and 5.69 on $D_2O$ exchange); 5.15 (brm, 1H, sharpens to 2m, 5.20 and 5.19 and 1s, 4.83 on $D_2O$ exchange), 2.4 (m, 3H); 2.08 and 2.06 (2s, 3H); 1.3(m); 0.88 (t, J=6.6 Hz, 3H).

$^{13}C$NMR (mixture of diasteriomers) ($CDCl_3$): 176.0, 171.1, 101.1, 73.1 and 72.3, 60.5, 46.2, 32.4, 31.8, 30.8, 29.6, 29.4, 29.3, 29.25, 29.20, 29.0, 28.6, 25.2, 22.6, 20.8, 14.0.

m/z Calculated for $C_{22}H_{43}O_5Si$ (M=TMS): 415..2880, obtained ($CI^+$): 415.2897.

EXAMPLE 26

4-[1-(4-Phenylbutanoyloxy)tridecyl]-2-trimethylsilylfuran

tert-Butyl lithium (a 1.7 M solution in pentane; 0.47 ml, 0.79 mmol) was added dropwise to a solution of 4-(1-hydroxytridecyl)-2-trimethylsilylfuran (256.4 mg, 0.76 mmol), prepared as in Example 38, in tetrahydrofuran (5 ml) at -78° under argon. After 10 minutes, a solution of 4-phenylbutyryl chloride (145 mg, 0.79 mmol) was added. Stirring was continued at room temperature for 2 days and the mixture was quenched with water. Extraction and evaporation of the dried (magnesium sulphate) extracts gave an oil, which was purified by preparative thin-layer chromatography (20x20 cm, 1000µ silica plate; developed with 10% ethyl ether/hexane). The title ester was obtained as a light yellow oil.

$^{1}H$ NMR ($CDCl_3$): 0.28 (s, 9H), 0.91 (t, 3H, J=7.0 Hz), 1.28 (m, 20H), 1.85 (m, 2H), 1.97 (p, 2H, J=7.7 Hz), 2.35 (t, 2H, J=7.4 Hz), 2.65 (t, 2H, J=7.8 Hz), 5.82 (t, 1H, J=6.9 Hz), 6.61 (s, 1H), 7.25 (m, 2H) and 7.62 (s, 1H).

MS m/e (% abundance) 485 ($M^+$, 7), 339 (28), 321 (32), 170 (12), 154 (19), 153 (18), 147 (84), 91 (46) and 73 (100).

4-[1-(4-Phenylbutanoyloxy)tridecyl]-5-hydroxy-2(5H)-furanone

A mixture of 4-[1-(4-phenylbutanoyloxy)tridecyl]-2-trimethylsilylfuran (203 mg, 0.42 mmol) and Rose Bengal (5 mg) in tetrahydrofuran (7 ml) was exposed to singlet oxygen at -78° for 100 minutes. The

residue, after solvent removal, was purified by preparative TLC (20x20 cm, 500μ silica plate; developed with 60% ethyl ether/hexane). The title furanone was isolated as a colorless oil.

$^1$H NMR ($CDCl_3$): 0.93 (t, 3H, J = 6.4 Hz), 1.30 (brs, 20H), 1.95 (br, 2H), 2.03 (p, 2H, J = 7.5 Hz), 2.43 (t, 2H, J = 7.3 Hz), 2.71 (t, 2H, J = 7.4 Hz), 5.45 (br, 1H), 5.99 (brs, 1H), 6.05 (brs, 1H), 5.25 (br, 1H), 6.20 (br, 1H) and 7.30 (m, 5H).

$^{13}$C NMR ($CDCl_3$): 14.1, 22.7, 25.0, 26.2, 29.1, 29.3, 29.5, 29.6, 31.9, 33.2, 33.4, 35.0, 69.0, 69.6, 98.0, 118.5, 119.1, 126.2, 128.4, 140.9, 167.2, 169.7 and 173.6.

MS m/e: exact mass calculated for $C_{27}H_{44}NO_5$ $(M+NH_4)^+$ 462.3219, found 462.3220.

EXAMPLE 27

4-[1-(3-Phenylpropanoyloxy(tridecyl]-2-trimethylsilylfuran

tert-Butyl lithium (a 1.7 M solution in pentane; 0.25 ml, 0.42 mmol) was added dropwise to a solution of 4-(1-hydroxytridecyl)-2-trimethylsilylfuran (118 mg, 0.35 mmol) in tetrahydrofuran (6 ml) at -78° under argon. After 25 minutes, a solution of hydrocinnamoyl chloride (62.2 μl, 0.42 mmol) in tetrahydrofuran (1/2 ml) was added. Stirring was continued at room temperature for 7 hours and quenched with water. Extraction (ethyl ether) and evaporation of the dried (magnesium sulphate) extracts gave an oil, which was purified by preparative TLC (20x20 cm, 500μ silica plate; developed with 60% ethyl ether/hexane). The title ester was obtained as a colorless oil.

$^1$H NMR ($CDCl_3$): 0.27 (s, 9H), 0.93 (t, 3H), 1.27 (brs, 20H), 1.85 (m, 2H), 2.65 (t, 2H, J = 6.9 Hz), 2.96 (2H, t, J = 6.9 Hz), 5.80 (t, 1H, J = 7.5 Hz), 6.57 (s, 1H), 7.17-7.32 (m, 5H) and 7.57 (s, 1H).

MS m/e (% abundance) 456 ($M^+$, 8), 338 (30), 321 (15), 247 (5), 193 (9), 153 (17), 91 (56) and 73 (100).

4-[1-(3-Phenylpropanoyloxy)tridecyl]-5-hydroxy-2(5H)-furanone

A mixture of 4-[1-(3-phenylpropanoyloxy)tridecyl]-2-trimethylsilylfuran (70 mg, 0.15 mmol) and Rose Bengal (5 mg) in tetrahydrofuran (7 ml) was exposed to singlet oxygen at -78° for 80 minutes. The residue, after solvent removal, was purified by preparative TLC (20x20 cm, 500μ silica plate; developed with 60% ethyl ether/hexane). The title furanone was isolated as a light yellow oil.

$^1$H NMR (mixture of diasteriomers) ($CDCl_3$): 0.87 (t, 3H, J = 6.9 Hz), 1.26 (brs, 20H), 1.72 (m, 2H), 2.73 (brt, 2H), 2.97 (t, 2H, J = 7.2 Hz), 4.77 (br, 1H), 5.30 (brt, 1H), 5.48 (brt, 1H), 5.65 (s, 1H), 5.85 (s, 1H), 5.90 (s, 1H), 5.95 (s, 1H) and 7.25 (m, 5H).

$^{13}$C NMR (mixture of diasteriomers) ($CDCl_3$): 14.1, 22.7 24.8, 25.9, 28,9, 29.1, 29.3, 29.5, 29.6, 30.6, 30.8, 31.7, 31.8, 31.9, 32.7, 32.9, 33.1, 35.3, 35.4, 35.6, 53.3, 69.7, 97.9, 118.5, 126.7, 128.2, 128.6, 139.8, 166.8 and 169.7.

MS m/e: exact mass calculated for $C_{26}H_{42}NO_5$ $(M+NH_4)^+$ 448.3062, found 448.3052.

EXAMPLE 28

4-[1-(Phenylacetoxy)tridecyl]-2-trimethylsilylfuran

tert-Butyl lithium (a 1.7 M solution in pentane; 0.29 ml, 0.49 mmol) was added dropwise to a solution of 4-(1-hydroxytridecyl)-2-trimethylsilylfuran (138.2 mg, 0.41 mmol) in tetrahydrofuran (7 ml) at -78° under argon. After 25 minutes, a solution of phenylacetyl chloride (65 μl, 0.49 mmol) in tetrahydrofuran (1/2 ml) was added. Stirring was continued at room temperature for 16 hours and quenched with water. Extraction (ethyl ether) and evaporation of the dried (magnesium sulphate) extracts gave an oil, which was purified by preparative TLC (20x20 cm, 500μ silica plate; developed with 10% ethyl ether/hexane). The title ester was obtained as a pale yellow oil.

$^1$H NMR ($CDCl_3$): 0.25 (s, 9H), 0.89 (t, 3H, J = 7.0 Hz), 1.27 (brs, 20H), 1.85 (m, 2H), 3.75 (dd, 2H), 5.80 (m, 1H), 6.52 (2s, 2H), 7.20 (m, 5H), 7.52 (s, 1H) and 7.54 (s, 1H).

MS m/e (% abundance) 456 ($M^+$, 8), 338 (30), 321 (15), 247 (5), 193 (9), 153 (17), 91 (56) and 73 (100).

4-[1-(Phenylacetoxy)tridecyl]-5-hydroxy-2(5H)-furanone

A mixture of 4-[1-(phenylacetoxy)tridecyl]-2-trimethylsilylfuran (60 mg, 0.13 mmol) and Rose Bengal (5 mg) in tetrahydrofuran (5 ml) was exposed to singlet oxygen at -78° for 2 hours. The residue, after solvent removal, was purified by preparative TLC (20x20 cm, 500μ silica plate; developed with 60% ethyl

ether/hexane). The title furanone was isolated as a yellow oil.

$^{1}H$ NMR (mixture of diasteriomers and enol forms) ($CDCl_3$): 0.91 (t, 3H, J=6.3 Hz), 1.28 (brs, 20H), 1.85 (m, 2H), 3.65 (brs, 2H), 5.40-6.20 (m, 4H) and 7.40 (m, 5H).

$^{13}C$ NMR (mixture of diasteriomers and enol forms) ($CDCl_3$): 14.1, 22.7, 24.5, 24.7, 24.9, 28.8, 29.1, 29.3, 29.5, 29.6, 31.9, 32.9, 33.1, 33.2, 41.4, 43.6, 48.5, 53.4, 69.5, 70.0, 70.1, 97.3, 97.7, 97.8, 118.4, 119.2, 126.0, 126.1, 126.3, 126.9, 127.1, 127.2, 127.5, 127.6, 128.3, 128.5, 128.7. 128.8, 129.0, 129.2, 129.4, 129.5, 129.8, 129.9, 130.0, 131.3, 166.7, 169.5 and 171.6.

MS m/e: exact mass calculated for $C_{25}H_{40}NO_5$ $(M+NH_4)^+$ 434.2906, found 434.2914.

## EXAMPLE 29

### 4-[1-(Cyclohexanoyloxy)tridecyl]-2-trimethylsilylfuran

tert-Butyl lithium (a 1.7 M solution in pentane; 0.33 ml, 0.55 mmol) was added dropwise to a solution of 4-(1-hydroxytridecyl)-2-trimethylsilylfuran (170 mg, 0.50 mmol) in tetrahydrofuran (5 ml) at 0° under argon. After 10 minutes, cyclohexanecarboxylic acid chloride (74 µl, 0.55 mmol) was added. The mixture was stirred at room temperature for 15 hours and quenched with water. Extraction and evaporation of the dried (magnesium sulphate) extracts gave an oil, which was purified by preparative TLC (20x20 cm, 1000µ silica plate; developed with 5% ethyl ether/hexane). The title ester was obtained as a pale yellow oil.

$^{1}H$ NMR ($CDCl_3$): 0.23 (s, 9H), 0.94 (t, 3H, J=6.9 Hz), 1.31 (br, 20H), 1.21 (m, 12H), 2.35 (tt, 1H, J=11.3 Hz, 3.7 Hz), 5.82 (t, 1H, J=7.5 Hz), 6.61 (s, 1H), and 7.62 (s, 1H).

MS m/e (% abundance) 448 ($M^+$, 12), 339 (18), 338 (67), 337 (20), 185 (12), 170 (10), 154 (13), 153 (13), 111 (33), 84 (11), 83 (100) and 73 (71).

### 4-[1-(Cyclohexanoyloxy)tridecyl]-5-hydroxy-2(5H)-furanone

A mixture of 4-[1-(cyclohexanoyloxy)tridecyl)-2-trimethylsilylfuran (70 mg, 0.16 mmol) and Rose Bengal (5 mg) in tetrahydrofuran (6 ml) was exposed to singlet oxygen at -78° for 2 hours. The residue, after solvent removal, was purified by preparative TLC (20x20 cm, 1000µ silica plate; developed with 60% ethyl ether/hexane). The title furanone was obtained as a colorless oil.

$^{1}H$ NMR ($CDCl_3$): 0.91 (t, 3H, J=6.8 Hz), 1.29 (m, 6H), 1.70-2.00 (m, 6H), 2.40 (m, 1H), 5.45 (br, 2H), 5.98 (br, 1H) and 6.05 (br, 1H).

$^{13}C$ NMR ($CDCl_3$): 14.1, 22.7, 25.1, 25.3, 25.6, 28.8, 28.9, 29.2, 29.4, 29.5, 29.7, 31.9, 33.3, 43.1, 68.7, 69.3, 98.1, 118.3, 119.0 and 167.6.

MS m/e: exact mass calculated for $C_{24}H_{41}O_5$ $(M+H)^+$ 409.2953, found 409.2971.

## EXAMPLE 30

### 4-[1-(Dodecanoyloxy)tridecyl]-2-trimethylsilylfuran

tert-Butyl lithium (a 1.7 M solution in pentane; 0.21 ml, 0.36 mmol) was added dropwise to a solution of 4-(1-hydroxytridecyl)-2-trimethylsilylfuran (124.5 mg, 0.37 mmol) in tetrahydrofuran (5 ml) at 0° under argon. After 5 minutes, the cooling bath was removed and lauroyl chloride (88 µl) was added. The mixture was stirred at room temperature for 16 hours and quenched with water. Extraction (ethyl ether) and evaporation of the dried (magnesium sulphate) extracts gave an oil, which was purified by preparative TLC (20x20 cm, 1000µ silica plate; developed with 5% ethyl ether/hexane). The title ester was obtained as a colorless oil.

$^{1}H$ NMR ($CDCl_3$): 0.23 (s, 9H), 0.88 (t, 3H, J=6.9 Hz), 1.25 (brs, 36H), 1.57-1.92 (m, 2H), 2.29 (t, 2H, J=7.2 Hz), 5.77 (t, 1H, J=7.5 Hz), 6.57 (s, 1H) and 7.57 (s, 1H).

MS m/e (% abundance) 520 ($M^+$, 9), 338 (45), 320 (18), 257 (10), 183 (16), 170 (10), 154 (25), 73 (100).

### 4-[1-(Dodecanoyloxy)tridecyl]-5-hydroxy-2(5H)-furanone

A mixture of 4-[1-(dodecanoyloxy)tridecyl]-2-trimethylsilylfuran (55 mg, 0.11 mmol) and Rose Bengal (5 mg) in tetrahydrofuran (7 ml) was exposed to singlet oxygen at -78° for 2 hours. The residue, after solvent removal, was purified by preparative TLC (20x20 cm, 500µ silica plate; developed with 60% ether/hexane). The title furanone was obtained as a colorless oil.

$^{1}H$ NMR ($CDCl_3$): 0.92 (t, 6H, J=6.4 Hz), 1.29 (brs, 38H), 1.60-1.90 (m, 2H), 2.40 (t, 2H, J=7.4 Hz), 5.40

(br, 1H) and 6.02 (br, 2H).

$^{13}C$ NMR (mixture of diasteriomers) ($CDCl_3$): 14.0, 14.1, 15.2, 22.7, 24.7, 24.9, 25.0, 25.4, 25.6, 27.9, 28.1, 28.2, 28.3, 28.5, 28.6, 28.7, 28.8, 28.9, 29.1, 29.3, 29.5, 29.6, 30.0, 30.2, 30.4, 30.5, 31.9, 33.1, 34.2, 34.3, 53.4, 65.9, 68.0, 69.3, 69.4, 69.5, 98.0, 118.3, 167.0, 170.0 and 173.5.

MS m/e: exact mass calculated for $C_{29}H_{53}O_5$ $(M+H)^+$ 481.3893, found 481.3895.

EXAMPLE 31

4-[1-(Methylcarbamoyloxy)tridecyl]-2-trimethylsilylfuran

tert-Butyl lithium (a 1.7 M solution in pentane; 0.21 ml, 0.36 mmol) was added dropwise to a solution of 4-(1-hydroxytridecyl)-2-trimethylsilylfuran (110 mg, 0.33 mmol) in tetrahydrofuran (5 ml) at 0° under argon. After 10 minutes, a solution of methyl isocyanate (21 µl, 0.36 mmol) in tetrahydrofuran (1/2 ml) was added. Stirring was continued at room temperature for 2 days and the mixture was quenched with water. Extraction (ethyl ether) and evaporation of the dried (magnesium sulphate) extracts gave a product which was purified by preparative TLC on a 20x20 cm 1000µ silica plate; developed with 20% ethyl ether/hexane). The title carbamate was obtained as a yellow oil.

$^1H$ NMR ($CDCl_3$): 0.27 (s, 9H), 0.91 (t, 3H, J=6.9 Hz), 1.28 (brs, 20H), 1.85 (m, 2H), 2.81 (d, 3H, J=4.9 Hz), 4.60 (br, 1H), 5.70 (t, 1H, J=7.5 Hz), 6.61 (s, 1H) and 7.62 (s, 1H).

MS m/e (% abundance) 395 ($M^+$, 3), 339 (19), 338 (71), 320 (16), 183 (17), 169 (15), 154 (25), 132 (11), 75 (25) and 73 (100).

4-[1-(Methylcarbamoyloxy)tridecyl]-5-hydroxy-2(5H)-furanone

A mixture of 4-[1-(methylcarbamoyloxy)tridecyl]-2-trimethylsilylfuran (12.5 mg, 0.03 mmol) and Rose Bengal (2 mg) in tetrahydrofuran (5 ml) was exposed to singlet oxygen at -78° for 80 minutes. The residue, after solvent removal, was purified by preparative TLC (20x20 cm, 250µ silica plate; developed with 70% ethyl ether/hexane). The title furanone was obtained as a pale yellow oil.

$^1H$ NMR (mixture of diasteriomers) ($CDCl_3$): 0.91 (t, 3H, J=6.8 Hz), 1.29 (brs, 20H), 1.80 (m, 2H), 2.83 (d, 3H, J=4.9 Hz), 2.90 (d, 3H, J=4.9 Hz), 4.80 (br, 1H), 4.95 (bd, 1H), 5.25 (brt, 1H), 5.45 (brt, 1H), 6.01 (brs, 1H) and 6.04 (brs, 1H).

$^{13}C$ NMR (mixture of diasteriomers) ($CDCl_3$): 14.1, 22.7, 25.1, 27.6, 29.2, 29.4, 29.5, 29.6, 31.9, 33.2, 33.7, 69.7, 69.9, 97.9, 98.5, 118.4, 118.9, 156.7, 168.4 and 169.9.

MS m/e exact mass calculated for $C_{19}H_{33}NO_5$ $(M+NH_4)^+$ 373.2702, found 373.2711.

EXAMPLE 32

4-[1-((R)-(+)-α-Methylbenzylcarbamoyloxy)tridecyl]-2-trimethylsilylfuran

tert-Butyl lithium (a 1.7 M solution in pentane; 0.59 ml, 1.0 mmol) was added dropwise to a solution of 4-(1-hydroxytridecyl)-2-trimethylsilylfuran (335.5 mg, 0.99 mmol) in tetrahydrofuran (6 ml) at 0° under argon. After 20 minutes, a solution of (R)-(+)-α-methylbenzyl isocyanate (146 mg, 0.99 mmol) in THF (1/2 ml) was added. Stirring was continued for 16 hours while the cooling bath attained room temperature. The mixture was quenched with water and extracted with ethyl ether. Evaporation of the dried (magnesium sulphate) extracts gave an oil, which was purified by preparative TLC (20x20 cm, two 1000µ silica plates; developed with 20% ethyl ether/hexane). The title carbamate was obtained as a light yellow oil.

$^1H$ NMR ($CDCl_3$): 0.30 (2s, 9H), 0.92 (brt, 3H), 1.29 (brs, 28H), 1.80 (br, 2H), 4.85-5.00 (2 brs, 2H), 5.68 (t, 1H, J=7.5 Hz), 6.60 (s, 1H), 6.70 (s, 1H), and 7.30 (m, 5H).

MS m/e (% abundance) 503 ($(M+NH_4^+)$, 0.1), 485 ($M^+$, 0.2), 442 (1), 322 (33), 321 (100), 320 (4), 238 (4), 183 (5), 122 (8) and 106 (4).

4-[1-((R)-(+)-α-Methylbenzylcarbamoyloxy)tridecyl]-5-hydroxy-2(5H)-furanone

A mixture of 4-[1-((R)-(+)-α-methylbenzylcarbamoyloxy)tridecyl]-2-trimethylsilylfuran (71 mg, 0.15 mmol) and Rose Bengal (5 mg) in tetrahydrofuran (5 ml) was exposed to singlet oxygen at -78° for 2 hours. The residue, after solvent removal, was purified by preparative TLC (20x20 cm, 500µ silica plate; developed with 60% ethyl ether/hexane). The title furanone was obtained as a pale yellow oil.

$^1H$ NMR (mixture of diasteriomers) ($CDCl_3$): 0.91 (t, 3H, J=7.0 Hz), 1.28 (brs, 18H), 1.38 (d, 3H, J=7.0

Hz), 1.50 (m, 2H), 1.78 (m, 2H), 4.80 (brm, 1H), 5.26 (m, 1H), 5.38 (m, 1H), 5.98 (brs, 1H), 6.05 (brs, 1H) and 7.35 (m, 5H).

$^{13}C$ NMR (mixture of diasteriomers) 14.1, 15.3, 17.9, 22.1, 22.3, 22.7, 25.0, 29.0, 29.1, 29.3, 29.5, 29.6, 31.9, 33.2, 33.4, 33.6, 51.0, 63.8, 69.8, 69.9, 97.9, 98.3, 98.4, 104.2, 118.3, 118.4, 118.8, 125.7, 125.8, 125.9, 127.7, 128.8, 142.7, 155.3, 161.8, 169.8 and 169.9.

MS m/e: exact mass calculated for $C_{25}H_{36}NO_5$ ($M^+$-$CH_3$): 430.2593, found 430.2603.

EXAMPLE 33

4-(1-Trichloroacetoxy)tridecyl-2-trimethylsilylfuran

tert-Butyl lithium (a 1.7 M solution in pentane; 0.32 ml, 0.54 mmol) was added dropwise to a solution of 4-(1-hydroxytridecyl)-2-trimethylsilylfuran (151.3 mg, 0.45 mmol) in tetrahydrofuran (10 ml) at 0° under argon. After 10 minutes trichloroacetic anhydride (98 µl, 0.54 mmol) was added and stirring was continued at room temperature overnight. The mixture was quenched with water and extracted with ethyl ether. Evaporation of the dried (magnesium sulphate) extracts afforded an oil, which was purified by preparative TLC (20x20 cm, 500µ silica plate; developed with 10% ethyl ether/petroleum ether). The title ester was obtained as a pale yellow oil.

$^1H$ NMR ($CDCl_3$): 0.28 (s, 9H), 0.91 (t, 3H, J=6.0 Hz), 1.28 (brs, 20H), 2.0 (m, 2H), 5.88 (t, 1H, J=7.5 Hz), 6.64 (s, 1H) and 7.70 (s, 1H).

MS m/e (% abundance) 460/462/464 ($(M+NH_4)^+$, 16, 16, 5), 426 (13), 316 (21), 300 (69), 299 (21) and 298 (100).

4-(1-Trichloroacetoxy)tridecyl-5-hydroxy-2(5H)-furanone

A mixture of 4-(1-trichloroacetoxy)tridecyl-2-trimethylsilylfuran (120 mg, 0.25 mmol) and Rose Bengal (5 mg) in tetrahydrofuran (5 mg) was exposed to singlet oxygen at -78° for 1/2 hour. The residue, after solvent removal, was purified by preparative TLC (20x20 cm, 500µ silica plate; developed with 60% ethyl ether/hexane). The title furanone was obtained as a light yellow oil.

$^1H$ NMR ($CDCl_3$): 0.92 (t, 3H, J=7.0 Hz), 1.29 (brs, 20H), 2.02 (br, 2H), 5.75 (br, 1H), 6.18 (brs + s, 2H).

$^{13}C$ NMR ($CDCl_3$): 14.1, 22.7, 24.6, 28.8, 29.0, 29.3, 29.4, 29.5, 29.6, 29.9, 31.7, 31.9, 32.7, 74.6, 89.5, 97.6, 119.1, 161.1, 164.5 and 169.8.

MS m/e: exact mass calculated for $C_{19}H_{33}Cl_3NO_5$ $(M+NH_4)^+$ 460.1424, found 460.1403.

EXAMPLE 34

5-Palmitoyloxy-4-(1-acetoxytridecyl)-2(H)-furanone

To a stirred solution of palmitoyl chloride (41 mg, 0.15 mmole) in THF (2 ml) at 0°C, was added 4-(1-acetoxytridecyl)-5-hydroxy-2(5H)-furanone, see Example 14 (30 mg, 0.088 mmole), and triethylamine (15 mg, 0.15 mmole). The flask was warmed to room temperature until no starting material remained (as monitored by TLC). The solution was diluted with pentane, then washed with water. The aqueous portion was extracted with ethyl ether and the combined organic portions were dried over magnesium sulfate, filtered, concentrated and purified by preparative TLC (20 x 20 cm, 250 µ silica plate; developed with 30% ethyl ether/hexane). The titled furanone was obtained as a colorless solid.

'H NMR ($CDCl_3$): 7.02 (s, 1H), 6.95 (s, 1H), 6.12 (s, 1H), 6.08 (s, 1H), 5.65 (t, J=3.75, 1H), 5.54 (t, J=3.75, 1H), 2.4 (t, J=7.5 Hz, 2H), 2.35 (t, J=7.5 Hz, 2H), 2.1 (s, 3H), 2.05 (s, 3H), 1.82 (m, 2H), 1.63 (m, 2H), 1.25 (m, 46H), 0.85 (m, 6H).

$^{13}C$ NMR ($CDCl_3$): 172, 170, 169, 164.6, 120, 119, 92.6, 92, 69, 68, 66, 33.9, 33.8, 33.1, 29.5, 29.3, 29.2, 29.1, 28.9, 24.7, 24.5, 22.7, 20.7, 15.2, 14.1.

MS m/e = exact mass calculated for $C_{35}H_{62}O_6$ 578.4546 ($M^+$), found 578.4560.

EXAMPLE 35

Ethyl 9-phenylnonanoate

To (E)-ethyl 9-phenyl-2,6-nonadienoate (0.798 g, 3.09 mmol) in ethyl acetate (5 ml) was added 10%

palladium on carbon (0.013 g). This mixture was subjected to one atmosphere of hydrogen at room temperature with stirring for 12 hours. The reaction mixture was filtered and concentrated to give the desired saturated ester which was carried on without further purification.

IR ($CHCl_3$): 2930, 2860, 1720 $cm^{-1}$.

'H NMR ($CDCl_3$): 7.0 to 7.2 (m, 5H); 4.01 (q, J=7.0 Hz, 2H); 2.49 (t, J=7.8 Hz, 2H); 2.17 (t, J=7.6 Hz, 2H); 1.4 to 1.6 (m, 4H); 1.18 to 1.30 (m, 8H); 1.13 (t, J=7.1 Hz, 3H).

$^{13}C$ NMR ($CDCl_3$): 173.5, 142.6, 128.2, 128.0, 127.8, 125.4, 59.9, 35.8, 34.2, 31.3, 29.1, 29.0, 28.9, 28.7, 24.8, 14.1.

m/z Calculated for $C_{17}H_{26}O_2$ ($M^+$): 262.1933, obtained ($EI^+$): 262.1933.

9-Phenylnonan-1-ol

To a stirred solution of ethyl 9-phenylnonanoate (0.884 g, 3.37 mmol) in methylene chloride (10 ml) under argon at 0° was added diisobutylaluminum hydride (8.43 ml of a 1.0 M solution). This mixture was warmed to room temperature and quenched with 10% aqueous hydrochloric acid. The organic portion was washed with 5% sodium bicarbonate, water, saturated sodium chloride solution, dried over magnesium sulfate, filtered and concentrated to give the desired alcohol which was carried on without further purification.

IR ($CHCl_3$): 3610, 3440 (broad), 2910 $cm^{-1}$.

'H NMR ($CDCl_3$): 7.10 to 7.30 (m, 5H); 3.59 (t, J=6.7 Hz, 2H); 2.59 (t, J=7.7 Hz, 2H); 1.6 (broad s, 1H); 1.45 to 1.75 (m, 4H); 1.29 (m, 10H).

$^{13}C$ NMR ($CDCl_3$): 142.8, 128.3, 128.1, 125.5, 62.8, 35.9, 32.7, 31.4, 29.4, 29.3, 29.2, 25.6.

m/z Calculated for $C_{15}H_{24}O$ ($M^+$): 220.1827, obtained ($EI^+$): 220.1831.

1-Bromo-9-phenylnonane

To 1,2 bis(diphenylphosphino)ethane (1.53 g, 3.84 mmol) in methylene chloride (25 ml) at 0° was added bromine (1.23 g, 7.68 mmol) in methylene chloride (4 ml) and stirred for 15 minutes. To this mixture was added 9-phenylnonan-1-ol (0.705 g, 3.20 mmol) in methylene chloride (5 ml). The reaction mixture was allowed to warm to room temperature, stirred for 1 hour and quenched by the addition of ethyl ether/pentane (1:2). This mixture was filtered through silica and concentrated to give the desired bromide which was carried on without further purification.

IR ($CHCl_3$): 3028, 2910, 2850 $cm^{-1}$.

'H NMR ($CDCl_3$): 7.10 to 7.30 (m, 5H); 3.35 (t, J=6.8 Hz, 2H); 2.58 (t, J=7.8 Hz, 2H); 1.75 to 1.87 (m, 2H); 1.55 to 1.67 (m, 2H); 1.20 to 1.45 (m, 10H).

$^{13}C$ NMR ($CDCl_3$): 142.7, 128.3, 128.1, 125.5, 35.9, 33.8, 32.8, 31.4, 29.3, 29.2, 28.7, 28.1, 28.0.

m/z Calculated for $C_{15}H_{23}Br$ ($M^+$): 282.0983, obtained ($EI^+$): 282.0976.

4-(1-Hydroxy-10-phenyldecyl)-2-trimethylsilylfuran

To a stirred solution of 9-phenylnonylmagnesium bromide (1.35 mmol, prepared from 0.383 g, 1.35 mmol of 1-bromo-9-phenylnonane and 4.11 mmol magnesium) in tetrahydrofuran (5 ml) at 0° under argon was added dropwise, 5-trimethylsilyl-3-furaldehyde (0.227 g, 1.35 mmol) in tetrahydrofuran (3 ml). The solution was warmed to room temperature and stirred for 30 minutes, quenched with 5% ammonium chloride and extracted into ethyl ether. The organic portion was washed with saturated sodium bicarbonate, water, saturated sodium chloride solution, dried over magnesium sulfate, filtered and concentrated to give a yellow oil. Purification by flash chromatography (silica, 5 to 20% ethyl acetate/hexane) gave the desired alcohol.

IR ($CHCl_3$) : 3620, 3450 (broad), 2930, 1250 $cm^{-1}$.

'H NMR ($CDCl_3$): 7.51 (s, 1H); 7.10 to 7.30 (m, 5H); 6.61 (s, 1H); 4.58 (t, J=6.7 Hz, 1H); 2.58 (t, J=7.8 Hz, 2H); 2.01 (s, 1H); 1.65 to 1.80 (m, 2H); 1.53 to 1.65 (m, 2H); 1.20 to 1.45 (m, 12H); 0.25 (s, 9H).

$^{13}C$ NMR ($CDCl_3$): 161.2, 143.1, 142.8, 129.2, 128.3, 128.2, 125.5, 118.3, 66.8, 37.9, 35.9, 31.4, 29.5, 29.3, 25.7, -1.7.

m/z Calculated for $C_{23}H_{36}O_2Si$ ($M^+$): 372.2485, obtained ($EI^+$): 372.2496.

4-(1-Acetoxy-10-phenyldecyl)-2-trimethylsilylfuran

A solution of 4-(1-hydroxy-10-phenyldecyl)-2-trimethylsilylfuran (0.178 g, 0.478 mmol), acetic anhydride

(0.5 ml) and pyridine (0.081 g, 0.956 mmol) was stirred at room temperature until no starting material was visible by TLC. The reaction mixture was taken up in ethyl ether, washed repeatedly with saturated sodium bicarbonate, aqueous cupric sulfate solution, water, saturated sodium chloride solution, dried over magnesium sulfate, filtered and concentrated to give the desired acetate which was carried on without further purification.

IR ($CHCl_3$): 2920, 1720, 1250 $cm^{-1}$.

'H NMR ($CDCl_3$): 7.58 (s, 1H); 7.10 to 7.30 (m, 5H); 6.59 (s, 1H); 5.77 (t, J=6.8 Hz, 1H); 2.58 (t, J=7.6 Hz, 2H); 2.01 (s, 3H); 1.70 to 1.95 (m, 2H); 1.54 to 1.68 (m, 2H); 1.15 to 1.39 (m, 12H); 0.24 (s, 9H).

$^{13}C$ NMR ($CDCl_3$): 170.3, 161.0, 144.4, 142.7, 128.3, 128.1, 125.5, 124.9, 118.6, 68.5, 35.9, 34.7, 31.4, 29.4, 29.2, 25.4, 21.1, -1.8.

m/z Calculated for $C_{25}H_{38}O_3Si$ ($M^+$): 414.2590, obtained ($EI^+$): 414.2610.

4-(1-Acetoxy-10-phenyldecyl)-5-hydroxy-2(5H)-furanone

A stirred solution of 4-(1-acetoxy-10-phenydecyl)-2-trimethylsilylfuran (0.167 g, 0.403 mmol) and Rose Bengal (3 mg) in acetone (25 ml) was flushed with oxygen and cooled to -78°. The solution was subsequently irradiated with a 150 watt flood lamp while under constant positive pressure of oxygen until starting material was no longer visible by TLC. The solution was warmed to room temperature and concentrated to a red oil (0.198 g). Purification by flash chromatography (silica, 20 to 40% ethyl acetate/hexane) gave the desired hydroxybutenolide.

IR ($CHCl_3$): 3380 (broad), 3050, 2920, 2850, 1700 (broad) $cm^{-1}$.

'H NMR ($CDCl_3$): (mixture of diastereomers): 7.15 to 7.35 (m, 5H); 6.10 (brs, 1H); 5.96 (s, 1H); 5.78 (brs, 1H); 5.55 (brs, 1H); 2.59 (t, J=7.7 Hz, 2H); 2.11 (s, 3H); 1.69 to 1.90 (m, 2H); 1.50 to 1.67 (m, 2H); 1.15 to 1.40 (m, 12H).

$^{13}C$ NMR ($CDCl_3$): (mixture of diastereomers): 170.8, 170.4, 166.9, 142.7, 128.3, 128.1, 125.4, 118.4 and 118.2, 98.0, 69.5, 35.8, 32.8, 31.3, 29.3, 29.2, 29.1, 29.0, 24.8, 20.7.

m/z Calculated for $C_{22}H_{34}O_5N$ ($M+NH_4^+$): 392.2437, obtained ($CI^+$): 392.2426.

EXAMPLE 36

5-Phenylpent-1-en-3-ol

To vinyl magnesium bromide (550 ml of a 1.0 M solution) was added 3-phenylpropionaldehyde (36.68 g, 0.273 mol) in tetrahydrofuran (300 ml) under argon at 0°. This mixture was stirred for one hour at room temperature, quenched with aqueous ammonium chloride and ice, and extracted into ethyl ether. The organic portion was washed with 5% sodium bicarbonate, water, saturated sodium chloride solution, dried over magnesium sulfate, filtered and concentrated to give the desired alcohol which was carried on without further purification.

**(E)-Ethyl 7-phenyl-4-heptenoate**

A mixture of 5-phenylpent-1-en-3-ol (11.48 g, 70.9 mmol), triethylorthoacetate (80.47 g, 496.0 mmol) and p-toluenesulfonic acid (0.81 g, 4.25 mmol) was refluxed under argon for 5.5 hours. The reaction mixture was cooled to room temperature, diluted with water and stirred for an additional 1.5 hours. This mixture was partitioned between an aqueous saturated sodium bicarbonate solution and ethyl ether. The organic layer was washed with water, dried over magnesium sulfate, filtered and concentrated to give a yellow oil (14.57 g). Purification by flash chromatography (silica, 3% ethyl acetate/hexane) gave the desired ester.

IR ($CHCl_3$): 3028, 2930, 1736 $cm^{-1}$.

'H NMR ($CDCl_3$): 7.1 to 7.3 (m, 5H); 5.35 to 5.55 (m, 2H); 4.13 (q, J=7.4 Hz, 2H); 2.64 (t, J=7.3 Hz, 2H); 2.2 to 2.4 (m, 6H); 1.23 (t, J=6.5 Hz, 3H).

$^{13}C$ NMR ($CDCl_3$): 173.0, 141.8, 130.6, 128.7, 128.3, 128.2, 125.7, 60.1, 35.9, 34.2, 27.8, 14.2.

m/z Calculated for $C_{15}H_{20}O_2$ ($M^+$):232.1463, obtained ($EI^+$): 232.1463.

**(E), (E)-Ethyl 9-phenyl-2,6-nonadienoate and (E), (Z)-Ethyl 9-phenyl-2,6-nonadienoate**

To triethylphosphonoacetate (12.38 g, 55.2 mmol) in tetrahydrofuran (500 ml) at -78° under argon was added n-butyl lithium (36.90 ml of a 1.53 M solution) followed after 20 minutes by (E)-ethyl 7-phenylhept-4-enoate (12.60 g, 54.28 mmol) in tetrahydrofuran (60 ml). After one hour stirring at -78°, diisobutylaluminum

hydride (54.28 ml of a 1.0 M solution) was added dropwise. The mixture was slowly warmed and allowed to stir at room temperature for 15 hours. The reaction was quenched with aqueous sodium sulfate, filtered and concentrated. The residue was taken up in ethyl ether, washed with saturated sodium bicarbonate, water, saturated sodium chloride solution, dried over magnesium sulfate, filtered and concentrated to give a pale oil. Purification by flash chromatography (silica, 3 to 5% ethyl acetate/hexane) gave the desired trans,trans-unsaturated ester and a mixture of cis-trans isomers.

IR ($CHCl_3$): 2930, 1710 $cm^{-1}$.

'H NMR ($CDCl_3$): 7.1-7.3 (m, 5H); 6.94 (dt, J = 15.7 Hz, J = 7.4 Hz, 1H); 5.80 (d, J = 15.7 Hz, 1H); 5.32 to 5.55 (m, 2H); 4.18 (q, J = 7.0 Hz, 2H); 2.66 (t, J = 7.9 Hz, 2H); 2.10 to 2.35 (m, 6H); 1.28 (t, J = 7.1 Hz, 3H).

$^{13}C$ NMR ($CDCl_3$): 166.5, 148.4, 141.8, 130.6, 129.1, 128.4, 128.2, 125.7, 121.5, 60.0, 35.9, 34.2, 32.0, 30.9, 14.2.

m/z Calculated for $C_{17}H_{22}O_2$ ($M^+$): 258.1620, obtained ($EI^+$): 258.1616.

**(E)-Ethyl 9-phenylnon-6-enoate**

To 1.24 g of magnesium stirring in methanol (85 ml) at 0° was added a cis-trans mixture of (E),(Z)-ethyl 9-phenyl-2,6-nonadienoate (4.14 g, 16.96 mmol) in methanol (15 ml). After the magnesium had been consumed the reaction was quenched with 10% HCl at 0° and extracted with ethyl ether. The organic portions were combined, washed with 5% sodium bicarbonate, water, saturated sodium chloride solution, dried over magnesium sulfate, filtered and concentrated to give a pale oil. Purification by flash chromatography (silica, 3 to 5% ethyl acetate/hexane) gave the desired ester.

IR ($CHCl_3$): 3028, 2935, 1735 $cm^{-1}$.

'H NMR ($CDCl_3$): 7.1 to 7.3 (m, 5H); 5.3 to 5.5 (m, 2H); 4.05 (q, J = 7.0 Hz, 2H); 2.63 (t, J = 7.8 Hz, 2H); 2.14 to 2.35 (m, 4H); 1.8 to 2.0 (m, 2H); 1.45 to 1.65 (m, 2H); 1.2 to 1.4 (m, 2H); 1.27 (t, J = 7.0 Hz, 3H).

**(E)-9-Phenylnon-6-en-1-ol**

To a stirred solution of (E)-ethyl 9-phenylnon-6-enoate (1.45 g, 5.53 mmol) in methylene chloride (35 ml) under argon at 0° was added diisobutylaluminum hydride (15.0 ml of a 1.0 M solution). This mixture was warmed to room temperature and quenched with 10% aqueous hydrochloric acid. The organic portion was washed with saturated sodium bicarbonate solution, water, saturated sodium chloride solution, dried over magnesium sulfate, filtered and concentrated to give a colorless oil. Purification by flash chromatography (silica, 20% ethyl acetate/hexane) gave the desired alcohol.

IR ($CDCl_3$): 3622, 3450 (broad), 3028, 2932 $cm^{-1}$.

'H NMR ($CDCl_3$): 7.07 to 7.27 (m, 5H); 5.30 to 5.50 (m, 2H); 3.53 (t, J = 6.6 Hz, 2H); 3.29 (s, 1H); 2.63 (t, J = 7.8 Hz, 2H); 2.15 to 2.35 (m, 2H); 1.90 to 2.05 (m, 2H); 1.40 to 1.55 (m, 2H); 1.20 to 1.40 (m, 4H).

$^{13}C$ NMR ($CDCl_3$): 141.8, 130.6, 129.2, 128.2, 127.9, 125.4, 62.2, 35.8, 34.2, 32.3, 29.1, 25.0.

m/z Calculated for $C_{15}H_{26}ON$ $(M + NH_4)^+$: 236.2014, obtained ($CI^+$): 236.2024.

**(E)-1-Bromo-9-phenylnon-6-ene**

To 1,2-bis(diphenylphosphino)ethane (0.918 g, 2.30 mmol) in methylene chloride (12 ml) at 0° was added bromine (0.736 g, 4.60 mmol) in methylene chloride (4 ml) and stirred for 15 minutes. To this mixture was added (E)-9-phenylnon-6-en-1-ol (0.419 g, 1.92 mmol) in methylene chloride (5 ml). The reaction mixture was allowed to warm to room temperature, stirred for 1 1/2 hours and quenched by the addition of ethyl ether/pentane (1:2). This mixture was passed through silica to filter off the solid residues and the filtrate was concentrated to give the desired bromide which was carried on without further purification.

IR ($CHCl_3$) : 3000, 2930, 2860 $cm^{-1}$.

'H NMR ($CDCl_3$): 7.13 to 7.33 (m, 5H); 5.35 to 5.52 (m, 2H); 3.38 (t, J = 6.9 Hz, 2H); 2.67 (t, J = 7.8 Hz, 2H); 2.25 to 2.40 (m, 2H); 1.95 to 2.05 (m, 2H); 1.78 to 1.90 (m, 2H); 1.27 to 1.45 (m, 4H).

$^{13}C$ NMR ($CDCl_3$): 142.1, 130.6, 129.8, 128.4, 128.2, 125.7, 36.1, 34.3, 33.8, 32.7, 32.2, 28.6, 27.6.

m/z Calculated for $C_{15}H_{21}Br$ ($M^+$): 280.0827, obtained ($EI^+$): 280.0822.

**(E)-4-(1-Hydroxy-10-phenyldec-7-enyl)-2-trimethylsilylfuran**

To a stirred solution of (E)-9-phenylnon-6-enyl magnesium bromide (0.861 mmol, prepared from 0.242 g, 0.861 mmol, of (E)-1-bromo-9-phenylnon-6-ene and 2.88 mmol magnesium) in tetrahydrofuran (10 ml) at 0° under argon was added dropwise, 5-trimethylsilyl-3-furaldehyde (0.145 g, 0.861 mmol) in tetrahydrofuran

(5 ml). The solution was warmed to room temperature, stirred for 5 hours, quenched with 5% ammonium chloride and extracted into ethyl ether. The organic portion was washed with 5% sodium bicarbonate, water, saturated sodium chloride solution, dried over magnesium sulfate, filtered and concentrated to give yellow oil. Purification by flash chromatography (silica, 5 to 20% ethyl acetate/hexane) gave the desired alcohol.

IR ($CHCl_3$): 3600, 3440 (broad), 2930, 1250 $cm^{-1}$.

'H NMR ($CDCl_3$): 7.55 (s, 1H); 7.12 to 7.35 (m, 5H); 6.62 (s, 1H); 5.37 to 5.50 (m, 2H); 4.63 (t, J=6,7 Hz, 1H); 2.66 (t, J=7,8 Hz, 2H); 2.25 to 2.40 (m, 2H); 1.90 to 2.05 (m, 2H); 1.60 to 1.80 (m, 3H); 1.20 to 1.50 (m, 6H); 0.25 (s, 9H).

$^{13}C$ NMR ($CDCl_3$): 161.4, 143.1, 142.1, 130.9, 129.4, 129.2, 128:4, 128.2, 125.6, 118.2, 67.0, 37.8, 36.1, 34.4, 32.4, 29.4, 28.9, 25.6, -1.7.

m/z Calculated for $C_{23}H_{34}O_2Si$ ($M^+$): 370.2328, obtained ($EI^+$): 370.2332.

**(E)-4-(1-Acetoxy-10-phenyldec-7-enyl)-2-trimethylsilylfuran**

A solution of 4-(1-hydroxy-10-phenyldec-7-enyl)-2-trimethylsilylfuran(0.136 g, 0.367 mmol), acetic anhydride (1 ml) and pyridine (0.062 g, 0.735 mmol) was stirred at room temperature until no starting material was visible by TLC. The reaction mixture was taken up in ethyl ether, washed repeatedly with saturated sodium bicarbonate solution, aqueous cupric sulfate solution, water, saturated sodium chloride solution, dried over magnesium sulfate, filtered and concentrated to give the desired acetate which was carried on without further purification. [Yield: 0.133 g, 88%.]

IR ($CHCl_3$): 2920, 1720, 1250 $cm^{-1}$.

'H NMR ($CDCl_3$): 7.59 (s, 1H); 7.12 to 7.22 (m, 5H); 6.58 (s, 1H); 5.76 (t, J=7,0 Hz, 1H); 5.35 to 5.49 (m, 2H); 2.66 (t, J=7,8 Hz, 2H); 2.25 to 2.37 (m, 2H); 2.05 (s, 3H); 1.65 to 2.05 (m, 4H); 1.20 to 1.40 (m, 6H); 0.25 (s, 9H).

$^{13}C$ NMR ($CDCl_3$): 170.5, 161.2, 144.4, 142.1, 130.9, 129.5, 128.4, 128.2, 125.7, 125.0, 118.6, 68.6, 36.1, 34.7, 34.4, 32.4, 29.3, 28.7, 25.4, 21.3, -1.7.

m/z Calculated for $C_{25}H_{36}O_3Si$ ($M^+$): 412.2434, obtained ($EI^+$): 412.2433.

**(E)-4-(1-Acetoxy-10-phenyldec-7-enyl)-5-hydroxy-2(5H)-furanone**

A stirred solution of 4-(1-acetoxy-10-phenyldec-7-enyl)-2- trimethylsilylfuran (0.133 g, 0.322 mmol) and Rose Bengal (3 mg) in acetone (10 ml) was flushed with oxygen and cooled to -78°. The solution was subsequently irradiated with a 150 watt flood lamp while under constant positive pressure of oxygen until starting material was no longer visible by TLC. The solution was warmed to room temperature and concentrated to a pink. Purification by flash chromatography (silica, 20 to 40% ethyl acetate/hexane) gave the title compound.

IR ($CHCl_3$): 3400 (broad), 3020, 2920, 1750 $cm^{-1}$.

'H NMR ($CDCl_3$): (mixture of diastereomers): 7.08 to 7.30 (m, 5H); 6.05 (broad s, 1H); 5.97 (s, 1H); 5.67 (broad s, 1H); 5.43 to 5.57 (broad m, 1H); 5.31 to 5.43 (m, 2H); 2.66 (t, J=7.8 Hz, 2H); 2.20 to 2.33 (m, 2H); 2.12 (s, 3H); 1.88 to 2.00 (m, 2H); 1.68 to 1.87 (m, 2H); 1.18 to 1.43 (m, 6H).

$^{13}C$ NMR ($CDCl_3$): (mixture of diastereomers): 170.8, 170.3, 166.9, 142.0, 130.6, 129.6, 128.3, 128.1, 125.6, 118.6 and 118.3, 98.0, 69.6, 36.0, 34.2, 32.8, 32.2, 29.1, 28.4, 24.7, 20.7.

m/z Calculated for $C_{22}H_{32}O_5N$ $(M+NH_4)^+$: 390.2280, obtained ($CI^+$): 390.2281.

EXAMPLE 37

1-(4-Bromophenyl)pent-1-yne

A solution of 4-bromo-iodobenzene (4.23 g, 14.9 mmol), palladium (II) acetate (5 mg), triphenylphosphine (5 mg) in triethylamine (40 ml) was deareated with argon for 5 minutes. After 1-pentyne (1.77 ml, 18 mmol) was added, the solution was rapidly heated to reflux under argon and conditions maintained for 16 hours. On cooling, the mixture was poured into excess ice cold dilute hydrochloric acid and extracted with ethyl ether. Evaporation of the dried (magnesium sulphate) extracts gave an oil, which was flash chromatographed on silica using 5% ethyl ether/hexane. The title acetylene was obtained as a light brown oil (3.27 g, 99%).

$^{1}H$ NMR ($CDCl_3$): 1.07 (t, 3H, J=7.4 Hz), 1.63 (sextet, 2H, J=8,3 Hz), 2.39 (t, 2H, J=7.1 Hz), 7.29 (d, 2H, J=7.8 Hz) and 7.42 (d, 2H, J=7.8 Hz).

MS m/e (% abundance) 224/222 ($M^+$, 65, 67), 195 (60), 193 (61), 143 (48), 128 (100) and 114 (38).

4-[1-Acetoxy-1-[4-(pent-1-ynyl)phenyl]methyl]-2-trimethylsilylfuran

tert-Butyl lithium (a 1.7 M solution in pentane; 5.27 ml, 8.9 mmol) was added dropwise to a solution of 1-(4-bromophenyl)pent-1-yne (1 g, 4.48 mmol) in tetrahydrofuran (15 ml) at -78° under argon. After 25 minutes, a solution of 5-trimethylsilyl-3-furaldehyde (753 mg, 4,48 mmol) in tetrahydrofuran (2 ml) was added, followed by acetic anhydride (1.26 ml, 1.35 mmol) after 1 hour. The mixture was allowed to warm to room temperature gradually over 4 hours and quenched with water. Extraction (ethyl ether) and evaporation of the dried (magnesium sulphate) extract gave an oil, which was flash chromatographed using 5% ethyl ether/hexane. Fractions with $R_f$ of about 0.22 on evaporation afforded the title acetate as a light yellow oil.

$^1$H NMR ($CDCl_3$): 0.15 (s, 9H), 0.97 (t, 3H, J = 7.3 Hz), 1.56 (sextet, 2H, J = 8.3 Hz), 2.04 (s, 3H), 2.31 (t, 2H, J = 7.0 Hz), 6.41 (s, 1H), 6.71 (s, 1H), 7.22 (d, 2H, J = 8.3 Hz), 7.31 (d, 2H, J = 8.3 Hz) and 7.34 (s, 1H).

MS m/e (% abundance) 354 ($M^+$, 62), 312 (45), 295 (47), 222 (15), 169 (24), 144 (11), 117 (12), 83 (23) and 73 (100).

4-[1-Acetoxy-1-[4-(pent-1-ynyl)phenyl]methyl]-5-hydroxy-2(5H)-furanone

A mixture of 4-[1-acetoxy-1-[4-(pent-1-ynyl)phenyl]methyl]-2-trimethylsilylfuran (271.5 mg) and Rose Bengal (5 mg) in tetrahydrofuran (7 ml) was exposed to singlet oxygen at -78° for 2 hours. The residue, after solvent removal, was purified by preparative TLC (20x20 cm, 1000μ, developed with 60% ethyl ether/hexane). The title furanone was obtained as a yellow oil.

$^1$H NMR ($CDCl_3$): 1.08 (t, 3H, J = 7.4 Hz), 1.66 (sextet, 2H, J = 6.8 Hz), 2.17 (s, 3H), 2.19 (s, 3H), 2.42 (t, 2H, J = 6.8 Hz), 4.45 (br, 1H), 4,75 (br, 1H), 5.70 (d, 1H, J = 8.6 Hz), 5.85 (s, 1H), 6.19 (s, 1H), 6.28 (d, 1H, J = 8.6 Hz), 6.55 (brs, 1H), 7.32 (d, 1H, J = 8.2 Hz) and 7.45 (d, 2H, J = 8.3 Hz).

$^{13}$C NMR (mixture of diasteriomers) ($CDCl_3$): 13.4, 20.7, 20.8, 21.1, 21.3, 22.0, 25.5, 70.4, 71.5, 79.8, 80.0, 91.6, 91.9, 97.5, 97.9, 117.3, 120.9, 125.11, 125.5, 127.3, 127.6, 131.9, 132.2, 134.0, 134.7, 165.7, 165.9, 169.9, 170.2 and 170.4.

MS m/e: exact mass calculated for $C_{18}H_{18}O_5$ ($M^+$) 314.1154, found 314.1169.

EXAMPLE 38

4-[1-Acetoxy-1-(4-pentylphenyl)methyl]-2-trimethylsilylfuran and 4-[1-Acetoxy-1-(4-pentylphenyl)methyl]-5-hydroxy-2(5H)-furanone

A solution of 4-[1-acetoxy-1-[4-(pent-1-ynyl)phenyl]methyl]-2-trimethylsilylfuran (187.5 mg, 0.53 mmol) in ethyl ether (10 ml) was hydrogenated over Lindlar catalyst (10 mg) for 2-1/2 hours at room temperature. The mixture was filtered through celite and on evaporation gave the title acetate, which was used in the next step.

$^1$H NMR ($CDCl_3$): 0.26 (s, 9H), 0.92 (t, 3H, J = 6.7 Hz), 1.35 (m, 4H), 1.65 (p, 2H, J = 7.5 Hz), 2.14 (s, 3H), 2.62 (t, 2H, J = 8.0 Hz), 6.56 (s, 1H), 6.82 (s, 1H), 7.19 (d, 2H, J = 8.0 Hz), 7.32 (d, 2H, J = 8.0 Hz) and 7.46 (s, 1H).

MS m/e (% abundance) 358 ($M^+$, 40), 343 (8), 316 (40), 299 (38), 245 (15), 169 (20), 117 (15), 75 (31) and 73 (100).

A mixture of 4-[1-acetoxy-1-(4-pentylphenyl)methyl]-2-trimethylsilylfuran (from above) and Rose Bengal (5 mg) in tetrahydrofuran (7 ml) was exposed to singlet oxygen at -78° for 2 hours. The residue, after solvent removal, was purified by preparative TLC (20x20 cm, 1000μ silica plate; developed with 60% ethyl ether/hexane). The title furanone was obtained as a yellow oil

$^1$H NMR (mixture of diasteriomers) ($CDCl_3$): 0.93 (t, 3H, J = 6.5 Hz), 1.35 (m, 4H), 1.65 (m, 2H), 2.17 (s, 3H), 2.20 (s, 3H), 2.65 (t, 2H, J = 8.2 Hz), 4.39 (d, 1H, J = 9.2 Hz), 5.74 (d, 1H, J = 9.2 Hz), 5.90 (s, 1H), 6.19 (brs, 1H), 6.26 (d, 1H), 6.54 (brs, 1H) and 6.57 (s, 1H).

$^{13}$C NMR (mixture of diasteriomers) ($CDCl_3$): 13.9, 20.7, 20.9, 22.4, 30.9, 31.4, 35.6, 70.7, 71.8, 76.6, 97.6, 98.0, 117.0, 120.8, 127.4, 127.7, 128.0, 128.9, 129.2, 132.1, 144.6, 166.5, 170.1, 170.2 and 170.5.

MS m/e: exact mass calculated for $C_{16}H_{18}O_3$ ($M^+$-HOAc) 258.1256, found 258.1246.

EXAMPLE 39

4-(1-Acetoxyundeca-2,6-diynyl)-2-trimethylsilylfuran

n-Butyl lithium (a 1.6 M solution in hexane; 0.98 ml, 1.56 mmol) was added dropwise to a solution of

1,5-decadiyne (199.7 mg, 1.49 mmol) in tetrahydrofuran (5 ml) at 0° under argon. After 30 minutes, a solution of 5-trimethylsilyl-3-furaldehyde (250 mg, 1.49 mmol) in tetrahydrofuran (250 mg, 1.49 mmol) was added, followed by acetic anhydride (0.42 ml) after 1 hour. The mixture was stirred at room temperature for 4 hours and quenched with water. Extraction (ethyl ether) and evaporation of the dried (magnesium sulphate) extracts gave an oil, which was flash chromatographed on silica using 10% ethyl ether/petroleum ether. Fractions with $R_f$ of about 0.5 on evaporation afforded the title acetate as a light yellow oil.

$^1$H NMR ($CDCl_3$): 0.30 (s, 9H), 0.94 (t, 3H, J=6.9 Hz), 1.45 (m, 4H), 2.14 (s, 3H), 2.15 (m, 2H), 2.45 (m, 4H), 6.45 (brs, 1H), 6.72 (s, 1H) and 7.85 (s, 1H).

MS m/e (% abundance) 344 ($M^+$, 7), 301 (3), 285 (10), 242 (4), 207 (4), 169 (16), 75 (23) and 73 (100).

4-(1-Acetoxyundeca-2.6-diynyl)-5-hydroxy-2(5H)-furanone

A mixture of 4-(1-acetoxyundeca-2,6-diynyl)-2-trimethylsilylfuran (240 mg, 0.70 mmol) and Rose Bengal (5 mg) in tetrahydrofuran (7 ml) was exposed to singlet oxygen at -78° for 2 hours. The residue, after solvent removal, was purified by preparative TLC (20x20 cm, 1000µ silica plate; developed with 60% ethyl ether/hexane). The title furanone was obtained as a light yellow oil.

$^1$H NMR ($CDCl_3$): 0.92 (t, 3H, J=7.2 Hz), 1.40 (m, 4H), 2.17-2.19 (2s + brs, 5H), 2.43 (brm, 4H), 4.15 (d, 1H), 4.25 (d, 1H), 5.32 (brs, 1H), 6.10-6.25 (brs + d, 2H) and 6.40 (brs, 1H).

$^{13}$C NMR ($CDCl_3$): 13.5, 18.2, 18.4, 19.2, 20.6, 21.8, 30.8, 58.6, 59.5, 73.3, 73.9, 81.8, 88.0, 88.3, 97.2, 97.3, 97.6, 118.7, 122.1, 162.6, 162.9, 169.6, 169.6 and 170.0.

MS m/e: exact mass calculated for $C_{17}H_{20}O_5$ ($M^+$) 304.1311, found 304.1316.

EXAMPLE 40

5-(2-Pyridyl)-4-pentyn-1-ol

A mixture of 2-bromopyridine (5 g, 31.6 mmol), palladium (II) acetate (5 mg), copper (I) iodide (5 mg), triphenylphosphine (5 mg) in triethylamine (40 ml) was deaerated with argon for 5 minutes. After 4-pentyn-1-ol (3.49 ml, 37.9 mmol) was added, the solution was rapidly heated to reflux under argon and conditions maintained for 22 hours. On cooling, the mixture was poured into 2M sodium hydroxide (ca. 30 ml) and extracted thoroughly with ethyl acetate. Evaporation of the dried (magnesium sulphate) extracts gave an oil, which was flash chromatographed on silica using 60% ethyl acetate/chloroform. Fractions with $R_f$ of about 0.12 on evaporation gave the title alcohol as a pale brown oil.

$^1$H NMR ($CDCl_3$): 1.91 (p, 2H, J=6.4 Hz), 2.20 (br, 1H), 2.60 (t, 2H, J=7.0 Hz), 3.85 (t, 2H, J=6.1 Hz), 7.18 (m, 1H), 7.30 (dd, 1H, J=7.8 Hz, 1.2 Hz), 7.64 (dt, 1H, J=7.7 Hz, 1.9 Hz) and 8.55 (d, 1H, J=4.8 Hz).

$^{13}$C NMR ($CDCl_3$): 15.7, 30.9, 60.6, 80.1, 90.7, 122.2, 126.7, 136.1, 143.3 and 149.2.

MS m/e (% abundance) 161 ($M^+$, 25), 142 (11), 131 (100), 130 (99), 117 (44), 104 (17), 89 (18) and 78 (21).

5-(2-Pyridyl)-1-pentanol

A solution of 5-(2-pyridyl)-4-pentyn-1-ol (1 g, 6.2 mmol) in diethyl ether (15 ml) was hydrogenated over 10% palladium on carbon at room temperature for 16 h. The mixture was filtered through celite and the filtrate on evaporation gave an oil, which was flash chromatographed on silica using 80% ethyl acetate/chloroform. Fractions with $R_f$ of about 0.06 on evaporation gave the title alcohol as a yellow oil.

$^1$H NMR ($CDCl_3$): 1.49 (p, 2H, J=8.8 Hz), 1.66 (p, 2H, J=7.3 Hz), 1.82 (p, 2H, J=7.5 Hz), 2.86 (t, 2H, J=7.8 Hz), 2.95 (br, 1H), 3.71 (t, 2H, J=6.3 Hz), 7.15 (m, 2H), 7.65 (dt, 1H, J=9.2 Hz, 1.9 Hz) and 8.55 (d, 1H, J=4.9 Hz).

$^{13}$C NMR ($CDCl_3$): 25.1, 29.2, 32.1, 37.6, 61.4, 120.5, 122.4, 136.1, 148.3 and 161.6.

MS m/e (% abundance) 166 ($M^+$, 18), 120 (17), 106 (22) and 93 (100).

2-(5-Bromopentyl)pyridine

A mixture of 5-(2-pyridyl)-1-pentanol (680 mg, 4.12 mmol) and 48% hydrobromic acid (10 ml) was warmed at ca. 90° for 22 h. On cooling, the mixture was neutralized with sodium bicarbonate. Extraction (ethyl acetate) and evaporation of the dried (magnesium sulphate) extracts gave an oil, which was flash chromatographed on silica using 40% ethyl acetate/hexane. Fractions with $R_f$ of about 0.24 on evaporation gave the title bromide as a yellow oil.

$^1$H NMR ($CDCl_3$): 1.54 (p, 2H, J=7.3 Hz), 1.80 (p, 2H, J=7.3 Hz), 1.94 (p, 2H, J=7.3 Hz), 2.83 (t, 2H, J=7.7 Hz), 3.44 (t, 2H, J=6.9 Hz), 7.15 (m, 2H), 7.63 (t, 1H, J=7.7 Hz) and 8.55 (d, 1H, J=4.4 Hz).

MS m/e (% abundance) 148 ($M^+$ -br, 9), 120 (16), 106 (10) and 93 (100).

4-[1-Acetoxy-6-(2-pyridyl)hexyl]-2-trimethylsilylfuran

tert-Butyl lithium (a 1.7 M solution in pentane; 2.4 ml, 4.14 mmol) was added dropwise to a solution of 2-(5-bromopentyl)pyridine (472.3 mg, 2.07 mmol) in tetrahydrofuran (7 ml) at -78° under argon. After 1 hour, a solution of 5-trimethylsilyl-3-furaldehyde (348 mg, 2.07 mmol) in tetrahydrofuran (1 ml) was added, followed by acetic anhydride (0.59 ml, 6.21 mmol) after 2 hours. The mixture was allowed to warm to room temperature and quenched with water (after 22 hours). Extraction (ethyl ether) and evaporation of the dried (magnesium sulphate) extracts gave an oil, which was flash chromatographed on silica using 40% ethyl acetate/hexane. Fractions with $R_f$ of about 0.25 on evaporation gave the title silylfuran as a light brown oil.

$^1$H NMR (mixtures of diasteriomers) ($CDCl_3$): 0.28 (s, 9H), 1.35 (brm, 4H), 1.75 (brm, 4H), 2.07 (s, 3H), 2.13 (s, 3H), 2.80 (brt, 2H), 5.79 (t, 1H, J=7.0 Hz), 6.61 (s, 1H), 7.15 (brm, 2H), 7.65 (s+m, 2H) and 8.57 (brd, 1H).

MX m/e (% abundance) 359($M^+$, 2), 344(7), 317(14), 316(38), 300(11), 299(15), 117(11), 107(17), 106-(46), 93(100) and 73(48).

4-[1-Acetoxy-6-(2-pyridyl)hexyl]-5-hydroxy-2(5H)-furanone

A mixture of 4-[1-acetoxy-6-(2-pyridyl)hexyl]-2-trimethylsilylfuran (115 mg, 0.32 mmol) and Rose Bengal (5 mg) in tetrahydrofuran (6 ml) was exposed to singlet oxygen at -78° for 2 h. The residue, after solvent removal, was purified by preparative TLC (20x20 cm, 1000 $\mu$ silica plate; developed with 80% ethyl acetate/hexane). The title furanone was obtained as a pale yellow oil.

$^1$H NMR (mixture of diasteriomers) ($CDCl_3$): 1.40 (brm, 4H), 1.70 (m, 2H), 1.85 (m, 2H), 2.07 (s, 3H), 2.12 (s, 3H), 2.81 (t, 2H, J=7.3 Hz), 5.60 (br, 1H), 5.98 (s, 1H), 6.21 (br, 1H), 7.20 (m, 2H), 7.69 (t, 1H, J=7.6 Hz) and 8.48 (d, 1H, J=4.8 Hz).

$^{13}$C NMR ($CDCl_3$): 20.8, 24.6, 28.7, 29.7, 32.7, 37.4, 69.5, 98.4, 118.4, 121.6, 123.4, 137.6, 148.1, 161.5, 167.1 and 170.3.

MS exact mass caluculated for $C_{17}H_{20}NO_5$ $(M-H)^+$ 318.1341, found 318.1339.

EXAMPLE 41

5-(2-Naphthyl)-pent-4-yn-1-ol

A mixture of 2-bromonaphthalene (5 g, 24.1 mmol), 4-pentyn-1-ol (2.67 ml, 28.9 mmol), bis-(triphenylphosphine) palladium (II) chloride (30 mg) and copper (I) iodide (10 mg) in triethylamine (40 ml) was refluxed under argon for 15 hours. After cooling, the mixture was acidified with ice-cold dilute hydrochloric acid and extracted with ethyl ether. Evaporation of the dried (magnesium sulfate) extracts gave an oil, which was purified by flash chromatography on silica using 60% ethyl ether/hexane. Fractions with $R_f$ of about 0.32 on evaporation gave a solid, which was recrystallized from dichloromethane/hexane to give the title alkynol as an off-white solid, mp 37-8°.

$^1$H NMR ($CDCl_3$): 1.60 (br, 1H), 1.94 (p, 2H, J=6.4 Hz), 2.63 (t, 2H, J=7.0 Hz), 3.89 (brm, 2H), 7.50-7.80 (m, 6H) and 7.94 (brs, 1H).

$^{13}$C NMR ($CDCl_3$): 15.8, 31.2, 61.2, 81.3, 89.8, 120.9, 126.1, 126.2, 127.4, 127.5, 127.7, 128.4, 130.9, 132.2 and 132.8.

MS m/e (% abundance) 210 ($M^+$, 96), 191 (34), 178 (36), 165 (100), 154 (54), 141 (27), 128 (23) and 115 (9).

5-(2-Napthyl)pentan-1-ol

A mixture of 5-(2-napthyl)-4-pentyn-1-ol (520 mg, 247 mmol) and 10% palladium on activated carbon in ethyl ether (50 ml) was stirred at room temperature under a hydrogen atmosphere for 2 days. Solvent removal, after filtration through celite, gave the titled compound as a light yellow oil.

$^1$H NMR ($CDCl_3$): 7.78 (m, 3H), 7.6 (s, 1H), 7.4 (m, 3H), 3.64 (t, 2H, J=6.5 Hz), 2.79 (t, 2H, J=7.4 Hz), 1.7 (m, 2H), 1.6 (m, 2H) and 1.4 (m, 2H).

1-Bromo-5-(2-napthyl)-pentane

Bromine (a 2 M solution in dichloromethane; 955 mg, 5.97 mmol) was added to a solution of 1,2-bis (diphenyl)phosphinoethane (1.16 g, 2.99 mmol) in dichloromethane (15 ml) at 0°. A solution of 5-(2-napthyl)pentan-1-ol (513 mg, 2.39 mmol) in dichloromethane (5 ml) was added to the 0° solution. The solution was warmed to room temperature and stirred for 1.5 hours. The solution was diluted with ethyl ether/pentane (1:3), filtered through a thin pad of celite and the solvent evaporated. The resulting oil was purified by flash chromatography (hexane) giving the title compound as a colorless oil.

$^1$H NMR ($CDCl_3$): 7.75 (m, 3H), 7.6 (s, 1H), 7.4 (m, 3H), 3.4 (t, 2H, J = 6.8 Hz), 2.78 (t, 2H, J = 7.7 Hz), 1.9 (m, 2H), 1.7 (m, 2H) and 1.5 (m, 2H).

3-[1-Hydroxy-6-(2-napthyl)-hexyl]-4-trimethylsilylfuran

t-BuLi (a 1.7 M solution in hexane; 2.35 ml, 4.00 mmol) was added to a -78° solution of 1-bromo-5-(2-napthyl)pentane (555 mg, 2.00 mmol) in terahydrofuran (4 ml). After stirring for 30 minutes at -78°, a solution of 5-trimethylsilyl-3-furaldehyde (336 mg, 2.0 mmol) in tetrahydrofuran (0.5 ml) was added, and the solution stirred overnight at room temperature. The reaction was quenched with water. Extraction (ethyl ether) and evaporation of the dried (magnesium sulfate) extracts gave an oil, which was flash chromatographed on silica gel (30% ethyl ether/hexane). The title compound was obtained as a light yellow oil.

$^1$H NMR ($CDCl_3$): 7.8 (m, 3H), 7.59 (s, 1H), 7.55 (s, 1H), 7.4 (m, 3H), 6.61 (s, 1H), 4.62 (t, 1H, J = 4.0 Hz), 2.76 (t, 2H, J = 7.6 Hz), 1.7 (m, 4H), 1.4(m,4H) and 0.26 (s, 9H).

$^{13}$C NMR ($CDCl_3$) 161.2, 143.0, 140.1, 133.5, 131.8, 129.0, 127.6, 127.5, 127.3, 126.2, 125.7, 124.9, 118.2, 66.7, 37.7, 35.9, 31.1, 28.9, 25.5, -1.7.

3-[1-Acetoxy-5-(2-napthyl)hexyl]-5-trimethylsilylfuran

A mixture of 3-[1-hydroxy-5-(2-napthyl)hexyl]-5-trimethylsilylfuran (120 mg, 0.3 mmol) and acetic anhydride (66 mg, 651 mmol) in pyridine (1.3 ml) was stirred at room temperature overnight. After removal of the solvent, the residue was purified by preparative TLC (20x20 cm, 500 $\mu$ silica gel plate; developed with 30% ethyl ether/hexane) yielding the title compound as a yellow oil.

$^1$H NMR ($CDCl_3$): 7.8 (m, 3H), 7.59 (d, 2H, J = 3.2 Hz), 7.43 (m, 2H), 7.3 (m, 1H), 6.57 (s, 1H), 5.76 (t, 1H, J = 6.6 Hz), 2.75 (t, 2H, J = 7.7 Hz), 2.05 (s, 3H), 1.8 (m, 4H), 1.45 (m, 4H) and 0.24 (s, 9H)

$^{13}$C NMR ($CDCl_3$): 170.4, 161.1, 144.4, 140.0, 133.5, 131.9, 127.7, 127.5, 127.3, 126.2, 125.8, 124.9, 124.8, 118.6, 68.4, 35.9, 34.6, 31.1, 28.8, 25.4, 21.2, -1.7.

MS exact mass calculated for $C_{25}H_{32}O_3Si$ ($M^+$) 408.2120, found 408.2121.

4-[1-Acetoxy-6-(2-naphthyl)hexyl]-5-hydroxy-2(5H)-furanone

Oxidizing the above prepared trimethylsilylfuran by the procedure of Example 40 gave the title compound.

$^1$HNMR ($CDCl_3$) (mixture of diastereomers) 7.78 (m,2H), 7.59(brs, 1H) 7.45(m, 2H), 7.3(m, 1H), 6.17(brs, 1H), 5.97(brs, 2H), 5.47(t, 1H), 5.4j0((t, 1H), 5.05(brs, 1H), 4.65(brs, 1H), 2.a76(t, 2H, J = 7.8Hz), 2.1(s, 3H), 2.08(s,3H), 1.7(m, 4H) and 1.35(m,4H).

$^{13}$CNMR ($CDCl_3$) 170.9, 166.9, 139.8, 139.7, 133.5, 131.8, 127.7, 127.5, 127.3, 127.2, 126.2, 125.8, 125.1, 125.0, 118.9, 118.1, 98.1, 97.6, 76.6, 69.7, 69.1, 35.8, 32.7, 30.8, 28.6, 24.7 and 20.7.

MS exact mass calculated for $C_{22}H_{24}O_5$ ($M^+$) 368.1623, found 368.1621.

EXAMPLE 42

2-(2-Naphthyl)ethanol is reacted with methanesulfonyl chloride in the presence of triethylamine to give the methanesulfonyloxyethyl compound. Reacting this intermediate with 3-propyn-1-ol, then brominating gives 2-(5-bromopent-3-ynyl)naphthalene. Reacting this intermediate with t-butyl lithium and 5-trimethylsilyl-3-furaldehyde and then oxidizing by the procedure of Example 45 gives 4-[1-acetoxy-6-(2-naphthyl)hex-3-ynyl]-5-hydroxy-2(5H)-furanone.

EXAMPLE 43

Using the procedure of Example 41, except that the hydrogenation step is omitted, the product is 4-[1-acetoxy-6-(2-naphthyl)hex-5-ynyl]-5-hydroxy-2(5H)-furanone,

EXAMPLE 44

4-[1-Acetoxy-6-(2-pyridyl)hexyl]-5-hydroxy-2(5H)-furanone is reacted with methyl iodide to give the N-methyl ammonium iodide salt.

EXAMPLE 45

Using 2-chloroquinoline in place of 2-bromopyridine in the procedure of Example 40, the product is 4-[1-acetoxy-6-(2-quinolyl)hexyl]-5-hydroxy-2(5H)-furanone.

EXAMPLE 46

Beginning with 2-bromopyridine and carrying out the procedure of Example 40 except omitting the second step (hydrogenating), the product is 4-[1-acetoxy-6-(2-pyridyl)hex-5-ynyl]-5-hydroxy-2(5H)-furanone.

EXAMPLE 47

4-Bromobenzaldehyde is converted to 4-(5-hydroxypent-1-ylyl)benzaldehyde by the procedure of Example 40. This intermediate is brominated, then oxidized (Jones oxidation) to give the 4-substituted benzoic acid. Treating with t-butyl lithium and 5-trimethylsilyl-3-furaldehyde and then with acetic anhydride, hydrogenating the triple bond, then oxidizing gives 4-[1-acetoxy-6-(4-carboxyphenyl)hexyl]-5-hydroxy-2(5H)-furanone.

EXAMPLE 48

1-(2-Bromophenyl)tridec-1-yne

A mixture of 1-bromo-2-iodobenzene (5 g, 17.7 mmol), 1-tridecyne (4.9 ml, 21.2 mmol), triphenylphosphine (5 mg), palladium (II) chloride (5 mg) in triethylamine (45 ml) was refluxed under argon for 15 h. After cooling, the mixture was poured into ice-cold dilute hydrochloric acid and extracted thoroughly with ethyl ether. Evaporation of the dried (magnesium sulfate) extracts gave an oil, which was purified by a silica column using hexane. Fractions with $R_f$ of about 0.36 on evaporation gave the title alkyne as a brown oil.

$^1$H NMR ($CDCl_3$): 0.89 (t, 3H, J-6.9 Hz), 1.28 (brs, 14H), 1.50 (m, 2H), 1.65 (m, 2H), 2.48 (t, 2H, J = 7.0 Hz), 7.12 (dt, 1H, J = 7.7 Hz, 1.7 Hz), 7.24 (dt, 1H, J = 8.1 Hz, 1.2 Hz), 7.44 (dd, 1H, J = 7.7 Hz, 1.8 Hz) and 7.56 (dd, 1H, J = 8.1 Hz, 1.2 Hz).

MS m/e (% abundance) 335/337 [(M + $H^+$), 21/19], 334/336 ($M^+$, 26/29), 235/237 (39/42), 221/223 (45/48), 195/197 (63/67), 169/171 (49/51), 129 (100), 116 (78), 109 (32) and 95 (96).

3-[1-Acetoxy-1-(2-(1-tridecynyl)phenyl)methyl]-5-trimethylsilylfuran

tert-Butyl lithium (a 1.7 M solution in pentane; 2.44 ml, 4.1 mmol) was added dropwise to a solution of 1-(2-bromophenyl)tridec-1-yne (692 mg, 2.07 mmol) in tetrahydrofuran (5 ml) at -78° under argon. After 30 min, a solution of 5-trimethylsilyl-3-furaldehyde (348 mg, 2.07 mmol) in tetrahydrofuran (0.5 ml), followed by acetic anhydride (0.6 ml, 6.2 mmol) after 2 hours, was added. Stirring was continued at -78° for 4 hours and at room temperature for 2 hours. The mixture was poured into water and extracted with ethyl ether. Evaporation of the dried (magnesium sulfate) extracts gave an oil, which was purified by flash chromatography on silica using 10% ethyl ether/hexane. Fractions with $R_f$ of about 0.43 on evaporation gave the title acetate as a pale yellow oil.

$^1$H NMR ($CDCl_3$): 0.26 (s, 9H), 0.92 (t, 3H, J-6.7 Hz), 1.29 (brs, 14H), 1.45 (m, 2H), 1.60 (m, 2H), 2.16 (s, 3H), 2.45 (t, 2H, J = 7.0 Hz), 6.59 (s, 1H), 7.29 (s, 1H), 7.25-7.40 (2t, 2H), 7.42 (d, 1H, J = 7.0 Hz), 7.48 (s, 1H) and 7.51 (d, 1H, J = 7.5 Hz).

MS m/e (% abundance) 466 ($M^+$, 5), 407 (100), 335 (9), 267 (7), 253 (14), 117 (26), 75 (12), 73 (57), 61 (13) and 57 (10).

4-[1-Acetoxy-1-(2-(1-tridecynyl)phenyl)methyl]-5-hydroxy-2(5H)-furanone

A mixture of 3-[1-acetoxy-1-(2-tridecynylphenyl)methyl]-5-trimethylsilylfuran (170 mg, 0.36 mmol) and Rose Bengal (5 mg) in tetrahydrofuran (7 ml) was exposed to singlet oxygen at -78° for 2.5 hours. The residue, after solvent removal, was purified by preparative TLC (20x20 cm, 1000 μ; developed with 60% ethyl ethyl/hexane). The title furanone was obtained as a deep yellow oil.

$^1$H NMR (mixture of diasteriomers) ($CDCl_3$): 0.89 (t, 3H, J = 6.4 Hz), 1.25 (brs, 16H), 1.40 (brm, 4H), 1.60 (m, 2H), 2.17 (s, 3H), 2.44 (t, 2H, J = 7.2 Hz), 5.30 (br, 1H), 5.90 (brs, 1H), 6.10 (brs, 1H), 6.20 (br, 1H), 7.0 (brs, 1H) and 7.25-7.50 (m, 4H).

$^{13}$C NMR (mixture of diasteriomers) ($CDCl_3$): 14.1, 19.5, 20.8, 22.6, 28.5, 28.8, 29.0, 29.1, 29.3, 29.5, 29.6, 30.0, 31.8, 69.7, 69.8, 76.6, 76.9, 97.6, 97.8, 98.0, 118.4, 123.5, 126.6, 127.3, 128.0, 128.1, 128.3, 128.5, 128.9, 129.0, 129.1, 132.7, 136.2, 166.3, 170.2 and 170.3.

MS: exact mass calculated for $C_{26}H_{38}O_5N$ $(M+NH_4)^+$ 444.2750, found 444.2731.

EXAMPLE 49

5-(2-Pyridyl)-4-pentyn-1-ol, prepared as in Example 40, is reduced by hydrogenating using rhodium on carbon as catalyst to give 5-(2-piperidyl)-pentan-1-ol. Treating that intermediate with methyl iodide and sodium hydride gives the N-methyl compound. Brominating, reacting the bromo compound with t-butyl lithium and 5-trimethylsilyl-3- furaldehyde, then oxidizing by the procedure of Example 40 gives 4-[1-acetoxy-6-(N-methylpiperidyl)hexyl]-5-hydroxy-2(5H)-furanone.

EXAMPLE 50

By hydrogenating 6,6-diphenyl-4-hexyn-1-ol (prepared from diphenylmethyl bromide and dilithium salt of 4-pentyn-1-ol) then brominating the reduced product, treating that with t-butyl lithium and 5-trimethylsilyl-3-furaldehyde gives a product which when treated with acetic anhydride and oxidized gives 4-(1-acetoxy-7,7-diphenylheptyl)-5-hydroxy-2(5H)-furanone.

EXAMPLE 51

Using 1,1,1-triphenyl-2-hexyn-1-ol (prepared from triphenylcarbenium tetrafluoroborate and di-lithium salt of 4-pentyn-1-ol) in the procedure of Example 50 gives 4-(1-acetoxy-7,7,7-triphenylheptyl)-5-hydroxy-2-(5H)-furanone.

EXAMPLE 52

4-(1-Acetoxy-11-phenylundecyl)-5-hydroxy-2(5H)-furanone

Using the procedures of Example 40, 10-phenyldecan-1-ol was brominated, then reacted with t-butyl lithium and 5-trimethylsilyl-3-furaldehyde, then with acetic anhydride and oxidized to give the title compound.

$_1$HNMR ($CDCl_3$): 7.2(m, 5H), 6.02(brs, 1H), 5.96(s, 1H), 5.55(brs, 1H), 5.50(t, 1H, J = 6.2Hz), 2.59(t, 2H, J = 7.5Hz), 2.12(s, 3H), 1.80(m, 2H), 1.55(m, 2H) and 1.25(m, 16H).

$^{13}$CNMR ($CDCl_3$): 171.0, 170.2, 167.1, 142.8, 128.3, 128.1, 125.5, 118.2, 98.0, 69.7, 69,4, 35.9, 32.9, 31.4, 29.4, 24.9 and 20.8.

EXAMPLE 53

4-[1-Acetoxy-6-(4-phenyl)phenylhexyl]-5-hydroxy-2(5H)-furanone

Using the procedures of Example 40, 5-(4-phenyl)phenylpentan-1-ol was brominated, reacted with magnesium and 5-trimethylsily-3-furaldehyde, then with acetic anhydride and then oxidized to the obtain the title compound.

$^1$HNMR ($CDCL_3$): 7.4(m, 9H), 6.05 (brs, 1H), 5.97 (s, 1H), 5.45 (t, 1H, J~6.3 Hz), 2.64 (t, 2H, J~7.4 Hz), 2.18 (s, 3H), 2.17 (s, 3H), 1.8 (m, 2H), 1.65 (m, 2H) and 1.4 (m, 6H).

$^{13}$CNMR ($CDCl_3$): 171.2, 169.9, 167.0, 141.4, 140.9, 138.7, 128.8, 127.0, 126.9, 119.1, 118.4, 97.9, 69.7, 69.5, 35.3, 32.9, 31.6, 31.0, 28.7, 25.1 and 20.8.

EXAMPLE 54

4-(1-Acetoxy-6-(2,4,5-trifluorophenyl)hexyl)-5-hydroxy-2(5H)-furanone

Using the procedure of Example 40, 2,4,5-trifluorophenylpentan-1-ol is brominated, reacted with magnesium and 5-trimethylsilyl-3-furaldehyde, then with acetic anhydride, and oxidized to give the title compound.

EXAMPLE 55

By procedures described herein, the following compounds of this invention are prepared:
4-[1-acetoxy-10-(2-(N-methyl)pyrrolyl)decyl]-5-hydroxy-2(5H)-furanone.
4-[1-acetoxy-6-(2-pentylphenyl)hexyl]-5-hydroxy-2(5H)-furanone.
4-(1-acetoxy-11,11-dimethyldecyl)-5-hydroxy-2(5H)-furanone.
4-(1-acetoxy-10-methoxydecyl)-5-hydroxy-2(5H)-furanone.
4-(1-acetoxy-7-pentoxyheptyl)-5-hydroxy-2(5H)-furanone.
4-(1-acetoxy-8-carboxyoctyl)-5-hydroxy-2(5H)-furanone and the ethyl ester thereof.

EXAMPLE 56

3-[1-Acetoxy-1-(2-(1-tridecynyl)phenyl)methyl]-5-trimethylsilylfuran, prepared as in Example 48, is hydrogenated using palladium on charcoal to give the 2-tridecylphenyl compound. Oxidizing by the procedure of Example 48 gives 4-[1-acetoxy-1-(2-tridecylphenyl)methyl]-5-hydroxy-2(5H)-furanone.

EXAMPLE 57

1-Bromo-8-t-butyldimethylsiloxyoctane

A solution of 8-bromooctan-1-ol (2.15 g, 10.3 mmol), t-butyldimethylsilyl chloride (1.7 g, 11.3 mmol) and imidazole (1.7 g, 25.0 mmol) in dimethylformamide (4 ml) was stirred at room temperature under argon for 48 hours. The reaction was quenched by the addition of water and extracted with hexane. The organic layer was washed with 5% hydrochloric acid, 5% sodium bicarbonate, water, saturated sodium chloride solution, dried over magnesium sulfate, filtered and concentrated to give a colorless oil. Purification by flash chromatography (silica, 0 to 2% ethyl ether/hexane) gave the desired protected alcohol.

$^1$H NMR ($CDCl_3$): 3.58 (t, 2H, J=6.5 Hz), 3.38 (t, 2H, J=6.9 Hz), 1.25 to 1.55 (m, 12H), 0.88 (s, 9H), 0.03 (s, 6H).

8-(Thiophen-2-yl)octan-1-ol

To a stirred solution of 2-bromothiophene (0.341 g, 2.09 mmol) in tetrahydrofuran (30 ml) under argon at -78° was added t-butyl lithium (2.53 ml of a 1.7 M solution) followed by 1,3-dimethyl-3,4,5,6-tetrahydro-2-(1H)-pyrimidinone (0.268 g, 2.09 mmol). To this mixture was added 1-bromo-8-t-butyldimethylsiloxyoctane (0.615 g, 1.90 mmol) in tetrahydrofuran (10 ml). After gradual warming followed by stirring at room temperature for 18 hours, the mixture was concentrated, taken up in ethyl ether and washed consecutively with cold 10% hydrochloric acid, saturated sodium bicarbonate, water, saturated sodium chloride solution, dried over magnesium sulfate, filtered and concentrated to give a yellow oil. To this residue in tetrahydrofuran (20 ml) at 0° was added tetrabutylammonium fluoride (8.4 ml of a 1.0 M solution). After stirring for 16 hours at room temperature, the mixture was concentrated, taken up in ethyl ether and washed with 10% hydrochloric acid, saturated sodium bicarbonate, water, saturated sodium chloride solution, dried over magnesium sulfate, filtered and concentrated to give a yellow oil. Further purification by flash chromatography (silica, 30% ethyl acetate/hexane) gave the desired alcohol.

IR (neat): 3340 (broad), 2930, 1460, 1440, 690 $cm^{-1}$.

$^1$H NMR ($CDCl_3$): 7.10 (d, 1H, J=5.1 Hz), 6.91 (dd, 1H, J=5.0, 3.5 Hz), 6.77 (d, 1H, J=3.4 Hz), 3.62 (t, 2H, J=6.6 Hz), 2.81 (t, 2H, J=7.6 Hz), 1.60 to 1.72 (m, 2H), 1.45 to 1.60 (m, 3H) and 1.25 to 1.45 (m, 8H).

$^{13}$C NMR ($CDCl_3$): 145.7, 126.6, 123.9, 122.7, 62.9, 32.7, 31.7, 29.8, 29.3, 29.0, 25.6.

m/z calculated for $C_{12}H_{20}OS$ ($M^+$): 212.1235; obtained ($EI^+$): 212.1238.

8-(Thiophen-2-yl)octan-1-ol

To a stirred solution of 2-bromothiophene (0.341g, 2.09 mmol) in tetrahydrofuran (30ml) under argon at

-78° was added t-butyl lithium (2.53 ml of a 1.7M solution) followed by 1,3-dimethyl-3,4,5,6-tetrahydro-2-(1H)-pyrimidinone (0.268g, 2.09 mmol). To this mixture was added 1-bromo-8-t-butyldimethylsiloxyoctane (0.615g, 1.90 mmol in tetrahydrofuran (10ml). After gradual warming followed by stirring at room temperature for 18 hours, the mixture was concentrated, taken up in ethyl ether and washed consecutively with cold 10% hydrochloric acid, saturated sodium bicarbonate, water, saturated sodium chloride solution, dried over magnesium sulfate, filtered and concentrated to give a yellow oil. To this residue in tetrahydrofuran (20ml) at 0° was added tetrabutylammonium fluoride (8.4 ml of a 1.0M solution). After stirring for 16 hours at room temperature the mixture was concentrated, taken up in ethyl ether and washed with 10% hydrochloric acid, saturated sodium bicarbonate, water, saturated sodium chloride solution, dried over magnesium sulfate, filtered and concentrated to give a yellow oil. Further purification by flash chromatography (silica, 30% ethyl acetate/hexane) gve the desired alcohol.

IR (neat): 3340 (broad), 2930, 1460, 1440, and 690 $cm^{-1}$.

$^1H$ NMR ($CDCl_3$): 7.10 (d, 1H, J~5.1Hz); 6.91 (dd, 1H, J~5.0, 3.5Hz), 6.77 (d, 1H, J~3.4Hz) 3.62 (t, 2H, J~6.6 Hz); 2.81 (t, 2H, J~7.6 Hz); 1.60 to 1.72 (m, 2H); 1.45 to 1.60 (m, 3H) and 1.25 to 1.45 (m, 8H).

$^{13}C$ NMR ($CDCl_3$): 145.7, 126.6, 123.9, 122.7, 62.9, 32.7, 31.7, 29.8, 29.3, 29.0, 25.6.

m/z Calculated for $C_{12}H_{20}OS$ ($M^+$): 212.1235, obtained ($EI^+$): 212.1238.

1-Bromo-8-(thiophen-2-yl)octane

To a stirring mixture of 8-(thiophen-2-yl)octan-1-ol (0.132 g, 0.622 mmol) and triethylamine (0.126 g, 1.245 mmol) in methylene chloride (5 ml) at 0° under argon was added methanesulfonyl chloride (0.107 g, 0.933 mmol). After stirring for 2 hours at 0° the reaction was quenched with water and the organic layer was washed with 10% hydrochloric acid, saturated sodium bicarbonate, water, saturated sodium chloride solution, dried over magnesium sulfate, filtered and concentrated to give a pale oil. To this oil in acetone (2 ml) was added lithium bromide (0.216 g, 2.49 mmol) and the mixture was refluxed for 15 hours. The acetone was evaporated and the residue was taken up in ethyl ether, washed with 10% hydrochloric acid, saturated sodium bicarbonate, water, saturated sodium chloride, dried over magnesium sulfate, filtered through silica and concentrated to give the title compound which was carried on without further purification.

IR ($CHCl_3$): 2920, 1455, 1435, 685 $cm^{-1}$.

$^1H$ NMR ($CDCL_3$): 7.10 (dd, 1H, J=5.2, 1.3 Hz), 6.91 (dd, 1H, J=5.1, 3.3 Hz), 6.77 (d, 1H, J=3.6 Hz), 3.4 (t, 2H, J=6.9 Hz), 2.81 (t, 2H, J=7.6 Hz), 1.80 to 1.95 (m, 2H), 1.60 to 1.75 (m, 2H) and 1.25 to 1.50 (m, 8H).

$^{13}C$ NMR ($CDCl_3$): 145.7, 126.6, 123.9, 122.7, 34.0, 32.8, 31.7, 29.9, 29.1, 29.0, 28.6, 28.1.

m/z Calculated for $C_{12}H_{19}BrS$ ($M^+$): 274.0390; obtained ($EI^+$): 274.0398.

4-(1-Hydroxy-9-thiophen-2-ylnonyl)-2-trimethylsilylfuran

To a stirred solution of 8-(thiophene-2-yl)octyl magnesium bromide [0.62 mmol, prepared from 1-bromo-8-(thiophen-2-yl)octane; 0.170 g, 0.620 mmol and 1.24 mmol magnesium with a catalytic amount of 1,2 dibromoethane as initiator] in tetrahydrofuran (2 ml) at 0° under argon was added dropwise 5-trimethylsilyl-3-furaldehyde (0.104 g, 0.620 mmol) in tetrahydrofuran (3 ml). The solution was warmed to room temperature, stirred for one hour, quenched with saturated ammonium chloride solution and extracted into ethyl ether. The organic portion was washed with saturated sodium bicarbonate solution, water, saturated sodium chloride solution, dried over magnesium sulfate, filtered and concentrated to a yellow oil. Purification by flash chromatography (silica, 5 to 10% ethyl acetate/hexane) gave the desired alcohol.

IR ($CHCl_3$): 3600, 3000, 2920, 1220 $cm^{-1}$.

$^1H$ NMR ($CDCl_3$): 7.56 (s, 1H), 7.10 (d, 1H, J=2.7 Hz), 6.91 (dd, 1H, J=3.5, 2.4 Hz), 6.77 (d, 1H, J=3.3 Hz), 6.62 (s, 1H), 4.64 (t, 1H, J=6.6 Hz), 2.81 (t, 2H, J=7.6 Hz), 1.58 to 1.83 (m, 5H); 1.20 to 1.50 (m, 10H), and 0.25 (s, 9H).

$^{13}C$ NMR ($CDCl_3$): 161.4, 145.8, 143.1, 129.2, 126.6, 123.9, 122.7, 118.3, 67.0, 37.9, 37.9, 31.8, 29.9, 29.4, 29.3, 29.1, 25.7, -1.7.

c/z Calculated for $C_{20}H_{32}O_2SSi$($M^+$):364.1892; obtained ($CI^+$): 364.1885.

4(1-Acetoxy-9-thiophen-2-ylnonyl)-2-trimethylsilylfuran

A solution of 4-(1-hydroxy-9-thiophen-2-ylnonyl)-2-trimethylsilylfuran (0.034 g, 0.093 mmol), acetic anhydride (1 ml) and pyridine (0.25 ml) was stirred at room temperature until no starting material was visible by TLC. The reaction mixture was concentrated and the residue taken up in ethyl ether, washed with 10%

hydrochloric acid, saturated sodium chloride, aqueous 5% cupric sulfate solution, water, saturated sodium chloride solution, dried over magnesium sulfate, filtered and concentrated to give a pale oil. Purification by flash chromatography (silica, 100% hexane to 5% ethyl ether/hexane) gave the title compound.

IR ($CHCl_3$): 2920, 2840, 1720, 1370, 1250 $cm^{-1}$.

$^1H$ NMR ($CDCl_3$): 7.59 (s, 1H), 7.10 (dd, 1H, J = 5.1, 1.2 Hz), 6.91 (dd, 1H, J = 5.1, 3.6 Hz), 6.77 (d, 1H, J = 2.4 Hz), 6.58 (s, 1H), 5.76 (t, 1H, J = 7.0 Hz), 2.81 (t, 2H, J = 7.6 Hz), 2.05 (s, 3H), 1.60 to 1.92 (m, 4H), 1.20 to 1.50 (m, 10H) and 0.25 (s, 9H).

$^{13}C$ NMR ($CDCl_3$): 170.5, 161.2, 145.7, 144.4, 126.6, 124.9, 123.4, 122.7, 118.6, 68.5, 34.7, 31.7, 29.9, 29.3, 29.2, 29.0, 25.5, 21.3, -1.7.

m/z Calculated for $C_{22}H_{34}O_3$ SSi ($M^+$): 406.1998; obtained 406.1979.

4-(1-Acetoxy-9-thiophen-2-ylnonyl)-5-hydroxy-2(5H)-furanone

A stirred solution of 4-(1-acetoxy-9-thiophen-2-ylnonyl)-2-trimethylsilylfuran (0.024 g, 0.059 mmol) and Rose Bengal (2 mg) in acetone (4 ml) was flushed with oxygen and cooled to -78°. The solution was subsequently irradiated with a 150 watt flood lamp while under constant positive pressure of oxygen until starting material was no longer visible by TLC. The solution was warmed to room temperature and concentrated to a pink oil. Purification by flash chromatography (silica, 40% ethyl acetate/hexane) gave the desired hydroxybutenolide.

IR ($CHCl_3$): 3400 (broad), 2930, 1750 (broad) $cm^{-1}$.

$^1H$ NMR (mixture of diastereomers) ($CDCl_3$): 7.10 (dd, 1H, J = 5.1, 1.2 Hz), 6.91 (dd, 1H, J = 5.1, 3.3 Hz), 6.77 (d, 1H, J = 1.2 Hz), 5.95 to 6.10 (m, 2H), 5.35 to 5.45 (brt, 1H), 5.1 to 5.3 (brd, 1H), 2.82 (t, 2H, J = 7.6 Hz), 2.13 (s, 3H), 1.75 to 1.95 (m, 2H), 1.63 to 1.75 (m, 2H) and 1.20 to 1.55 (m, 10H).

$^{13}C$ NMR (mixture of diastereomers) ($CDCl_3$): 171.1, 170.0, 167.2, 145.7, 126.6, 123.9, 122.7, 118.6, 118.4, 98.0, 69.7, 69.6, 33.0, 31.7, 29.8, 29.14, 29.06, 29.0, 24.9, and 20.8.

m/z Calculated for $C_{19}H_{27}O_5S$ ($MH^+$): 367.1579; obtained ($CI^+$):367.1594.

EXAMPLE 58

4-(1-Hydroxy-9-t-butyldimethylsiloxynonyl)-2-trimethylsilylfuran

To a stirred solution of 8-t-butyldimethylsiloxyoctylmagnesium bromide (5.63 mmol prepared from 1-bromo-8-t-butyldimethylsiloxyoctane 1.82 g, 5.63 mmol and 6.19 mmol magnesium; with a catalytic amount of 1,2-dibromoethane as initiator) in tetrahydrofuran (2 ml) at 0° under argon was added dropwise 5-trimethylsilyl-3-furaldehyde (0.866 g, 5.15 mmol) in tetrahydrofuran (10 ml). The solution was warmed to room temperature, stirred for one hour, quenched with cold 10% hydrochloric acid and extracted into ethyl ether. The organic portion was washed with saturated sodium bicarbonate, water saturated sodium chloride solution, dried over magnesium sulfate, filtered and concentrated to give a pale oil. Purification by flash chromatography (silica, 5 to 10% ethyl acetate/hexane) gave the desired alcohol.

IR (neat): 3350, 1250 $cm^{-1}$.

$^1H$ NMR ($CDCl_3$): 7.59 (s, 1H), 6.65 (s, 1H), 4.67 (t, 1H, J = 6.3 Hz), 3.62 (t, 2H, J = 6.6 Hz), 1.20 to 1.90 (m, 15H), 0.92 (s, 9H), 0.28 (s, 9H), and 0.07 (s, 6H).

$^{13}C$ NMR ($CDCl_3$): 161.3, 143.1, 129.1, 118.2, 66.9, 63.3, 37.9, 32.8, 29.5, 29.4, 29.3, 26.0, 25.7, 18.4, -1.7.

m/z Calculated for $C_{22}H_{44}O_3Si_2$ ($M^+$): 412.2629; obtained ($CI^+$): 412.2834.

4-(1-Acetoxy-9-t-butyldimethylsiloxynonyl)-2-trimethylsilylfuran

A solution of 4-(1-hydroxy-9-t-butyldimethylsiloxynonyl)-2-trimethylsilylfuran (0.892 g, 2.17 mmol), acetic anhydride (5 ml) and pyridine (0.729 g, 8.66 mmol) was stirred at room temperature until no starting material was visible by TLC. The reaction mixture was concentrated and the residue was taken up in ethyl ether, washed with 10% hydrochloric acid, saturated sodium bicarbonate, aqueous 5% cupric sulfate solution, water, saturated sodium chloride, dried over magnesium sulfate, filtered and concentrated to give the title compound which was carried on without further purification.

IR (neat): 2940, 1740, 1250 $cm^{-1}$.

$^1H$ NMR ($CDCl_3$): 7.64 (s, 1H), 6.63 (s, 1H), t, 1H, J = 6.9 Hz), 3.64 (t, 2H, J = 6.6 Hz), 2.09 (s, 3H), 1.73 to 2.00 (m, 2H), 1.50 to 1.60 (m, 2H), 1.25 to 1.45 (m, 10H), 0.94 (s, 9H), 0.29 (s, 9H), 0.09 (s, 6H).

$^{13}C$ NMR ($CDCl_3$): 170.6, 161.2, 144.4, 124.9, 118.6, 68.6, 63.3, 34.8, 32.8, 29.4, 29.3, 29.2, 26.0, 25.8,

25.5, 21.4, -1.7.

m/z Calculated for $C_{23}H_{43}O_4Si_2$ ($M^+$-$CH_3$): 439.2700; obtained ($CI^+$): 439.2704.

4-(1-Acetoxy-9-hydroxynonyl)-2-trimethylsilylfuran

4-(1-Acetoxy-9-t-butyldimethylsiloxynonyl)-2-trimethylsilylfuran (1.00 g, 2.21 mmol) was stirred at 35° for 36 hours in a mixture of acetic acid-water-tetrahydrofuran (1:1:1). The reaction mixture was concentrated and the residue was taken up in ethyl ether, washed with 10% hydrochloric acid, saturated sodium bicarbonate, water, saturated sodium chloride solution, dried over magnesium sulfate, filtered and concentrated to a pale oil. Purification by flash chromatography (silica, 10% ethyl acetate/hexane) gave the desired alcohol.

IR ($CHCl_3$): 3615, 3430 (br), 2920, 1720, 1230 $cm^{-1}$.

$^1H$ NMR ($CDCl_3$): 7.64 (s, 1H), 6.63 (s, 1H), 5.81 (t, 1H, J=6.9 Hz), 3.65 (t, 2H, J=6.6 Hz), 2.26 (s, 1H), 2.08 (s, 3H), 1.70 to 1.95 (m, 2H), 1.53 to 1.67 (m, 2H), 1.23 to 1.46 (m, 10H), and 0.29 (s, 9H).

$^{13}C$ NMR ($CDCl_3$): 170.6, 161.1, 144.3, 124.8, 118.5, 68.5, 62.7, 34.6, 32.6, 29.3, 29.2, 29.0, 25.6, 25.4, 21.2, -1.8.

m/z Calculated for $C_{18}H_{32}O_4Si$ ($M^+$): 340.2070; obtained ($CI^+$): 340.2072.

4-(1,9-Diacetoxynonyl)-2-trimethylsilylfuran

To 4-(1-acetoxy-9-hydroxynonyl)-2-trimethylsilylfuran (0.075 g, 0.221 mmol) and pyridine (0.056 g, 0.662 mmol) in tetrahydrofuran (2 ml) under argon at 0° was added dropwise acetyl chloride (0.052 g, 0.662 mmol). The mixture was warmed to room temperature, stirred for one hour, quenched with water and extracted into ethyl ether. The organic portion was washed with 10% hydrochloric acid, saturated sodium bicarbonate, aqueous 5% cupric sulfate solution, water, saturated sodium chloride solution, dried over magnesium sulfate, filtered and concentrated to an oil. Purification by flash chromatography (silica, 5 to 10% ethyl acetate/hexane) gave the desired product.

IR ($CHCl_3$): 3010, 2940, 1725, 1370, 1750 $cm^{-1}$.

$^1H$ NMR ($CDCl_3$): 7.57 (s, 1H), 6.56 (s, 1H), 5.74 (t, 1H, J=6.9 Hz), 4.02 (t, 2H, J=6.7 Hz), 2.01 (s, 6H), 1.65 to 1.91 (m, 2H), 1.54 to 1.65 (m, 2H), 1.18 to 1.40 (m, 10H), and 0.22 (s, 9H).

$^{13}C$ NMR ($CDCl_3$): 171.1, 170.4, 161.1, 144.4, 124.9, 118.5, 68.4, 64.5, 34.7, 29.2, 29.1, 28.5, 25.8, 25.4, 21.2, 20.9, and -1.8.

m/z Calculated for $C_{20}H_{34}O_5Si$ ($M^+$): 382.2175; obtained ($CI^+$): 382.2196.

4-(1,9-Diacetoxynonyl)-5-hydroxy-2(5H)-furanone

A stirred solution of 4-(1,9-diacetoxynonyl)-2-trimethylsilylfuran (0.075 g, 0.196 mmol) and Rose Bengal (2 mg) in acetone (70 ml) was flushed with oxygen and cooled to -78°C. The solution was subsequently irradiated with a 150 Watt flood lamp while under constant positive pressure of oxygen until starting material was no longer visible by TLC. The solution was warmed to room temperature and concentrated to a pink oil. Purification by flash chromatography (silica, 5 to 50% ethyl acetate/hexane) gave the desired hydroxybutenolide.

IR ($CHCl_3$): 3500 (br), 3020, 2930, 2860, 1755, 1730, 1370 $cm^{-1}$.

$^1H$ NMR (mixture of diastereomers) ($CDCl_3$): 6.00 to 6.25 (brs, 1H), 5.98 (s, 1H), 5.75 to 5.90 (brs, 1H), 5.53 (brt, 1H, J=5.7 Hz), 4.05 (t, 2H, J=6.7 Hz), 2.14 (s, 3H), 2.06 (s, 3H), 1.70 to 1.94 (m, 2H), 1.55 to 1.70 (m, 2H), 1.20 to 1.50 (m, 10H).

$^{13}C$ NMR (mixture of diastereomers) ($CDCl_3$): 171.7, 170.8, 170.2, 166.9, 118.5, 118.4, 98.0, 69.5, 64.6, 32.8, 29.1, 28.9, 28.4, 25.7, 24.8, 21.0, and 20.8.

m/z Calculated for $C_{17}H_{27}O_7$ ($MH^+$):343.1757; obtained ($CI^+$): 343.1775.

EXAMPLE 59

4-(1-Acetoxy-9-carboxynonyl)-2-trimethylsilylfuran

To a solution of 4-(1-acetoxy-9-hydroxynonyl)-2-trimethylsilylfuran (0.176 g, 0.518 mmol) in acetone (5 ml) at room temperature was added Jones reagent until the mixture remained orange. The mixture was then taken up in ethyl ether, filtered through celite, washed with saturated sodium bicarbonate, water, saturated sodium chloride solution, dried over magnesium sulfate, refiltered, and concentrated to give an oil which

was carried on without further purification.

IR ($CHCl_3$): 3100 (very broad), 2930, 1710, 1250 $cm^{-1}$.

$^1H$ NMR ($CDCl_3$): 8.0 to 8.5 (brs, 1H), 7.62 (s, 1H), 6.62 (s, 1H), 5.80 (t, 1H, J=6.9 Hz), 2.37 (2H, t, J=7.3 Hz), 2.08 (s, 3H), 1.80 to 1.97 (m, 2H), 1.58 to 1.74 (m, 2H), 1.25 to 1.50 (m, 8H), and 0.28 (s, 9H).

$^{13}C$ NMR ($CDCl_3$): 179.4, 170.7, 161.1, 144.4, 124.8, 118.5, 68.5, 34.6, 33.9, 29.0, 28.9, 25.4, 24.6, 21.2, -1.8.

m/z Calculated for $C_{18}H_{30}O_5Si$ ($M^+$): 354.1863; obtained ($CI^+$): 354.1868.

4-(1-Acetoxy-9-carboxynonyl)-5-hydroxy-2(5H)-furanone

A stirred solution of 4-(1-acetoxy-9-carboxynonyl)-2-trimethylsilylfuran (0.0262 g, 0.074 mmol) and Rose Bengal (1 mg) in acetone (7 ml) was flushed with oxygen and cooled to -78°. The solution was subsequently irradiated with a 150 Watt flood lamp while under constant positive pressure of oxygen until starting material was no longer visible by TLC. The solution was warmed to room temperature and concentrated to a pink oil. Purification by preparative plate chromatography (silica, 100% ethyl acetate) gave the desired hydroxybutenolide.

IR ($CHCl_3$): 3250 (very broad), 2920, 1740, 1705 $cm^{-1}$.

$^1H$ NMR (mixture of diastereomers) ($CDCl_3$): 6.09 (brs, 1H); 5.99 (s, 1H), 5.48 (t, 1H, J=6.1 Hz), 5.3 (very broad s, 2H), 2.36 (t, 2H, 7.4 Hz), 2.14 (s, 3H), 1.73 to 1.90 (m, 2H), 1.55 to 1.70 (m, 2H) and 1.20 to 1.50 (m, 8H).

$^{13}C$ NMR (mixture of diastereomers)($CDCl_3$) : 179.1, 171.1, 170.0, 166.9, 118.7, 98.0, 69.6, 33.8, 32.8, 28.6, 24.6, 24.4, and 20.9.

m/z Calculated for $C_{15}H_{23}O_7(M+H)^+$: 315.1444, found 315.1445

EXAMPLE 60

4-(1-Acetoxy-9-hydroxynonyl)-5-hydroxy-2(5H)-furanone

A stirred solution of 4-(1-acetoxy-9-hydroxynonyl)-2-trimethylsilylfuran (0.080 g, 0.235 mmol) and Rose Bengal (2 mg) in acetone (15 ml) was flushed with oxygen and cooled to -78°. The solution was then irradiated with a 150 Watt flood lamp while under constant positive pressure of oxygen until starting material was no longer visible by TLC. The solution was warmed to room temperature and concentrated to a pink oil. Purification by flash chromatography (silica, 50% ethyl acetate/hexane) gave the desired hydroxybutenolide.

IR ($CHCl_3$): 3350 (br), 2930, 1750 $cm^{-1}$.

$^1H$ NMR (mixture of diasteromers) ($CDCl_3$): 6.5 (brs, 1H), 6.07 (brs, 1H), 5.95 (m, 1H), 5.55 (brs, 1H), 3.62 (t, 2H, J=6.6 Hz), 2.60 (brs, 1H), 2.14 (s, 3H), 1.70 to 1.90 (m, 2H), 1.48 to 1.60 (m, 2H), 1.20 to 1.44 (m, 10H).

$^{13}C$ NMR (mixture of diastereomers) ($CDCl_3$): 170.8, 170.7, 167.0, 118.2 and 118.0, 98.1, 69.7 and 69.6, 62.8, 32.6, 32.2, 29.0, 28.7, 25.4, 24.7, 20.8.

MS exact mass calculated for $C_{15}H_{23}O_7$ $(M+H)^+$ 315.1444, found 315.1445.

EXAMPLE 61

1-Bromo-11-t-butyldimethylsiloxyundecane

To a stirred solution of 8-bromo-1-undecanol (2.02 g, 8.0 mmol) and imidazole (1.3 g, 19 mmol) at 0° under nitrogen in dimethylformamide (4 ml) was added t-butyldimethylsilylchloride (1.46 g, 9.6 mmol). After 1.5 hours the solution was warmed to room temperature, stirred an additional 1.5 hours, and poured into ethyl ether and water. The organic portion was washed successively twice with water, twice with 10% hydrochloric acid solution, once with water, once with saturated sodium bicarbonate solution, once with water, once with sodium chloride, dried over magnesium sulfate, filtered and concentrated to give the silyl ether as a yellow oil. This material was used without further purification.

IR (film): 1460 $cm^{-1}$.

$^1H$ NMR ($CDCl_3$): 3.61 (t, 2H, J=6.6 Hz), 3.42 (t, 2H, J=6.8 Hz), 1.87 (p, 2H, J=7.3 Hz), 1.6-1.2 (m, 16H), 0.06 (s, 9H), and 0.02 (s, 6H).

$^{13}C$ NMR ($CDCl_3$): 63.4, 34.1, 32.9, 29.6, 29.5, 28.8, 28.2, 26.0, 25.9, 25.7, 18.4, 0.06.

Calculated for $C_{17}H_{38}BrOSi$: 365.1875 $(M+H)^+$; obtained ($EI^+$): 365.1862.

**4-(12-t-butyldimethylsiloxy-1-hydroxydodecyl)-2-trimethylsilylfuran**

To a stirred suspension of magnesium turnings (0.142 g, 5.9 mmol) in tetrahydrofuran (1ml) at room temperature under nitrogen was added a solution of 1-bromo-11-t-butyldimethylsiloxyundecane (1.06 g, 2.9 mmol) in tetrahydrofuran (1.5ml). This mixture was heated to reflux and stirred for 1 hour, then cooled to 0° and diluted with tetrahydrofuran (3ml). Trimethylsilyl-3-furanaldehyde (0.491 g, 2.9 mmol) was added dropwise in 2ml of tetrahydrofuran. The solution was stirred for 1 hour at 0°, 4.5 hours at room temperature, quenched with saturated ammonium chloride solution and poured into ethyl ether. The organic portion was washed twice with water, once with saturated sodium chloride solution, dried over magnesium sulfate, filtered and concentrated to give a yellow liquid. The alcohol ($R_f$ = 0.33, 20% ethyl ether/hexane) was purified by flash chromatography (silica, 10-15% ethyl ether/petroleum ether) to give a pale oil.

IR (film): 3350 (br) $cm^{-1}$.

$^1H$ NMR ($CHCl_3$): 7.57 (s, 1H), 6.64 (s, 1H), 4.65 (brt, 1H), 3.61 (t, 2H, J = 6.6 Hz), 1.8-1.2 (m, 20H), 0.91 (s, 9H), 0.27 (s,6H), and 0.06 (s, 3H).

$^{13}C$ NMR ($CDCl_3$): 161.4, 143.1, 129.2, 118.3, 67.0, 63.4, 37.9, 32.9, 29.6, 29.5, 26.0, 25.8, 18.4, 15.3, 0.02 and -1.6.

m/z Calculated for $C_{25}H_{50}O_3Si_2$ ($M^+$): 454.3299; obtained ($EI^+$): 454.3288.

**4-(1-Acetoxy-12-t-butyldimethylsilyloxydodecyl)-2-trimethylsilylfuran**

A solution of 4-(12-t-butyldimethylsiloxy-1-hydroxydodecyl)-2-trimethylsilylfuran(0.46 g, 1.0 mmol) and pyridine (0.3 ml) in acetic anhydride (4ml) was stirred at 0° under nitrogen for 1/2 hour, then 3.5 hours at room temperature. Approximately 1 ml saturated sodium bicarbonate solution was added to the reaction mixture, which was subsequently poured into ethyl ether and water. The organic portion was washed successively three times with water, once with saturated sodium bicarbonate solution, once with water, once with 10% copper sulfate solution, twice with water, once with saturated sodium chloride solution, dried over magnesium sulfate, filtered and concentrated to give a pale oil. The acetate (20% ethyl ether/hexane) was purified by flash chromatography (silica, 7% ethyl ether/petroleum ether) to give a colorless oil.

IR (film): 1740 $cm^{-1}$.

$^1H$ NMR ($CDCl_3$): 7.61 (s, 1H), 6.60 (s, 1H), 5.78 (t, 1H), 3.62 (t, 2H, J = 6.6 Hz), 2.07 (s, 3H), 1.9-1.5 (m, 4H), 1.27 (m, 16H), 0.92 (s, 9H), 0.27 (s, 6H), 0.07 (s, 3H).

$^{13}C$ NMR ($CDCl_3$): 170.6, 161.2, 144.5, 125.0, 118.6, 68.6, 63.4, 34.8, 32.9, 29.6, 29.5, 29.3, 26.0, 25.8, 25.6, 21.4, 0.02, and -1.6.

m/z Calculated for $C_{26}H_{49}O_4Si_2$ ($M^+$-$CH_3$): 481.3169; obtained ($CH_4Cl^+$): 481.3170.

4-(1-Acetoxy-12-t-butyldimethylsilyloxydodecyl)-5-hydroxy-2(5H)-furanone

A stirred solution of 4-(1-acetoxy-12-t-butyldimethylsilyloxydodecyl)-2-trimethylsilylfuran (0.102 g, 0.2 mmol) and Rose Bengal (trace) in tetrahydrofuran (25ml) was flushed with oxygen and cooled to -78°. The solution was irradiated with a 200 Watt flood lamp while under a constant positive pressure of oxygen until starting material was no longer visible by TLC (about 1 hour). The solution was warmed to room temperature, concentrated to a pale oil, and purified by preparative thin layer chromatography (30% ethyl acetate/hexane). The desired hydroxybutenolide was obtained as a clear oil.

IR ($CHCl_3$): 3350 (br), 1760, 1745 $cm^{-1}$.

$^1H$ NMR (mixture of diasteriomers) ($CDCl_3$): 6.21 (brs, 0.25H), 6.0 (m, 1.75H), 5.50 (brt, 1H), 5.35 (brs, 1H), 3.62 (t, 2H, J = 6.7 Hz), 2.15 and 2.13 (2s, 3H), 1.9-1.2 (m), 0.90 (s, 9H), and 0.02 (s3H).

$^{13}C$ NMR ($CDCl_3$) (mixture of diasteriomers): 171.2, 170.1, 167.2, 166.7, 119.1, 118.4, 98.0, 69.9 and 69.3, 63.5, 33.1, 32.9, 29.9, 29.6, 29.5, 29.3, 29.2, 26.0, 25.8, 25.1, 25.0, 20.9, 18.4, and 0.05.

m/z Calculated for $C_{24}H_{45}O_6Si$ $(M+H)^+$: 457.2985; obtained ($CI^+$): 457.2966.

EXAMPLE 62

5-(1-Naphthyl)-pent-4-yn-1-ol

A mixture of 1-iodonaphthalene (6 g, 23.6 mmol), 4-pentyn-1-ol (2.38 g, 28.3 mmol), palladium (II) acetate (5 mg), triphenylphosphine (5 mg), copper (I) iodide (5 mg) in triethylamine (50 ml) was refluxed under argon for 17 hours. After cooling, the mixture was acidified with ice-cold dilute hydrochloric acid and extracted with ethyl ether. Evaporation of the dried (magnesium sulfate) extracts gave an oil, which was

purified by flash chromatography on silica using 30% ethyl acetate/hexane. Fractions with $R_f$ of about 0.20 on evaporation gave the title alkynol as a deep yellow oil.

$^1$H NMR ($CDCl_3$): 1.74 (br, 1H), 1.99 (p, 2H, J = 6.6 Hz), 2.74 (t, 2H, J = 7.0 Hz), 3.93 (t, 2H, J = 6.0 Hz), 7.40-7.90 (m, 7H) and 8.35 (d, 1H, J = 8.0 Hz).

$^{13}$C NMR ($CDCl_3$): 16.1, 31.4, 61.3, 78.9, 94.4, 121.3, 125.0, 126.0, 126.1, 126.4, 127.9, 128.1, 129.9, 133.0 and 133.2.

MS m/e (% abundance): 210 ($M^+$, 100), 191 (19), 165 (71) and 141 (15).

1-Bromo-5-(1-naphthyl)-pent-4-yne

Bromine (a 2M solution in dichloromethane: 950 mg, 5.95 mmol) was added to a 0° solution of 1,2-bis-(diphenyl)phosphinoethane (1.2 g, 2.97 mmol) in dichloromethane (15 ml). A solution of 5-(1-naphthyl)-4-pentyne-1-ol (500 mg, 2.38 mmol) in dichloromethane (2 ml) was added to the solution. The solution was warmed to room temperature and stirring continued for 1.5 hours. The solution was diluted with ethyl ether, then pentane, filtered through a thin pad of silica and the solvent removed. The resulting oil was purified by flash chromatography (hexane) to give the title bromide.

$^1$H NMR ($CDCl_3$): 8.3 (d, 1H, J = 7.8 Hz), 7.7 (m, 2H), 7.5 (m, 4H), 3.67 (t, 2H, J = 4.1 Hz), 2.78 (t, 2H, 6.6 Hz), and 2.26 (m, 2H).

MS m/e (% abundance): 274/272 ($M^+$, 36/36), 193 (22), 179 (11), 178 (28), 166 (16), 165 (100), 164 (20) and 163 (26).

3-[1-Acetoxy-6-(1-naphthynl)-5-hexyl]-5-trimethylsilylfuran

Tert-Butyl lithium (a 1.7 M solution in hexane, 13 ml, 2.2 mmol) was added to a solution of 5-(1-naphthyl)-1-bromopent-4-yne (300 mg, 1.1 mmol) in tetrahydrofuran (2 ml) at -78° under nitrogen. After 30 minutes, a solution of 5-trimethylsilyl-3-furaldehyde (168 mg, 0.99 mmole) in tetrahydrofuran (0.5 ml) was added. Stirring was continued for 2 hours, then acetic anhydride (224 mg, 2.2 mmol) was added and the solution stirred at room temperature overnight. The mixture was quenched with water. Extraction (ethyl ether) and evaporation of the dried (magnesium sulfate) extracts gave an oil, which was flash chromatographed on silica gel using 15% ethyl ether/hexane. The title compound was obtained as a yellow oil.

$^1$H NMR ($CDCl_3$): 8.31 (d, 1H, J = 7 Hz), 7.82 (m, 2H), 7.63 (m, 2H), 7.5 (m, 2H), 7.39 (t, 1H, J = 7.8 Hz), 6.62 (s, 1H), 5.88 (t, 1H, J = 6.95 Hz), 2.61 (t, 2H, J = 6.9 Hz), 2.1 (m, 2H), 1.75 (m, 2H), and 0.25 (s, 9H).

$^{13}$C NMR ($CDCl_3$): 170.5, 145.7, 144.5, 133.4, 133.1, 130.0, 128.2, 128.0, 126.5, 126.2, 125.3, 125.2, 124.6, 120.3, 120.1, 94.4, 79.1, 76.6, 68.1, 57.7, 34.0, 24.9, 21.3, 21.0, 19.4, -1.7.

4-[1-Acetoxy-6-(1-naphthyl)-5-hexynyl]-5-hydroxy-2(5H)-furanone

A mixture of 3-[1-acetoxy-6-(1-napthyl)-5-hexynyl]-5-trimethylsilylfuran (63.3 mg, 0.16 mmol) and Rose Bengal (5 mg) in acetone (10 ml) was exposed to singlet oxygen at -78° for two hours. The residue, after solvent removal, was purified by preparative TLC (80% ethyl ether/hexane). The title compound was obtained as a yellow oil.

$^1$H NMR ($CDCl_3$): 8.3 (d, 1H, J = 8 Hz), 7.8 (m, 2H), 7.2 (m, 4H), 6.2 (brs, 1H), 6.0 (brs, 1H), 5.6 (t, 1H, J = 6.8 Hz), 2.6 (t, 2H, J~6.8 Hz), 2.1 (m, 5H), and 1.8 (m, 2H).

$^{13}$C NMR ($CDCl_3$): 170.9, 169.8, 166.6, 133.3, 130.1, 128.3, 126.6, 126.3, 126.0, 125.2, 121.1, 119.3, 118.7, 97.8, 93.5, 97.7, 69.3, 68.8, 65.9, 32.1, 24.2, 20.9, 20.8, 19.2, 15.2.

MS exact mass calculated for $C_{22}H_{20}O_5$ 364.13107 ($M^+$), found 364.1295.

EXAMPLE 63

5-(1-Naphthyl)-pent-4-yn-1-ol

A mixture of 1-iodonaphthalene (6 g, 23.6 mmol), 4-pentyn-1-ol (2.38 g, 28.3 mmol), palladium (II) acetate (5 mg), triphenylphosphine (5 mg), copper (I) iodide (5 mg) in triethylamine (50 ml) was refluxed under argon for 17 hours. After cooling, the mixture was acidified with ice-cold dilute hydrochloric acid and extracted with ethyl ether. Evaporation of the dried (magnesium sulfate) extracts gave an oil, which was purified by flash chromatography on silica using 30% ethyl acetate/hexane). Fractions with $R_f$ of about 0.20 on evaporation gave the title alkynol as a deep yellow oil.

$^1$H NMR ($CDCl_3$): 1.74 (br, 1H), 1.99 (p, 2H, J = 6.6 Hz), 2.74 (t, 2H, J = 7.0 Hz), 3.93 (t, 2H, J = 6.0 Hz),

7.40-7.90 (m, 7H) and 8.35 (d, 1H, J = 8.0 Hz).

$^{13}C$ NMR ($CDCl_3$) 16.1, 31.4, 61.3, 78.9, 94.4, 121.3, 125.0, 126.0, 126.1, 126.4, 127.9, 128.1, 129.9, 133.0 and 133.2.

MS m/e (% abundance): 210 ($M^+$, 100), 191 (19), 165 (71) and 141 (15).

5-(1-Naphthyl)-pentan-1-ol

A mixture of 5-(1-napthyl)-4-pentyn-1-ol (500 ng, 2.37 mmol) and 5% palladium on barium sulfate (5 mg) in ethyl ether (30 ml) was stirred at room temperature under a hydrogen atmosphere for two days. Solvent removal, after filtration through celite, gave the title compound as a yellow oil.

$^1H$ NMR ($CDCl_3$): 8.0 (d, 1H, J = 7.8 Hz), 7.85 (d, 1H, J = 7.11 Hz), 7.69 (d, 1H, J = 7.9 Hz), 7.48 (m, 2H), 7.36 (m, 2H), 3.6 (t, 2H, J = 6.7 Hz), 3.08 (t, 2H, J = 7.6 Hz), 1.7 (m, 2H), 1.6 (m, 2H), and 1.26 (m, 2H).

1-Bromo-5-(1-naphthyl)-pentane

Bromine (a 2M solution in dichloromethane; (912.7 mg, 5.71 mmol) was added to a 0° solution of 1,2-bis(diphenyl)phosphinoethane (1.1 g, 2.85 mmol) in dichloromethane (15 ml). A solution of 5-(1-naphthyl) pentan-1-ol (489 mg, 2.28 mmol) in dichloromethane (5 ml) was added to the solution at 0°. The solution was warmed to room temperature and stirred 2 hours. Ethyl ether then pentane was added, the solution was filtered through a thin pad of silica gel and the solvent evaporated. The resulting oil was flash chromatographed to give the title bromide as a colorless oil.

$^1H$ NMR ($CDCl_3$): 8.0 (d, 1H, J = 7.8 Hz), 7.8 (d, 1H, J = 7.4 Hz), 7.7 (d, 1H, J = 8.3 Hz), 7.4 (m, 4H), 3.4 (t, 2H, J = 6.8 Hz), 3.1 (t, 2H, J = 7.5 Hz), 1.9 (m, 2H), 1.8 (m, 2H), and 1.58 (m, 2H).

3-[1-Acetoxy-6-(1-naphthyl)hexyl]-5-trimethylsilylfuran

Tert-butyl lithium (a 1.7 M solution in hexane (1.27 ml, 2.16 mmol) was added to a -78° solution of 1-bromo-5-(1-naphthyl)-pentane (300 mg, 1.08 mmol) in tetrahydrofuran (2 ml) under nitrogen. After stirring for 30 minutes at -78°, a solution of 5-trimethylsilyl-3-furaldehyde (182 mg, 1.08 mmol) in tetrahydrofuran (0.5 ml) was added and the solution was stirred 2 hours at room temperature. Acetic anhydride (20 mg, 0.2 mmol) was added and the solution stirred at room temperature overnight. The reaction was quenched with water. Extraction (ethyl ether) and evaporation of the dried (magnesium sulfate) extracts gave an oil. Purification of the crude material by preparative TLC (developed with 10% ethyl ether/hexane) gave the title compound as a yellow oil.

$^1H$ NMR ($CDCl_3$): 8.01 (d, 1H, J = 7.7 Hz), 7.85 (d, 1H, J = 7.5 Hz), 7.7 (d, 1H, J = 8.2 Hz), 7.58 (s, 1H), 7.4 (m, 4H), 6.57 (s, 1H), 5.76 (t, 1H, J = 6.6 Hz), 3.04 (t, 2H, 7.8 Hz), 2.01 (s, 3H), 1.85 (m, 2H), 1.74 (m, 2H), 1.4 (m, 4H), and 0.25 (s, 9H).

$^{13}C$ NMR ($CDCl_3$): 170.5, 161.2, 144.4, 138.6, 133.8, 131.8, 128.7, 126.4, 125.8, 125.6, 125.5, 125.3, 124.8, 123.8, 118.5, 68.5, 34.7, 32.9, 30.6, 29.3, 25.4, 21.3, and -1.7.

MS exact mass calculated for $C_{25}H_{32}O_3Si$ ($M^+$) 408.212, found 408.2113.

3-[1-Acetoxy-6-(1-naphthyl)hexyl]-5-hydroxy-2(5H)-furanone

A mixture of 3-[1-acetoxy-6-(1-naphthyl)hexyl]-5-trimethylsilylfuran (23 mg, 0.056 mmol) and Rose Bengal (5 mg) in acetone (20 ml) at -78° was exposed to singlet oxygen for 1.5 hours. The residue, after solvent removal, was purified by preparative TLC (developed with 60% ethyl ether/hexane). The title compound was obtained as a yellow oil.

$^1H$ NMR ($CDCl_3$): 8.01 (d, 1H, J = 7.7 Hz), 7.8 (d, 1H, J = 7.4 Hz), 7.7 (d, 1H, J = 8.2 Hz), 7.4 (m, 4H), 6.15 (br, 1H), 5.95 (br, 1H), 5.41 (br, 1H), 3.06 (t, 2H, J = 7.6 Hz), 2.1 (s, 3H), 1.8 (m, 6H), and 1.35 (m, 2H).

$^{13}C$ NMR ($CDCl_3$): 171.2, 169.7, 166.9, 138.3, 133.8, 131.7, 128.8, 126.6, 126.3, 125.9, 125.7, 125.5, 125.4, 123.7, 119.1, 118.5, 97.9, 76.6, 76.3, 76.2, 69.7, 69.1, 33.0, 32.9, 30.6, 30.4, 29.1, 24.9, 20.8, and -0.025.

EXAMPLE 64

3-(1-Acetoxy-6-phenylhexyn-2-yl)-5-trimethylsilylfuran

n-Butyl lithium (a 1.6 M solution in hexane; 0.9 ml, 1.44 mmol) was added dropwise to a solution of 5-phenyl-1-pentyne (198.1 mg, 1.38 mmol) in tetrahydrofuran (4 ml) at 0° under argon. After 30 minutes, a solution of 5-trimethylsilyl-3-furaldehyde (231 mg, 1.38 mmol) in tetrahydrofuran (1 ml) was added. Stirring was continued for 1 hour and acetic anhydride (0.4 ml) was added. After 2 hours, the mixture was poured into water and extracted thoroughly with ethyl ether. Evaporation of the dried (magnesium sulfate) extracts gave an oil, which was flash chromatographed on silica using 5% ethyl ether/hexane. Fractions with $R_f$ of about 0.33 on evaporation gave the title ester as an oil.

$^1$H NMR ($CDCl_3$): 0.28 (s, 9H), 1.88 (p, 2H, J=7.3 Hz), 2.12 (s, 3H), 2.29 (t, 2H, J=7.0 Hz), 2.75 (t, 2H, 3=7.4 Hz), 6.43 (brs, 1H), 6.72 (s, 1H), 7.15-7.35 (m, 5H) and 7.77 (s, 1H).

MS m/e (% abundance): 354 ($M^+$, 14), 312 (6), 294 (9), 266 (5), 221 (5), 208 (8), 155 (7), 117 (16) and 91 (16).

4-(1-Acetoxy-6-phenylhexyn-2-yl)-5-hydroxy-2(5H)-furanone

A mixture of 3-(1-acetoxy-6-phenylhexyn-2-yl)-5-trimethylsilylfuran (285 mg, 0.8 mmol) and Rose Bengal (5 mg) in tetrahydrofuran (7 ml) was exposed to singlet oxygen at -78° for 2.5 hours. The residue, after solvent removal, was purified by preparative TLC (developed with 60% ethyl ether/hexane). The title furanone was obtained as a yellow oil.

$^1$H NMR ($CDCl_3$): 1.87 (p, 2H, J=7.3 Hz), 2.18 (brs, 3H), 2.28 (brt, 2H), 2.71 (t, 2H, J=7.4 Hz), 5.85 (br, 1H), 6.15 (br, 1H), 6.23 (1H), 6.28 (s, 1H) and 7.15-7.35 (m, 5H).

$^{13}$C NMR ($CDCl_3$): 17.9, 20.6, 29.5, 34.6, 58.7, 59.4, 59.6, 72.5, 72.7, 89.1, 89.2, 97.3, 97.6, 118.4, 121.6, 125.9, 126.2, 128.3, 128.7, 140.9, 162.9, 163.0, 169.6 and 170.2.

MS m/e (% abundance): 332 [$(M+NH_4)^+$, 10], 315 [$(M+H)^+$, 1), 297 (4), 274 (20), 273 (53), 256 (20), 255 (100) and 229 (17).

EXAMPLE 65

4-(1-Acetoxy-12-hydroxydodecyl)-2-trimethylsilylfuran

A solution of 4-(1-acetoxy-12-t-butyldimethylsilyloxydodecyl)-2-trimethylsilylfuran, see Example 61, (0.297 g, 0.6 mmol) and aqueous acetic acid (2 ml, 1:1) in tetrahydrofuran (4ml) was stirred at room temperature for 60 hours and poured into ethyl ether/saturated sodium bicarbonate solution. The organic portion was washed twice with saturated sodium bicarbonate, once with water, once with saturated sodium chloride solution, dried over magnesium sulfate, filtered and concentrated to give a pale oil. This material was purified by flash chromatography (silica, 30% ethyl acetate/hexane) to give recovered starting material and the desired alcohol.

IR ($CHCl_3$): 3610, 3450 (br), and 1720 $cm^{-1}$.

$^1$H NMR ($CDCl_3$): 7.62 (s, 1H), 6.62 (s, 1H), 5.79 (t, 1H, J=7.0 Hz), 3.65 (t, 2H, J=6.6 Hz), 2.07 (s, 3H), 1.9-1.2 (m, 20H), and 0.28 (s, 9H).

$^{13}$C NMR ($CDCl_3$): 170.6, 161.1, 144.4, 124.9, 118.6, 68.6, 62.9, 34.7, 32.7, 29.5, 29.4, 29.2, 25.7, 25.5, 21.3, and -1.7.

m/z Calculated for $C_{21}H_{38}O_4Si(M^+)$:382.2539; obtained ($CI^+$): 382.2547.

4-(1,12-diacetoxydodecyl)-2-trimethylsilylfuran

To a solution of 4-(1-acetoxy-12-hydroxydodecyl)-2-trimethylsilylfuran and pyridine in tetrahydrofuran at 0° under nitrogen is added acetyl chloride. The mixture is warmed to room temperature, stirred for one hour, quenched with water, and extracted into ethyl ether. The organic portion is washed with 10% hydrochloric acid, saturated sodium bicarbonate, 10% copper sulfate solution, water, saturated sodium chloride solution, dried over magnesium sulfate, filtered and concentrated to give the diacetate.

4-(1,12-diacetoxydodecyl)-5-hydroxy-2(5H)-furanone

A stirred solution of 4-(1,11-diacetoxydodecyl)-2-trimethylsilylfuran and Rose Bengal in tetrahydrofuran is flushed with oxygen and cooled to -78°. The solution is irradiated with a 200 Watt flood lamp while under constant positive pressure of oxygen until starting material is no longer visible by TLC. The solution is warmed to room temperature, concentrated and purified by silica chromatography to give the title compound.

EXAMPLE 66

4-(1-Acetoxy-12-hydroxydodecyl)-5-hydroxy-2(5H)-furanone

A stirred solution of 4-(1-acetoxy-12-hydroxydodecyl)-2-trimethylsilylfuran (0.045 g, 0.12 mmol) and Rose Bengal (trace) in tetrahydrofuran (10ml) was flushed with oxygen and cooled to -78°. The solution was irradiated with a 200 Watt flood lamp while under constant positive pressure of oxygen until starting material was no longer visible by TLC (about 1 hour). The solution was warmed to room temperature, concentrated to a pink gum, and purified by preparative thin layer chromatography (1:1 hexane/ethyl acetate). The hydroxybutenolide was isolated as a gum (1:1 hexane/ethyl acetate).

IR ($CHCl_3$): 3350 (br), 1760, 1745 $cm^{-1}$.

$^1H$ NMR (Mixture of diasteriomers) ($CDCl_3$): 6.2 (brs, 0.5H), 6.0 (brm, 1.5H), 5.50 (t, 1H), 3.65 (t, 2H, J=6.6 Hz), 2.16 and 2.12 (2s, 3H), and 2.0-1.3 (m, 20H).

m/z Calculated for $C_{18}H_{31}O_6$ ($MH^+$):343.2120; obtained ($CI^+$): 343.2133.

EXAMPLE 67

4-(1-Acetoxy-11-carboxyundecyl)-2-trimethylsilylfuran

To a solution of 4-(1-acetoxy-12-hydroxydodecyl)-2-trimethylsilylfuran (0.083 g, 0.22 mmol) in acetone (5ml) at room temperature was added Jones reagent until the orange color persisted in the reaction mixture. Ethyl ether was added, and the resulting suspension filtered through celite. The filtrate was then washed twice with water, once with sodium chloride solution, dried over magnesium sulfate, filtered and concentrated to give a colorless gum. The carboxylic acid was purified by preparative thin layer chromatography (40% ethyl acetate/hexane).

IR ($CHCl_3$): 2400-3300 (br), 1725, and 1710 $cm^{-1}$.

$^1H$ NMR ($CDCl_3$): 7.60 (s, 1H), 6.60 (s, 1H), 5.77 (t, 1H, J=7 Hz), 2.06 (s, 3H), 2.4-1.2 (m, 20H), 0.26 (s, 3H).

$^{13}C$ NMR ($CDCl_3$): 170.6, 161.1, 144.4, 124.9, 118.6, 68.6, 34.7, 29.4, 29.2, 29.1, 25.5, 24.8, 21.3, -1.7.

m/z Calculated for $C_{21}H_{36}O_5Si$ ($M^+$): 396.2332; obtained ($CH_4CI^+$): 396.2332.

4-(1-Acetoxy-11-carboxydodecyl)-5-hydroxy-2(5H)-furanone

A stirred solution of 4-(1-acetoxy-11-carboxydodecyl)-2-trimethylsilylfuran and Rose Bengal in tetrahydrofuran is flushed with oxygen and cooled to -78°. The solution is irradiated with 200 Watt flood lamp while under a constant positive pressure of oxygen until starting material is no longer visible by TLC. The solution is warmed to room temperature, concentrated, and the residue purified to give the title compound.

EXAMPLE 68

The following test procedures may be used to demonstrate activity of the compounds of this invention:

Inhibition of Phospholipase $A_2$

The effect of compounds of this invention on bee venom phospholipase $A_2$ is determined by the following procedure:

a. Bee venom phospholipase $A_2$ in 10 μM HEPES (pH 7.4) with 1 mM $CaCl_2$ is incubated with vehicle or test agent for 1.0 hour at 41°.

b. 1.36 mM phosphotidylcholine, 2.76 mM Triton X-100 are dispersed in buffer by sonication and then mixed with L-3 phosphatidylcholine, 1-palmitoyl-2-(1-$^{14}C$) palmitoyl for 10 min.

c. Start the reaction by the addition of enzyme (0.495 units/ml).

d. Incubation for 15 sec. at 41°.

e. Reaction is terminated by addition of 2.5 ml of isopropanol: n-heptane: 0.5 M $H_2SO_4$ (40:10:1; v:v:v).

f. 2.0 ml n-heptane and 1.0 ml $H_2O$ added; mixture centrifuged.

g. 2.0 ml n-heptane removed and treated with 200-300 mg of silica gel HR60.

h. Samples centrifuged; 1 ml of n-heptane SN removed and added to 10 ml scintillation fluid.

i. Samples counted on a scintillation counter.

Mouse Ear Anti-Inflammatory Assay

Test compound and phorbol myristate acetate (PMA) are topically applied simultaneously to the pinnae of the left ears of mice. PMA alone is applied to the right ear. Three hours and 20 minutes after application, the mice are sacrificed, left and right ears removed, and standard sized bores taken. Edema (inflammation) is measured as the difference in weight between left and right ears [Van Arman, C.G., Clin Pharmacol Ther (1974) 16:900-904].

Inhibition of Ornithine Decarboxylase (ODC)

Tape-stripping mouse epidermis and TPA are quick and convenient methods of inducing ODC activity. M. Connor and N. Lowe (Cancer Res. 43, 5174, 1983; Brit. J. Dermatol. 275, 98, 1984) have studied the ability of retinoids to inhibit ODC. Trans-retinoic acid, 13-cis retinoic acid, and etretinate were all active at inhibiting ODC and therapeutically active in humans. Therefore, inhibition of ODC is an in vivo method to demonstrate the potential efficacy of drugs for epidermal hyperproliferation such as psoriasis. Lowe et al. (J. Amer. Acad. Dermatol. 6:697, 1982) have shown that polyamines and ODC are elevated in psoriasis.

In vitro methods have also been useful in determining the anti-hyperproliferative activity of drugs. C. Marcelo and 3. Tomich (J. Invest. Dermatol. 81, 64s, 1983) have shown that neonatal mouse keratinocyte cultures can be used to identify drugs that inhibit DNA synthesis. More recently, R. Weiss, Eichner, R. and Sunn, T. T, J. Cell Biol., 98:1397-1406, (1984) have shown that epidermal cultures are in fact, a model of epidermal hyperproliferation and therefore a good model for testing drugs that inhibit hyperproliferation.

Calcium Channel (mobilization) inhibition assay.

Polymorphonuclear leukocytes (PMNa), gastric glands, $GH_3$ cells, A431 cells, spleen cells, human keratinocytes corneal cells, etc. were loaded with the $Ca^{2+}$ sensitive fluorescent dye, Fura-2. The appropriate cell type was chosen and the potency and efficacy of the anti-inflammatory furanones on calcium mobilization, calcium channel inhibition quantitated. The methods used for A431 cells listed below are representative of those used for other cells.

A431 cells were detached using a 5-10 min trypsin-EDTA treatment whereas $GH_3$ cells were treated 2 to 5 min with a 1% pancreatin solution. Cells were immediately washed twice in a 20mM HEPES buffer (pH 7.4) containing 120mM NaCl, 6 mM KCl, 1 mM $MgSO_4$, 1 mg/ml glucose and 1 mg/ml pyruvate and 1.4mM calcium (medium A). Approximately 5 x $10^6$ cells were suspended in medium A and incubated with 4µM fura-2-AM for 15 min at 37°C. After washing the fura-2 loaded cells, the uptake of dye was checked using fluorescence microscopy and found to be evenly distributed in the cytosol of all cells. Fluorescence was continuously recorded with a Perkin-Elmer LS-5 spectrofluorometer. The excitation wavelength was set at 340nm and emission wavelength set at 500nm. The cell suspension was continually stirred, maintained at 37°C and equilibrated for approximately 5 min before addition of various agents. $[Ca^{2+}]_i$ was calculated using the following formula:

$$[Ca^{2+}]_i = 220 \frac{F - F_{min}}{F_{max} - F}$$

All fluorescence values were measured relative to a EGTA-quenched signal determined as follows: F was the relative fluorescence measurement of the sample. $F_{max}$ was determined by lysing the cells with digitonin (100µg/ml) in DMSO. After $F_{max}$ was determined the pH was adjusted to 8, with NaOH and $Ca^{2+}$ chelated with 3mM EGTA to totally quench the fura-2 signal and obtain $F_{min}$.

When quin-2 was used, cells were incubated with 10µM quin-2 at 37°C for 1 hr, washed and then used.

**Claims**

**Claims for the following Contracting States : AT, BE, CH, DE, FR, GB, IT, LI, LU, NL, SE**

**1.** A compound of the formula:

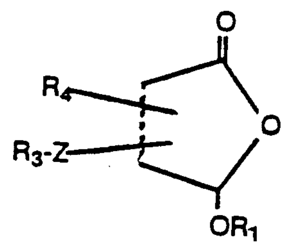

in which:

the dotted line represents a single or double bond;

$R_1$ is hydrogen, $C_1$-$C_4$ alkyl, benzyl or $C_1$-$C_{20}$ alkanoyl, cyclohexanoyl, benzoyl, phenyl ($C_{1-4}$ alkanoyl) or naphthoyl;

Z is -CO- or -C($OR_2$)H-;

$R_2$ is $C_1$-$C_{20}$ alkanoyl, trihaloacetyl, cyclohexanoyl, benzoyl, phenyl($C_{1-4}$ alkanoyl), phenyl($C_2$-$C_{14}$ alkenoyl), naphthoyl, or carbamoyl optionally N-substituted by one or two $C_{1-4}$ alkyl groups or by one $\alpha$-($C_1$-$C_4$ alkyl)benzyl group;

$R_3$ is hydrogen, $C_1$-$C_{20}$ straight chain alkyl, phenyl($C_1$-$C_{20}$ straight chain alkyl or $C_1$-$C_{20}$ straight chain alkenyl of 1-6 unconjugated double bonds), cyclohexyl($C_1$-$C_{20}$ straight chain alkyl or $C_1$-$C_{20}$ straight chain alkenyl having 1-6 unconjugated double bonds), phenyl, cyclohexyl or benzothienyl($C_1$-$C_{20}$ alkyl or $C_1$-$C_{20}$ alkenyl having 1-6 unconjugated double bonds), 4-methyl-3-pentenyl, 4-methyl-6-(2,6,6-trimethylcyclohex-1-enyl)hex-3-enyl, 1-(2-ethenyl)-1,5,9-trimethyldeca-4,8-dienyl, Y-$(CH_2)_n$ or B-(straight chain $C_3$-$C_{14}$ alkynyl);

$R_4$ is hydrogen, bromo or chloro but is not bromo or chloro when $R_3$ contains a double bond;

n is 6-12;

Y is $OR_5$, $CO_2R_6$, t-butyl,
t-butyldimethylsilyl, diphenylmethyl, triphenylmethyl, pyridyl, thienyl, quinolyl, N-$C_1$-$C_4$ alkylpyrrolyl, N-$C_1$-$C_4$ alkylpiperidyl, N-$C_1$-$C_4$ alkylpyridinium halide or naphthyl;

$R_5$ is hydrogen, $C_1$-$C_4$ alkyl or $C_1$-$C_4$ alkanoyl;

$R_6$ is hydrogen or $C_1$-$C_4$ alkyl;

B is hydrogen, phenyl, pyridyl or naphthyl; said phenyl in the definition of $R_3$ being optionally substituted by $C_1$-$C_{14}$ alkyl, $C_1$-$C_{14}$ alkenyl, $C_1$-$C_{14}$ alkynyl or aryl, $CO_2R_6$, $C_1$-$C_4$ alkoxy or halo.

**2.** A compound of claim 1 where $R_3$-Z- is in the 3-position on the 2-furanone ring.

**3.** A compound of claim 2 in which the dotted line represents a double bond; Z is -C($OR_2$)H-; and $R_3$ is hydrogen, $C_1$-$C_{20}$ straight chain alkyl, phenyl ($C_1$-$C_{20}$ straight chain alkyl), cyclohexyl ($C_1$-$C_{20}$ straight chain alkyl), phenyl, cyclohexyl or benzothienyl ($C_1$-$C_{20}$ alkyl), benzothienyl($C_1$-$C_{20}$alkyl) 4-methyl-3-pentenyl, 4-methyl-6-(2,6,6-trimethylcyclohex-1-enyl)hex-3-enyl, 1-(2-ethenyl)-1,5,9-trimethyldeca-4,8-dienyl, Y-$(CH_2)_n$ or B-(straight chain $C_3$-$C_{14}$ alkynyl).

**4.** A compound of claim 1 where $R_3$-Z- is in the 4-position on the 2-furanone ring.

**5.** A compound of claim 4 where $R_1$ is hydrogen, or $C_1$-$C_{20}$ alkanoyl; $R_2$ is $C_1$-$C_{20}$ alkanoyl or carbamoyl optionally N-substituted by one or two $C_{1-4}$ alkyl groups or by one $\alpha$-($C_1$-$C_4$ alkyl)benzyl group; $R_3$ is $C_5$-$C_{20}$ straight chain alkyl, benzothienyl($C_1$-$C_{20}$ alkyl). 4-methyl-3-pentenyl, 4-methyl-6-(2,6,6,-trimethylcyclohex-1-enyl)hex-3-enyl or 1-(2-ethenyl)-1,5,9-trimethyldeca-4,8-dienyl, Y-$(CH_2)_n$ or B-(straight chain $C_3$-$C_{14}$ alkynyl); and $R_4$ is hydrogen.

**6.** A compound of claim 5 in which the dotted line represents a double bond and Z is -C($OR_2$)H-.

**7.** A compound of claim 6 which is

4-[1-acetoxy-7-benzo(b)thien-2-yl-heptyl]-5-hydroxy-2(5H)-furanone;

4-(1-acetoxy-tridecyl)-5-hydroxy-2(5H)-furanone;

4-[1-acetoxy-6-phenylhexyl)-5-hydroxy-2(5H)-furanone;

4-[1-acetoxy-6-(2-naphthyl)hexyl)-5-hydroxy-2(5H)-furanone;

4-[1-($\alpha$-methylbenzylcarbamoyloxy)tridecyl]-5-hydroxy-2(5H)-furanone; and

4-[1-acetoxy-5-methyl-7-(2,6,6-trimethylcyclohex-1-enyl)-hept-4-enyl]-5-hydroxy-2(5H)-furanone.

**8.** A compound as defined in any one of claims 1 to 7 for use in medicine.

**9.** A pharmaceutical composition comprising a compound as defined in any one of claims 1 to 7 and a pharmaceutically acceptable carrier.

**10.** A process for preparing a compound as defined in any one of claims 1 to 7, which process comprises:
(i) when it is desired to prepare a compound wherein $R_1$ is hydrogen and Z is $-C(OR_2)H-$, the irradiation of a 4-($R_3$-CH($OR_2$)-2-trimethylsilylfuran in the presence of oxygen and an initiator; or
(ii) when it is desired to prepare a compound wherein Z is a group $-C(OR_2)H-$, the reaction of the corresponding compound, wherein $R_1$ and/or $R_2$ are hydrogen with the appropriate acylating agent-(s); or
(iii) when it is required to prepare a compound wherein Z is a group -CO-;
(a) irradiating a 4-($R_3$-CO)-2-trimethylsilylfuranone as defined in this claim in the presence of oxygen and an initiator; or
(b) irradiating a 4-($R_3$-CO)-2-trimethylsilylfuran in the presence of oxygen and an initiator; or
(c) reacting a 4-($R_3$-CHOH)-5-($R_1$O)-furanone with an oxidising agent.

**Claims for the following Contracting States : ES, GR**

**1.** A process for preparing a compound of the formula (I):

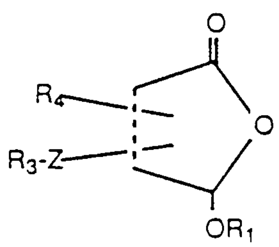

(I)

in which:
the dotted line represents a single or double bond;
$R_1$ is hydrogen;
Z is $-C(OR_2)H-$;
$R_2$ is $C_1$-$C_{20}$ alkanoyl, trihaloacetyl, cyclohexanoyl, benzoyl, phenyl($C_{1-4}$ alkanoyl), phenyl($C_2$-$C_{14}$ alkenoyl), naphthoyl, or carbamoyl optionally N-substituted by one or two $C_{1-4}$ alkyl groups or by one $\alpha$-($C_1$-$C_4$ alkyl)benzyl group;
$R_3$ is hydrogen, $C_1$-$C_{20}$ straight chain alkyl, phenyl($C_1$-$C_{20}$ straight chain alkyl or $C_1$-$C_{20}$ straight chain alkenyl of 1-6 unconjugated double bonds), cyclohexyl($C_1$-$C_{20}$ straight chain alkyl or $C_1$-$C_{20}$ straight chain alkenyl having 1-6 unconjugated double bonds), phenyl, cyclohexyl or benzothienyl($C_1$-$C_{20}$ alkyl or $C_1$-$C_{20}$ alkenyl having 1-6 unconjugated double bonds), 4-methyl-3-pentenyl, 4-methyl-6-(2,6,6-trimethylcyclohex-1-enyl)hex-3-enyl, 1-(2-ethenyl)-1,5,9-trimethyldeca-4,8-dienyl, Y-$(CH_2)_n$ or B-(straight chain $C_3$-$C_{14}$ alkynyl);
$R_4$ is hydrogen, bromo or chloro but is not bromo or chloro when $R_3$ contains a double bond;
n is 6-12;
Y is $OR_5$, $CO_2R_6$, t-butyl,
t-butyldimethylsilyl, diphenylmethyl, triphenylmethyl, pyridyl, thienyl, quinolyl, N-$C_1$-$C_4$ alkylpyrrolyl, N-$C_1$-$C_4$ alkylpiperidyl, N-$C_1$-$C_4$ alkylpyridinium halide or naphthyl;
$R_5$ is hydrogen, $C_1$-$C_4$ alkyl or $C_1$-$C_4$ alkanoyl;
$R_6$ is hydrogen or $C_1$-$C_4$ alkyl; and
B is hydrogen, phenyl, pyridyl or naphthyl; said phenyl in the definition of $R_3$ being optionally substituted by $C_1$-$C_{14}$ alkyl, $C_1$-$C_{14}$ alkenyl, $C_1$-$C_{14}$ alkynyl or aryl, $CO_2R_6$, $C_1$-$C_4$ alkoxy or halo;
which process comprises irradiating a 4-[$R_3$-CH($OR_2$)]-2-trimethylsilylfuran in the presence of oxygen and an initiator.

**2.** A process for preparing a compound of the formula (I):

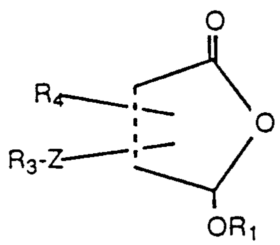

(I)

in which:

the dotted line represents a single or double bond;

$R_1$ is $C_1$-$C_4$ alkyl, benzyl, $C_1$-$C_{20}$ alkanoyl, cyclohexanoyl, benzoyl, phenyl ($C_{1-4}$ alkanoyl) or naphthoyl;

Z is -C($OR_2$)H-;

$R_2$ is $C_1$-$C_{20}$ alkanoyl, trihaloacetyl, cyclohexanoyl, benzoyl, phenyl($C_{1-4}$ alkanoyl), phenyl($C_2$-$C_{14}$ alkenoyl), naphthoyl, or carbamoyl optionally N-substituted by one or two $C_{1-4}$ alkyl groups or by one $\alpha$-($C_1$-$C_4$ alkyl)benzyl group;

$R_3$ is hydrogen, $C_1$-$C_{20}$ straight chain alkyl, phenyl($C_1$-$C_{20}$ straight chain alkyl or $C_1$-$C_{20}$ straight chain alkenyl of 1-6 unconjugated double bonds), cyclohexyl($C_1$-$C_{20}$ straight chain alkyl or $C_1$-$C_{20}$ straight chain alkenyl having 1-6 unconjugated double bonds), phenyl, cyclohexyl or benzothienyl($C_1$-$C_{20}$ alkyl or $C_1$-$C_{20}$ alkenyl having 1-6 unconjugated double bonds), 4-methyl-3-pentenyl, 4-methyl-6-(2,6,6-trimethylcyclohex-1-enyl)hex-3-enyl, 1-(2-ethenyl)-1,5,9-trimethyldeca-4,8-dienyl, Y-$(CH_2)_n$ or B-(straight chain $C_3$-$C_{14}$ alkynyl);

$R_4$ is hydrogen, bromo or chloro but is not bromo or chloro when $R_3$ contains a double bond;

n is 6-12;

Y is $OR_5$, $CO_2R_6$, t-butyl,
t-butyldimethylsilyl, diphenylmethyl, triphenylmethyl, pyridyl, thienyl, quinolyl, N-$C_1$-$C_4$ alkylpyrrolyl, N-$C_1$-$C_4$ alkylpiperidyl, N-$C_1$-$C_4$ alkylpyridinium halide or naphthyl;

$R_5$ is hydrogen, $C_1$-$C_4$ alkyl or $C_1$-$C_4$ alkanoyl;

$R_6$ is hydrogen or $C_1$-$C_4$ alkyl; and

B is hydrogen, phenyl, pyridyl or naphthyl; said phenyl in the definition of $R_3$ being optionally substituted by $C_1$-$C_{14}$ alkyl, $C_1$-$C_{14}$ alkenyl, $C_1$-$C_{14}$ alkynyl or aryl, $CO_2R_6$, $C_1$-$C_4$ alkoxy or halo;

which process comprises reacting the corresponding furanone wherein $R_1$ and/or $R_2$ is hydrogen with the appropriate acylating agent(s).

**3.** A process for preparing a compound of the formula (I):

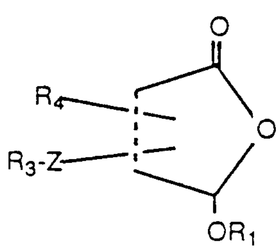

(I)

in which:

the dotted line represents a single or double bond;

$R_1$ is hydrogen;

Z is -CO-;

$R_3$ is hydrogen, $C_1$-$C_{20}$ straight chain alkyl, phenyl($C_1$-$C_{20}$ straight chain alkyl or $C_1$-$C_{20}$ straight chain alkenyl of 1-6 unconjugated double bonds), cyclohexyl($C_1$-$C_{20}$ straight chain alkyl or $C_1$-$C_{20}$

straight chain alkenyl having 1-6 unconjugated double bonds), phenyl, cyclohexyl or benzothienyl($C_1$-$C_{20}$ alkyl or $C_1$-$C_{20}$ alkenyl having 1-6 unconjugated double bonds), 4-methyl-3-pentenyl, 4-methyl-6-(2,6,6-trimethylcyclohex-1-enyl)hex-3-enyl, 1-(2-ethenyl)-1,5,9-trimethyldeca-4,8-dienyl, Y-$(CH_2)_n$ or B-(straight chain $C_3$-$C_{14}$ alkynyl);

$R_4$ is hydrogen, bromo or chloro but is not bromo or chloro when $R_3$ contains a double bond;

n is 6-12;

Y is $OR_5$, $CO_2R_6$, t-butyl, t-butyldimethylsilyl, diphenylmethyl, triphenylmethyl, pyridyl, thienyl, quinolyl, N-$C_1$-$C_4$ alkylpyrrolyl, N-$C_1$-$C_4$ alkylpiperidyl, N-$C_1$-$C_4$ alkylpyridinium halide or naphthyl;

$R_5$ is hydrogen, $C_1$-$C_4$ alkyl or $C_1$-$C_4$ alkanoyl;

$R_6$ is hydrogen or $C_1$-$C_4$ alkyl; and

B is hydrogen, phenyl, pyridyl or naphthyl; said phenyl in the definition of $R_3$ being optionally substituted by $C_1$-$C_{14}$ alkyl, $C_1$-$C_{14}$ alkenyl, $C_1$-$C_{14}$ alkynyl or aryl, $CO_2R_6$, $C_1$-$C_4$ alkoxy or halo;

which process comprises:

(i) irradiating a 4-($R_3$-CO)-2-trimethylsilylfuranone as defined in this claim in the presence of oxygen and an initiator; or

(ii) irradiating a 4-($R_3$-CO)-2-trimethylsilylfuran in the presence of oxygen and an initiator; or

(iii) reacting a 4-($R_3$-CHOH)-5-($R_1$O)-furanone with an oxidising agent.

**4.** A process according to any one of claims 1, 2 or 3 where $R_3$-Z- is in the 4-position on the 2-furanone ring.

**5.** A process according to any one of claims 1, 2 or 3 where $R_3$-Z- is in the 3-position on the 2-furanone ring.

**6.** A process according to claim 1 which gives the compound
4-[1-acetoxy-7-benzo(b)thien-2-yl-heptyl]-5-hydroxy-2(5H)-furanone;
4-(1-acetoxy-tridecyl)-5-hydroxy-2(5H)-furanone;
4-[1-acetoxy-6-phenylhexyl)-5-hydroxy-2(5H)-furanone;
4-[1-acetoxy-6-(2-naphthyl)hexyl)-5-hydroxy-2(5H)-furanone; or
4-[1-(α-methylbenzylcarbamoyloxy)tridecyl]-5-hydroxy-2(5H)-furanone.

**7.** A process according to claim 1 which gives the compound 4-[1-acetoxy-5-methyl-7-(2,6,6-trimethylcyclohex-1-enyl)-hept-4-enyl]-5-hydroxy-2(5H)-furanone.

**8.** A process for preparing a pharmaceutical composition containing a compound of formula I as prepared in any one of claims 1 to 7 which process comprises bringing the compound into association with a pharmaceutically acceptable carrier.

## Revendications

**Revendications pour les Etats contractants suivants : AT, BE, CH, DE, FR, GB, IT, LI, LU, NL, SE**

**1.** Composé de formule :

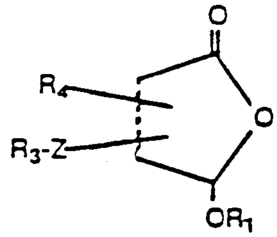

dans laquelle :

la ligne en pointillés représente une liaison simple ou double;

$R_1$ est un hydrogène, un alkyle en $C_1$-$C_4$, un benzyle, un alkanoyle en $C_1$ à $C_{20}$, un cyclohexanoyle, un

benzoyle, un phényl-(alkanoyle en $C_1$ à $C_4$) ou un naphtoyle;

Z est -CO - ou - $C(OR_2)$ H-;

$R_2$ est un alkanoyle en $C_1$ à $C_{20}$, un trihaloacétyle, un cyclohexanoyle, un benzoyle, un phényl-(alkanoyle en $C_1$ à $C_4$), un phényl-(alkénoyle en $C_2$ à $C_{14}$), un naphtoyle ou un carbamoyle éventuellement N-substitué par un ou deux groupes alkyle en $C_1$ à $C_4$ ou par un groupe alpha-(alkyl en $C_1$ à $C_4$)-benzyle;

$R_3$ est un hydrogène, une chaîne alkyle linéaire en $C_1$ a $C_{20}$, un phényl-(chaîne alkyle linéaire en $C_1$ à $C_{20}$ ou chaîne alkényle linéaire en $C_1$ à $C_{20}$ présentant de 1 à 6 doubles liaisons non conjugées), une cyclohexyle (chaîne alkyle linéaire en $C_1$ à $C_{20}$ ou chaîne alkényle linéaire en $C_1$ à $C_{20}$présentant de 1 à 6 doubles liaisons non conjuguées), un phényle, un cyclohexyle ou un benzothiényl-(alkyle en $C_1$ à $C_{20}$ ou alkényle en $C_1$ à $C_{20}$ présentant de 1 à 6 doubles liaisons non conjuguées), un méthyl-4-penténylе-3, un méthyl-4-(triméthyl 2,6,6-cyclohexényl-1) 6-hexényle-3, un (éthényl-2)-1-triméthyl-1, 5, 9-décadiényle-4, 8, Y-$(CH_2)_n$ ou B-(chaîne alkynyle linéaire en $C_3$ à $C_{14}$);

$R_4$ est un hydrogène, le brome, ou le chlore mais n'est ni le brome ni le chlore quand $R_3$ présente une double liaison;

n est égal à 6 à 12;

Y est $OR_5$, $CO_2R_6$, un t-butyle, un t-butyldiméthylsilyle, un diphénylméthyle, un triphénylméthyle, un pyridyle, un thiényle, un quinolyle, un N-alkylnyrolyle en $C_1$ à $C_4$ un N-alkylpipéridyle en $C_1$ à $C_4$, un halogénure de N-alkylpyridinium en $C_1$ à $C_4$ ou un naphtyle;

$R_5$ est un hydrogène, un alkyle en $C_1$ à $C_4$ ou un alkanoyle en $C_1$ à $C_4$;

$R_6$ est un hydrogène ou un malkyle en $C_1$ à $C_4$;

B est un hydrogène, un phényle, un pyridyle ou un naphtyle; ledit phényle dans la définition de $R_3$ pouvant être éventuellement substitué par un alkyle en $C_1$ à $C_{14}$, un alkényle en $C_1$ à $C_{14}$, un alkynyle en $C_1$ à $C_{14}$ ou un aryle, $CO_2R_6$, un alkoxy en $C_1$ à $C_4$ ou un halogène.

**2.** Composé selon la revendication 1 dans lequel $R_3$-Z- est en position 3 dans le cycle furanone-2.

**3.** Composé selon la revendication 2 dans lequel :
la ligne en pointillés représente une double liaison
Z est - $C(OR_2)$H-; et
$R_3$ est un hydrogène, une chaîne alkyle linéaire en $C_1$ à $C_{20}$, une phényl-(chaîne alkyle linéaire en $C_1$ à $C_{20}$), une cyclohexyl-(chaîne alkyle linéaire en $C_1$ à $C_{20}$), un phényle, un cyclohexyle ou un benzothiényl-(alkyle en $C_1$ à $C_{20}$), un méthyl-4-penténylе-3, un méthyl-4-(triméthyl-2,6,6-cyclohexényl-1)-6-hexényle-3, un (éthényl-2)-1-triméthyl-1,5,9-décadiényle 4,8, Y-$(CH_2)_n$ ou une B-(chaîne alkynyle linéaire en $C_3$ à $C_{14}$).

**4.** Composé selon la revendication 1 dans lequel $R_3$ -Z- est en position 4 sur le cycle furanone -2.

**5.** Composé selon la revendication 4 dans lequel :
$R_1$ est un hydrogène ou un alkanoyle en $C_1$ à $C_{20}$, $R_2$ est un alkanoyle en $C_1$ à $C_{20}$ ou un carbamoyle éventuellement N-substitué par un ou deux groupes alkyle en $C_1$ à $C_4$ ou par un groupe alpha-(alkyle en $C_1$ à $C_4$)-benzyle;
$R_3$ est une chaîne alkyle linéaire en $C_5$ à $C_{20}$, un benzothiényl-(alkyle en $C_1$ à $C_{20}$), un méthyl-4-penténylе-3, un méthyl-4-(triméthyl-2,6,6-cyclohexényl-1)-6-hexényle-3 ou un (éthényl-2)-1-triméthyl-1,5,9-décadiényle-4,8, Y-$(CH_2)$, ou une B-(chaîne alkynyle linéaire en $C_3$ à $C_{14}$); et
$R_4$ est un hydrogène.

**6.** Composé selon la revendication 5 dans lequel la ligne en pointillés représente une double liaison et Z est -$C(OR_2)$H-.

**7.** Composé selon la revendication 6 qui est
l' [acétoxy-1-benzo-7(b)-thiényl-2-heptyl]4-hydroxy-5-furanone-2(5H);
l' [acétoxy-1-tridécyl]-4-hydroxy-5-furanone-2(5H);
l'[acétoxy-1-phényl-6-hexyl]-4-hydroxy-5-furanone-2(5H);
l'[acétoxy-1-(naphtyl-2)-6-hexyl]-4-hydroxy-5-furanone-2(5H);
l' [alpha-(méthylbenzylcarbonoyloxy)-1-tridécyl]-4-hydroxy-5-furanone2(5H); et
l' [acétoxy-1-méthyl-5-(triméthyl-2,6,6-cyclohexényl-1)-7-hepténуl-4]-4-hydroxy-5-furanone-2(5H).

**8.** Composé selon l'une quelconque des revendications 1 à 7 pour utilisation en médecine.

**9.** Composition pharmaceutique comprenant un composé tel que défini dans l'une quelconque des revendications 1 à 7, et un support pharmaceutiquement acceptable.

**10.** Procédé pour la préparation d'un composé tel que défini dans l'une quelconque des revendications 1 à 7, ledit procédé comprenant le fait que

(i) si on désire préparer un composé dans lequel $R_1$ est un hydrogène et Z est-C $(OR_2)H$-, on irradie une $(R_3$-$CH(OR_2)$-4-triméthyl-silylfurane-2 en présence d'oxygène et d'un initiateur; ou

(ii) si on désire préparer un composé dans lequel Z est un groupe -$C(OR_2)H$-, on fait réagir le composé correspondant dans lequel $R_1$ et/ou $R_2$ sont l'hydrogène, avec un ou plusieurs agents d'acylation; ou

(iii) si on désire préparer un composé dans lequel Z est un groupe -CO-,

a) on irradie une $(R_3$-CO)-4-timéthylsilylfurane-2, telle que définie dans la présente revendication, en présence d'oxygène et d'un initiateur; ou

b) on irradie un $(R_3CO)$-4-triméthylsilylfurane-2 en présence d'oxygène ou d'un initiateur; ou

c) on fait réagir une $(R_3$-CHOH)-4-$(R_{10})$-5-furanone avec un agent oxydant.

**Revendications pour les Etats contractants suivants : ES, GR**

**1.** Procédé de préparation du composé de formule I

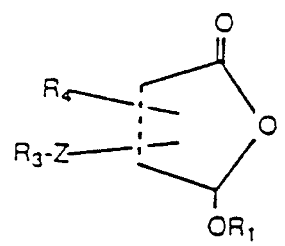

dans laquelle :

la ligne en pointillés représente une liaison simple ou double;

$R_1$ est un hydrogène;

Z est -$C(OR_2)H$- ;

$R_2$ est un alkanoyle en $C_1$ à $C_{20}$, un trihaloacétyle, un cyclohexanoyle, un benzoyle, un phényl-(alkanoyle en $C_1$ à $C_4$), un phényl-(alkénoyle en $C_2$ à $C_{14}$), un naphtoyle ou un carbamoyle éventuellement N-substitué par un ou deux groupes alkyle en $C_1$ à $C_4$ ou par un groupe alpha (akyl en $C_1$ à $C_4$) benzyle;

$R_3$ est un hydrogène, une chaîne alkyle linéaire en $C_1$ à $C_{20}$, une phényl-(chaîne alkyle linéaire en $C_1$ à $C_{20}$ ou chaîne alkényle linéaire en $C_1$ à $C_{20}$ présentant de 1 à 6 doubles liaisons non conjuguées), un phényle, un cyclohexyle ou un benzothiényl-(alkyle en $C_1$ à $C_{20}$ ou alkényle en $C_1$ à $C_{20}$ présentant de 1 à 6 doubles liaisons non conjuguées), un méthyl-4-penténыle-3, un méthyl-4-(triméthyl-2,6,6-cyclohexényl-1)-6-hexényle-3, un (éthényl-2)-1-triméthyl-1, 5, 9-décadiényle-4, 8, Y-$(CH_2)_n$ ou B-(chaîne alkynyle linéaire en $C_3$ à $C_{14}$);

$R_4$ est un hydrogène, un brome ou un chlore mais n'est ni le brome, ni le chlore quand $R_3$ présente une double liaison;

n est égal à 6 à 12;

Y est $OR_5$, $CO_2R_6$, un t-butyle, un t-butyldiméthylsilyle, un diphénylméthyle, un triphénylméthyle un pyridyle, un thiényle, un quinolyle, un N-alkypyrolyle en $C_1$ à $C_4$, un N-lkylpipéridyle en $C_1$ à $C_4$, un halogénure de N-alkylpyridinium en $C_1$ à $C_4$ ou un naphtyle;

$R_5$ est un hydrogène, un alkyle en $C_1$ à $C_4$ ou un alkanoyle en $C_1$ à $C_4$;

$R_6$ est un hydrogène ou un alkyle en $C_1$ à $C_4$

B est un hydrogène, un phényle, un pyridyle ou un naphtyle; ledit phényle dans la définition de $R_3$ pouvant être éventuellement substitué par un alkyle en $C_1$ à $C_{14}$, un alkényle en $C_1$ à $C_{14}$, un alkynyle en $C_1$ à $C_{14}$ ou un aryle, $CO_2R_6$, un alkoxy en $C_1$ à $C_4$ ou un halogène.

ledit procédé comprenant le fait que l'on irradie un $[R_3$-$CH(OR_2)]$-4- triméthylsilylfurane 2 en présence

d'oxygène et d'un initiateur,

**2.** Procédé de préparation d'un composé de formule (I)

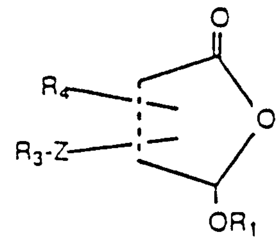

dans laquelle :
la ligne en pointillés est une liaison simple ou double;
$R_1$ est un alkyle en $C_1$ à $C_4$, un benzyle, un alkanoyle en $C_1$ à $C_{20}$, un cyclohexanoyle, un benzoyle, un phényl-(alkanoyle $C_1$ à $C_4$) ou un naphtoyle;
Z est $-C(OR_2)H-$;
$R_2$ est un alkanoyle en $C_1$ à $C_{20}$, un trihaloacétyle, un cyclohexanoyle, un benzoyle, un phényl-(alkanoyle en $C_1$ à $C_4$), un phényl-(alkénoyle en $C_2$ à $C_{14}$), un naphtoyle, ou un carbamoyle éventuellement N-substitué par un ou deux groupes alkyls en $C_1$ à $C_4$ ou par un groupe alpha-(alkyle en $C_1$ à $C_4$)-benzyle;
$R_3$ est un hydrogène, une chaîne alkyle linéaire en $C_1$ à $C_{20}$, une phényl-(chaîne alkyle linéaire en $C_1$ à $C_{20}$ ou chaîne alkényle linéaire en $C_1$ à $C_{20}$ présentant de 1 à 6 doubles liaisons non conjuguées), une cyclohexyl-(chaîne alkyle linéaire en $C_1$ à $C_{20}$ ou chaîne alkényle linéaire en $C_1$ à $C_{20}$ présentant de 1 à 6 doubles liaisons non conjuguées), un phényle, un cyclohexyle ou un benzothiényl-(alkyle en $C_1$ à $C_{20}$ ou alkényle en $C_1$ à $C_{20}$ présentant de 1 à 6 doubles liaisons non conjuguées), un méthyl-4-pentényle-3, un méthyl-4-(triméthyl-2,6,6-cyclohexényl-1)-6 hexényle-3, un (éthényl-2)-1-tryméthyl-1,5,9-décadiényle 4,8, $Y-(CH_2)_n$ ou B-(chaîne alkynyle linéaire en $C_3$ à $C_{14}$);
$R_4$ est un hydrogène, un brome ou un chlore mais n'est ni le brome ni le chlore quand $R_3$ présente une double liaison;
n est égal à 6 à 12;
Y est $OR_5$, $CO_2R_6$, un t-butyle, un t-butyldiméthylsilyle, un diphénylméthyle, un triphénylméthyle un pyridyle, un thiényle, un quinolyle, un N-alkylpyrrolyle en $C_1$ à $C_4$, un N-alkylpipéridyle en $C_1$ à $C_4$, un halogénure de N-alkylpyridinium en $C_1$ à $C_4$ ou un naphtyle;
$R_5$ est un hydrogène, un alkyle en $C_1$ à $C_4$ ou un alkanoyle en $C_1$ à $C_4$;
$R_6$ est un hydrogène ou un malkyle en $C_1$ à $C_4$;
B est un hydrogène, un phényle, un pyridyle ou un naphtyle; ledit phényle dans la définition de $R_3$ pouvant être éventuellement substitué par un alkyle en $C_1$ à $C_{14}$, un alkényle en $C_1$ à $C_{14}$, un alkynyle en $C_1$ à $C_{14}$ ou un aryle, $CO_2R_6$, un alkoxy en $C_1$ à $C_4$ ou un halogène ;
ledit procédé comprenant le fait que l'on fait réagir la furanone correspondante dans laquelle $R_1$ et/ou $R_2$ sont un hydrogène, avec un ou plusieurs agents d'acylation adéquats.

**3.** Procédé de préparation d'un composé de formule I

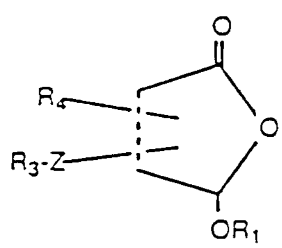

dans laquelle :
la ligne en pointillés représente une liaison simple ou double;

R est un hydrogène ;
Z est -CO- ;
$R_3$ est un hydrogène, une chaîne alkyle linéaire en $C_1$ à $C_{20}$, une phényl-(chaîne alkyle linéaire en $C_1$ à $C_{20}$ ou chaîne alkényle linéaire en $C_1$ à $C_{20}$ présentant de 1 à 6 doubles liaisons non conjugées), une cyclohexyl-(chaîne alkyle linéaire en C1 à C20 ou chaîne alkényle linéaire en C1 à C20 présentant de 1 à 6 doubles liaisons non conjuguées), un phényle, un cyclohexyle ou un benzothiényl-(alkyle en $C_1$ à $C_{20}$ ou alkényle en $C_1$ à $C_{20}$ présentant de 1 à 6 doubles liaisons non conjuguées), un méthyl-4-penténylе-3, un méthyl-4-(triméthyl-2,6,6- cyclohexényl-1)-6-hexényle-3, un (éthényl-2)-1-triméthyl-1,5,9-décadiényle-4,8, Y-$(CH_2)_n$ ou B-(chaîne alkynyle linéaire en $C_3$ à $C_{14}$);
$R_4$ est un hydrogène, un brome ou un chlore mais n'est ni le brome, ni le chlore quand $R_3$ présente une double liaison;
n est égale à 6 à 12;
Y est $OR_5$, $CO_2R_6$, un t-butyle, un t-butyldiméthylsilyle, un diphénylméthyle, un triphénylméthyle, un pyridyle, un thiényle, un quinolyle, un N-alkylpyrrolyle en $C_1$ à $C_4$, un N-alkylpiperdyle en $C_1$ à $C_4$ un halogènure de N-alkylpyridinium en $C_1$ à $C_4$ ou un naphtyle;
$R_5$ est un hydrogène, un alkyle en $C_1$ à $C_4$ ou un alkanoyle en $C_1$ à $C_4$;
$R_6$ est un hydrogène ou un alkyle en $C_1$ à $C_4$ ;
B est un hydrogène, un phényle, un pyridyle ou un naphtyle; ledit phényle dans la définition de $R_3$ pouvant être éventuellement substitué par un alkyle en $C_1$ à $C_{14}$, un alkényle en $C_1$ à $C_{14}$, un alkynyle en $C_1$ à $C_{14}$ ou un aryle, $CO_2R_6$, un alkoxy en $C_1$ à $C_4$ ou un halogène ;
ledit procédé comprenant le fait que :
(i) on irradie une ($R_3$-CO)-4-triméthylsilylfurane-2 telle que définie dans la présente revendication en présence d'oxygène et d'un initiateur; ou
(ii) on irradie un ($R_3$CO)-4 triméthylslylfurane 2 en présence d'oxygène ou d'un initiateur; ou
(iii) on fait réagir une ($R_3$-CHOH)-4-($R_1$O)-5-furanone avec un agent oxydant.

4. Procédé selon l'une quelconque des revendications 1, 2 ou 3 dans lequel $R_3$ -Z- est en position 4 sur le cycle furanone-2

5. Procédé selon l'une quelconque des revendications 1, 2 ou 3 dans lequel $R_3$ -Z- est en position 3 sur le cycle furanone-2.

6. Procédé selon la revendication 1 donnant le composé selon la revendication 1 qui est :
l' [acétoxy-1-benzo-7-(b)-thiényl-2-heptyl]-4-hydroxy-5-furanone 2(5H);
l' [acétoxy-1-tridécyl]-4-hydroxy-5-furanone-2(5H);
l' [acétoxy-1-phényl-6-hexyl]-4-hydroxy-5-furanone-2(5H);
l' [acétoxy-1-(naphtyl-2)-6-hexyl]-4-hydroxy-5-furanone-2(5H);
l' [alpha-(méthylbenzylcarbonoyloxy)-1-tridécyl]-4-hydroxy-5-furanone2(5H).

7. Procédé selon la revendication 1 donnant le composé qui est l'[acétoxy-1-méthyl-5-(triméthyl-2,6,6-cyclohexényl-1)-7-hepténуl-4]-4-hydroxy-5-furanone-2(5H).

8. Procédé de préparation d'une composition pharmaceutique comprenant un composé de formule I tel que préparé selon l'une quelconque des revendications 1 à 7, ledit procédé comprenant le fait d'associer ledit composé avec un support pharmaceutiquement acceptable.

**Patentansprüche**
**Patentansprüche für folgende Vertragsstaaten : AT, BE, CH, DE, FR, GB, IT, LI, LU, NL, SE**

1. Verbindung der Formel:

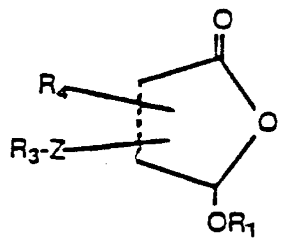

in welcher:

die gestrichelte Linie eine Einfach- oder Doppelbindung darstellt;

$R_1$ Wasserstoff, $C_1$-$C_4$ Alkyl, Benzyl oder $C_1$-$C_{20}$ Alkanoyl, Cyclohexanoyl, Benzoyl, Phenyl ($C_{1-4}$ Alkanoyl) oder Naphthoyl ist;

Z -CO- oder -C($OR_2$)H- ist,

$R_2$ $C_1$-$C_{20}$ Alkanoyl, Trihaloacetyl, Cyclohexanoyl, Benzoyl, Phenyl($C_{1-4}$ Alkanoyl), Phenyl($C_2$-$C_{14}$ Alkenoyl), Naphthoyl, oder Carbamoyl, gegebenenfalls N-substituiert durch eine oder zwei $C_{1-4}$ Alkylgruppen oder durch eine $\alpha$-($C_1$-$C_4$ Alkyl)benzylgruppe ist;

$R_3$ Wasserstoff, $C_1$-$C_{20}$ lineares Alkyl, Phenyl ($C_1$-$C_{20}$ linearkettiges Alkyl oder $C_1$-$C_{20}$ geradkettiges Alkenyl mit 1-6 nicht konjugierten Doppelbindungen), Cyclohexyl($C_1$-$C_{20}$ linearkettiges Alkyl oder $C_1$-$C_{20}$ geradkettiges Alkenyl mit 1-6 nicht konjugierten Doppelbindungen), Phenyl, Cyclohexyl oder Benzothienyl ($C_1$-$C_{20}$ Alkyl oder $C_1$-$C_{20}$ Alkenyl mit 1-6 nicht konjugierten Doppelbindungen), 4-Methyl-3-pentenyl, 4-Methyl-6-(2,6,6-Trimethylcyclohex-1-enyl)hex-3-enyl, 1-(2-Ethenyl)-1,5,9-trimethyldeca-4,8-dienyl, Y-($CH_2$)$_n$ oder B-(geradkettiges $C_3$-$C_{14}$ Alkynyl) ist;

$R_4$ Wasserstoff, Brom oder Chlor ist, jedoch nicht Brom oder Chlor, falls $R_3$ eine Doppelbindung enthält;

n den Wert 6-12 besitzt;

Y $OR_5$, $CO_2R_6$, t-Butyl,

t-Butyldimethylsilyl, Diphenylmethyl, Triphenylmethyl, Pyridyl, Thienyl, Quinolyl, N-$C_1$-$C_4$ Alkylpyrrolyl, N-$C_1$-$C_4$ Alkylpiperidyl, N-$C_1$-$C_4$ Alkylpyridinium Halogenid oder Naphthyl ist;

$R_5$ Wasserstoff, $C_1$-$C_4$ Alkyl oder $C_1$-$C_4$ Alkanoyl ist;

$R_6$ Wasserstoff oder $C_1$-$C_4$ Alkyl ist;

B Wasserstoff, Phenyl, Pyridyl oder Naphthyl ist; wobei das genannte Phenyl in der Definition von $R_3$ wahlweise durch $C_1$-$C_{14}$ Alkyl, $C_1$-$C_{14}$ Alkenyl, $C_1$-$C_{14}$ Alkynyl oder Aryl, $CO_2R_6$, $C_1$-$C_4$ Alkoxy oder Halo substituiert ist.

**2.** Verbindung nach Anspruch 1, wobei $R_3$-Z- sich in der 3-Stellung an dem 2-Furanonring befindet.

**3.** Verbindung nach Anspruch 2, in welcher die gestrichelte Linie eine Doppelbindung darstellt; Z -C($OR_2$)-H- ist; und $R_3$ Wasserstoff, $C_1$-$C_{20}$ geradkettiges Alkyl, Phenyl ($C_1$-$C_{20}$ geradkettiges Alkyl), Cyclohexyl ($C_1$-$C_{20}$ geradkettiges Alkyl), Phenyl, Cyclohexyl oder Benzothienyl ($C_1$-$C_{20}$ Alkyl), Benzothienyl($C_1$-$C_{20}$ Alkyl) 4-methyl-3-pentenyl, 4-Methyl-6-(2,6,6-trimethylcyclohex-1-enyl)hex-3-enyl, 1-(2-Ethenyl)-1,5,9-trimethyldeca-4,8-dienyl, Y-($CH_2$)$_n$ oder B-(geradkettiges $C_3$-$C_{14}$ Alkynyl) ist.

**4.** Verbindung nach Anspruch 1, worin $R_3$-Z- sich in der 4-Stellung an dem 2-Furanonring befindet.

**5.** Verbindung nach Anspruch 4, wobei $R_1$ Wasserstoff oder $C_1$-$C_{20}$ Alkanoyl ist; $R_2$ $C_1$-$C_{20}$ Alkanoyl oder gegebenfalls durch ein oder zwei $C_1$-$C_4$ Alkylgruppen oder durch eine $\alpha$-($C_1$-$C_4$ Alkyl)benzylgruppe N-substituiertes Carbamoyl ist; $R_3$ $C_5$-$C_{20}$ geradkettiges Alkyl, Benzothienyl($C_1$-$C_{20}$ Alkyl), 4-Methyl-3-pentenyl, 4-Methyl-6-(2,6,6,-trimethylcyclohex-1-enyl)hex-3-enyl oder 1-(2-Ethenyl)-1,5,9-trimethyldeca-4,8-dienyl, Y-($CH_2$)$_n$ oder B-(geradkettiges $C_3$-$C_{14}$ Alkynyl) ist und $R_4$ Wasserstoff ist.

**6.** Verbindung nach Anspruch 5, in welcher die gestrichelte Linie eine Doppelbindung darstellt und Z -C-($OR_2$)H- ist.

**7.** Verbindung nach Anspruch 6, bei der es sich um

4-[1-Acetoxy-7-benzo(b)thien-2-yl-heptyl]-5-hydroxy-2(5H)-furanon;

4-(1-Acetoxy-tridecyl)-5-hydroxy-2(5H)-furanon;

4-[1-Acetoxy-6-phenylhexyl)-5-hydroxy-2(5H)-furanon;
4-[1-Acetoxy-6-(2-naphthyl)hexyl)-5-hydroxy-2(5H)-furanon;
4-[1-($\alpha$-Methylbenzylcarbamoyloxy)tridecyl]-5-hydroxy-2(5H)-furanon; und
4-[1-Acetoxy-5-methyl-7-(2,6,6-trimethylcyclohex-1-enyl)-hept-4-enyl]-5-hydroxy-2(5H)-furanon handelt.

**8.** Verbindung wie in einem der Ansprüche 1 bis 7 definiert, zur Verwendung auf medizinischem Gebiet.

**9.** Pharmazeutische Zusammensetzung umfassend eine Verbindung wie in einem der Ansprüche 1 bis 7 definiert, und einen pharmazeutisch verträglichen Träger.

**10.** Verfahren zur Herstellung einer Verbindung wie in einem der Ansprüche 1 bis 7 definiert, wobei das Verfahren umfaßt:
(i) falls die Herstellung einer Verbindung gewünscht ist, in welcher $R_1$ Wasserstoff und Z $-C(OR_2)H-$ ist, die Bestrahlung eines 4-($R_3$-CH($OR_2$)-2-Trimethylsilylfurans in Gegenwart von Sauerstoff und einem Initiator; oder
(ii) falls die Herstellung einer Verbindung gewünscht ist, in welcher Z eine Gruppe $-C(OR_2)H-$ ist, die Reaktion der entsprechenden Verbindung, wobei $R_1$ und/oder $R_2$ Wasserstoff sind, mit dem(n) geeigneten Acylierungsmittel(n); oder
(iii) falls die Herstellung einer Verbindung gewünscht ist, bei welcher Z eine Gruppe -CO-ist:
(a) Bestrahlen eines 4-($R_3$-CO)-2-Trimethylsilylfuranons wie in diesem Anspruch bestimmt, in Gegenwart von Sauerstoff und einem Initiator; oder
(b) Bestrahlen eines 4-($R_3$-CO)-2-Trimethylsilylfurans in Gegenwart von Sauerstoff und einem Initiator; oder
(c) Reagieren eines 4-($R_3$-CHOH)-5-($R_1$O)-furanons mit einem Oxydationsmittel.

**Patentansprüche für folgende Vertragsstaaten : ES, GR**

**1.** Verfahren zur Herstellung einer Verbindung der Formel (I):

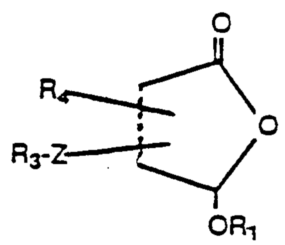

(I)

worin:
die gestrichelte Linie eine Einfach- oder Doppelbindung darstellt;
$R_1$ Wasserstoff ist;
Z $-C(OR_2)H-$ ist;
$R_2$ $C_1$-$C_{20}$ Alkanoyl, Trihaloacetyl, Cyclohexanoyl, Benzoyl, Phenyl($C_{1-4}$ Alkanoyl), Phenyl($C_2$-$C_{14}$ Alkenoyl), Naphthoyl, oder Carbamoyl, gegebenenfalls N-substituiert durch eine oder zwei $C_{1-4}$ Alkylgruppen oder durch eine $\alpha$-($C_1$-$C_4$ Alkyl)benzylgruppe ist;
$R_3$ Wasserstoff, $C_1$-$C_{20}$ lineares Alkyl, Phenyl ($C_1$-$C_{20}$ linearkettiges Alkyl oder $C_1$-$C_{20}$ geradkettiges Alkenyl mit 1-6 nicht konjugierten Doppelbindungen), Cyclohexyl($C_1$-$C_{20}$ linearkettiges Alkyl oder $C_1$-$C_{20}$ geradkettiges Alkenyl mit 1-6 nicht konjugierten Doppelbindungen), Phenyl, Cyclohexyl oder Benzothienyl ($C_1$-$C_{20}$ Alkyl oder $C_1$-$C_{20}$ Alkenyl mit 1-6 nicht konjugierten Doppelbindungen), 4-Methyl-3-pentenyl, 4-Methyl-6-(2,6,6-trimethylcyclohex-1-enyl)hex-3-enyl, 1-(2-Ethenyl)-1,5,9-trimethyldeca-4,8-dienyl, Y-$(CH_2)_n$ oder B-(geradkettiges $C_3$-$C_{14}$ Alkynyl) ist;
$R_4$ Wasserstoff, Brom oder Chlor ist, jedoch nicht Brom oder Chlor, falls $R_3$ eine Doppelbindung enthält;
n den Wert 6-12 besitzt;
Y $OR_5$, $CO_2R_6$, t-Butyl,

t-Butyldimethylsilyl, Diphenylmethyl, Triphenylmethyl, Pyridyl, Thienyl, Quinolyl, N-$C_1$-$C_4$ Alkylpyrrolyl, N-$C_1$-$C_4$ Alkylpiperidyl, N-$C_1$-$C_4$ Alkylpyridinium Halogenid oder Naphthyl ist;

$R_5$ Wasserstoff, $C_1$-$C_4$ Alkyl oder $C_1$-$C_4$ Alkanoyl ist;

$R_6$ Wasserstoff oder $C_1$-$C_4$ Alkyl ist;

B Wasserstoff, Phenyl, Pyridyl oder Naphthyl ist; wobei das genannte Phenyl in der Definition von $R_3$ wahlweise durch $C_1$-$C_{14}$ Alkyl, $C_1$-$C_{14}$ Alkenyl, $C_1$-$C_{14}$ Alkynyl oder Aryl, $CO_2R_6$, $C_1$-$C_4$ Alkoxy oder Halo substituiert ist;

wobei das Verfahren die Bestrahlung eines 4-[$R_3$-CH($OR_2$)]-2-Trimethylsilylfurans in Gegenwart von Sauerstoff und einem Initiator umfaßt.

**2.** Verfahren zur Herstellung einer Verbindung der Formel (I):

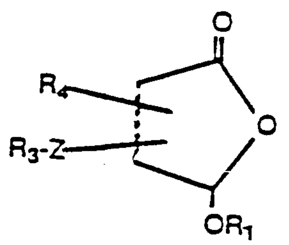

(I)

worin:

die gestrichelte Linie eine Einfach- oder Doppelbindung darstellt;

$R_1$ $C_1$-$C_4$ Alkyl, Benzyl, $C_1$-$C_{20}$ Alkanoyl, Cyclohexanoyl, Benzoyl, Phenyl ($C_{1-4}$ Alkanoyl) oder Naphthoyl ist;

Z -C($OR_2$)H- ist;

$R_2$ $C_1$-$C_{20}$ Alkanoyl, Trihaloacetyl, Cyclohexanoyl, Benzoyl, Phenyl($C_{1-4}$ Alkanoyl), Phenyl($C_2$-$C_{14}$ Alkenoyl), Naphthoyl, oder Carbamoyl, gegebenenfalls N-substituiert durch eine oder zwei $C_{1-4}$ Alkylgruppen oder durch eine $\alpha$-($C_1$-$C_4$ Alkyl)benzylgruppe ist;

$R_3$ Wasserstoff, $C_1$-$C_{20}$ lineares Alkyl, Phenyl ($C_1$-$C_{20}$ linearkettiges Alkyl oder $C_1$-$C_{20}$ geradkettiges Alkenyl mit 1-6 nicht konjugierten Doppelbindungen), Cyclohexyl($C_1$-$C_{20}$ linearkettiges Alkyl oder $C_1$-$C_{20}$ geradkettiges Alkenyl mit 1-6 nicht konjugierten Doppelbindungen), Phenyl, Cyclohexyl oder Benzothienyl ($C_1$-$C_{20}$ Alkyl oder $C_1$-$C_{20}$ Alkenyl mit 1-6 nicht konjugierten Doppelbindungen), 4-Methyl-3-pentenyl, 4-Methyl-6-(2,6,6-trimethylcyclohex-1-enyl)hex-3-enyl, 1-(2-Ethenyl)-1,5,9-trimethyldeca-4,8-dienyl, Y-$(CH_2)_n$ oder B-(geradkettiges $C_3$-$C_{14}$ Alkynyl) ist;

$R_4$ Wasserstoff, Brom oder Chlor ist, jedoch nicht Brom oder Chlor, falls $R_3$ eine Doppelbindung enthält;

n den Wert 6-12 besitzt;

Y $OR_5$, $CO_2R_6$, t-Butyl,
t-Butyldimethylsilyl, Diphenylmethyl, Triphenylmethyl, Pyridyl, Thienyl, Quinolyl, N-$C_1$-$C_4$ Alkylpyrrolyl, N-$C_1$-$C_4$ Alkylpiperidyl, N-$C_1$-$C_4$ Alkylpyridinium Halogenid oder Naphthyl ist;

$R_5$ Wasserstoff, $C_1$-$C_4$ Alkyl oder $C_1$-$C_4$ Alkanoyl ist;

$R_6$ Wasserstoff oder $C_1$-$C_4$ Alkyl ist;

B Wasserstoff, Phenyl, Pyridyl oder Naphthyl ist; wobei das genannte Phenyl in der Definition von $R_3$ wahlweise durch $C_1$-$C_{14}$ Alkyl, $C_1$-$C_{14}$ Alkenyl, $C_1$-$C_{14}$ Alkynyl oder Aryl, $CO_2R_6$, $C_1$-$C_4$ Alkoxy oder Halo substituiert ist;

wobei das Verfahren die Reaktion des entsprechenden Furanons, in welchem $R_1$ und/oder $R_2$ Wasserstoff ist, mit dem(n) geeigneten Aceliermittel(n) umfaßt.

**3.** Verfahren zur Herstellung einer Verbindung der Formel (I):

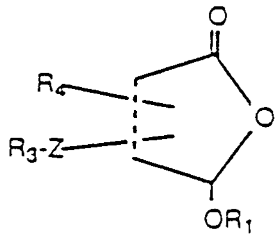

(I)

worin:

die gestrichelte Linie eine Einfach- oder Doppelbindung darstellt;

$R_1$ Wasserstoff ist;

Z -CO- ist;

$R_3$ Wasserstoff, $C_1$-$C_{20}$ lineares Alkyl, Phenyl ($C_1$-$C_{20}$ linearkettiges Alkyl oder $C_1$-$C_{20}$ geradkettiges Alkenyl mit 1-6 nicht konjugierten Doppelbindungen), Cyclohexyl($C_1$-$C_{20}$ linearkettiges Alkyl oder $C_1$-$C_{20}$ geradkettiges Alkenyl mit 1-6 nicht konjugierten Doppelbindungen), Phenyl, Cyclohexyl oder Benzothienyl ($C_1$-$C_{20}$ Alkyl oder $C_1$-$C_{20}$ Alkenyl mit 1-6 nicht konjugierten Doppelbindungen), 4-Methyl-3-pentenyl, 4-Methyl-6-(2,6,6-trimethylcyclohex-1-enyl)hex-3-enyl, 1-(2-Ethenyl)-1,5,9-trimethyldeca-4,8-dienyl, Y-$(CH_2)_n$ oder B-(geradkettiges $C_3$-$C_{14}$ Alkynyl) ist;

$R_4$ Wasserstoff, Brom oder Chlor ist, jedoch nicht Brom oder Chlor, falls $R_3$ eine Doppelbindung enthält;

n den Wert 6-12 besitzt;

Y $OR_5$, $CO_2R_6$, t-Butyl,
t-Butyldimethylsilyl, Diphenylmethyl, Triphenylmethyl, Pyridyl, Thienyl, Quinolyl, N-$C_1$-$C_4$ Alkylpyrrolyl, N-$C_1$-$C_4$ Alkylpiperidyl, N-$C_1$-$C_4$ Alkylpyridinium Halogenid oder Naphthyl ist;

$R_5$ Wasserstoff, $C_1$-$C_4$ Alkyl oder $C_1$-$C_4$ Alkanoyl ist;

$R_6$ Wasserstoff oder $C_1$-$C_4$ Alkyl ist;

B Wasserstoff, Phenyl, Pyridyl oder Naphthyl ist; wobei das genannte Phenyl in der Definition von $R_3$ wahlweise durch $C_1$-$C_{14}$ Alkyl, $C_1$-$C_{14}$ Alkenyl, $C_1$-$C_{14}$ Alkynyl oder Aryl, $CO_2R_6$, $C_1$-$C_4$ Alkoxy oder Halo substituiert ist;

wobei das Verfahren umfaßt:

(i) Bestrahlen eines 4-($R_3$-CO)-2-Trimethylsilylfuranons wie in diesem Anspruch beschrieben, in Gegenwart von Sauerstoff und einem Initiator; oder

(ii) Bestrahlen eines 4-($R_3$-CO)-2-Trimethylsilylfurans in Gegenwart von Sauerstoff und einem Initiator; oder

(iii) Reagieren eines 4-($R_3$-CHOH)-5-($R_1$O)-furanons mit einem Oxydationsmittel.

**4.** Verfahren nach einem der Ansprüche 1, 2 oder 3, wobei $R_3$-Z- sich in der 4-Stellung an dem 2-Furanonring befindet.

**5.** Verfahren nach einem der Ansprüche 1, 2 oder 3, wobei $R_3$-Z- sich in der 3-Stellung an dem 2-Furanonring befindet.

**6.** Verfahren nach Anspruch 1, welches die Verbindung

4-[1-Acetoxy-7-benzo(b)thien-2-yl-heptyl]-5-hydroxy-2(5H)-furanon;

4-(1-Acetoxy-tridecyl)-5-hydroxy-2(5H)-furanon;

4-[1-Acetoxy-6-phenylhexyl)-5-hydroxy-2(5H)-furanon;

4-[1-Acetoxy-6-(2-naphthyl)hexyl)-5-hydroxy-2(5H)-furanon;

4-[1-($\alpha$-Methylbenzylcarbamoyloxy)tridecyl]-5-hydroxy-2(5H)-furanon ergibt.

**7.** Verfahren nach Anspruch 1, welches 4-[1-Acetoxy-5-methyl-7-(2,6,6-trimethyl-cyclohex-1-enyl)-hept-4-enyl]-5-hydroxy-2(5H)-furanon ergibt.

**8.** Verfahren zur Herstellung einer pharmazeutischen Zusammensetzung, welche eine Verbindung der Formel I wie nach einem der Ansprüche 1 bis 7 hergestellt enthält, wobei das Verfahren umfaßt, daß man die Verbindung in Verbund mit einem pharmazeutisch verträglichen Träger bringt.